# EUROPEAN PATENT APPLICATION

(11) **EP 4 389 851 A1**
(43) Date of publication of application: **26.06.2024**
(21) Application number: 23199851.9
(22) Date of filing: 26.09.2023
(51) Int. Cl.: C09K 11/06, C07D 251/24

(54) **ORGANIC COMPOUND, ORGANIC LIGHT EMITTING DIODE AND ORGANIC LIGHT EMITTING DEVICE HAVING THE COMPOUND**

(30) Priority: 22.12.2022 KR 20220181920
(71) Applicant: LG Display Co., Ltd., Seoul 07336 (KR); LT Materials Co., Ltd., Yongin-si, Gyeonggi-do 17118 (KR)
(72) Inventor: Kim, Sang-Beom, 10845 Paju-si, Gyeonggi-do (KR); Yoo, Seon-Keun, 10845 Paju-si, Gyeonggi-do (KR); Yu, Young-Jun, 10845 Paju-si Gyeonggi-do (KR); Kim, Woo-Sam, 17118 Yongin-si, Gyeonggi-do (KR); Jang, Hyung-Keun, 17118 Yongin-si, Gyeonggi-do (KR); Choi, Dae-Hyuk, 17118 Yongin-si, Gyeonggi-do (KR); Kim, Dong-Jun, 17118 Yongin-si, Gyeonggi-do (KR); No, Young-Seok, 17118 Yongin-si, Gyeonggi-do (KR); Lee, Hyun-Joo, 17118 Yongin-si, Gyeonggi-do (KR); Park, Geon-Yu, 17118 Yongin-si, Gyeonggi-do (KR); Ahn, Sang-Hun, 17118 Yongin-si, Gyeonggi-do (KR)
(74) Representative: Ter Meer Steinmeister & Partner

(57) **Abstract**

The present invention relates to an organic compound including at least one fused hetero aromatic moiety with at least one nitrogen atom linked to a triazine moiety directly or through a linking group and a benzothiazole moiety linked to the triazine moiety, and substituted with at least one of deuterium and a deuterium-substituted group, an organic light emitting diode and an organic light emitting device having the organic compound. The organic light emitting diode and the organic light emitting device where an emissive layer includes the organic compound have beneficial luminous efficiency and luminous lifespan.

## Description

### BACKGROUND

### Technical Field

The present invention relates to an organic compound, and more particularly to, an organic compound with beneficial luminous efficiency and luminous lifespan and an organic light emitting diode and an organic light emitting device including the compound.

### Discussion of the Related Art

A flat display device including an organic light emitting diode (OLED) has attracted attention as a display device that can replace a liquid crystal display device (LCD). The electrode configurations in the OLED can implement unidirectional or bidirectional images. Also, the OLED can be formed even on a flexible transparent substrate such as a plastic substrate so that a flexible or a foldable display device can be realized with ease using the OLED. In addition, the OLED can be driven at a lower voltage and the OLED has advantageous high color purity compared to the LCD.

Since fluorescent material uses only singlet excitons in the luminous process, the related art fluorescent material shows low luminous efficiency. On the contrary, phosphorescent material can show high luminous efficiency since it uses triplet exciton as well as singlet excitons in the luminous process. However, examples of phosphorescent material include metal complexes, which have a short luminous lifespan for commercial use.

### SUMMARY

The present invention is directed to an organic compound, an organic light emitting diode and an organic light emitting device that substantially obviate one or more of the problems due to the limitations and disadvantages of the related art.

One objective of the present invention is to provide an organic compound having beneficial luminous efficiency and excellent luminous lifespan, and an organic light emitting diode and an organic light emitting device including the compound. This objective has been solved by the provision of the organic compound having the features as defined in the independent claims. Preferred embodiments are covered by the dependent claims.

Additional features and aspects will be set forth in the description that follows, and in part will be apparent from the description, or may be learned by practice of the disclosed concepts provided herein. Other features and aspects of the disclosed concept may be realized and attained by the structure particularly pointed out in the written description, or derivable therefrom, and the claims hereof as well as the appended drawings.

To achieve these and other aspects of the inventive concepts, as embodied and broadly described, in one aspect, the present invention provides an organic compound having the following structure of Chemical Formula 1: 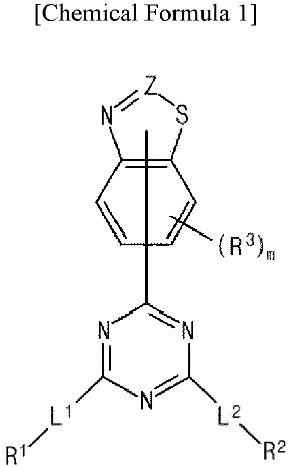 wherein, in the Chemical Formula 1,
each of R¹ and R² is independently an unsubstituted or substituted C₆-C₃₀ aryl group or an unsubstituted or substituted C₃-C₃₀ hetero aryl group;
R³ is independently deuterium, an unsubstituted or substituted C₁-C₂₀ alkyl group, an unsubstituted or substituted C₆-C₃₀ aryl group or an unsubstituted or substituted C₃-C₃₀ hetero aryl group, where each R³ is identical to or different from each other when m is 2, 3 or 4;
Z is CR⁴ or a carbon atom linked to a triazine moiety, where R⁴ is hydrogen, deuterium, an unsubstituted or substituted C₁-C₂₀ alkyl group, an unsubstituted or substituted C₆-C₃₀ aryl group or an unsubstituted or substituted C₃-C₃₀ hetero aryl group,
wherein at least one of R¹, R², R³ and R⁴ is deuterium, a deuterium-substituted C₁-C₂₀ alkyl group, a deuterium-substituted C₆-C₃₀ aryl group or a deuterium-substituted C₃-C₃₀ hetero aryl group, and at least one of R¹, R² and R⁴ is an unsubstituted or substituted C₁₀-C₃₀ fused hetero aryl group with at least one nitrogen atom;
   each of L¹ and L² is independently a single bond or an unsubstituted or substituted C6-C30 arylene group; and
m is 0, 1, 2 or 3 when Z is CR⁴ and m is 0, 1, 2, 3 or 4 when Z is the carbon atom linked to the triazine moiety.

In one example embodiment, the organic compound can have the following structure of Chemical Formula 2: 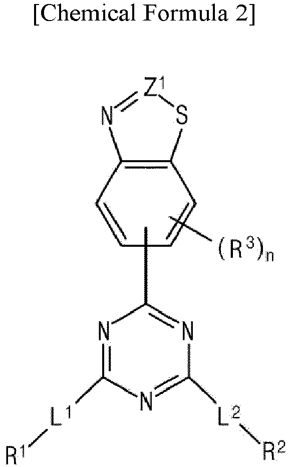 wherein, in the Chemical Formula 2,
each of R¹, R², R³, L¹ and L² is a same as defined in Chemical Formula 1; Z¹ is CR⁴, where R⁴ is a same as defined in Chemical Formula 1; and
n is 0, 1, 2 or 3.

As an example, the organic compound can have the following structure of Chemical Formula 3A, Chemical Formula 3B, Chemical Formula 3C or Chemical Formula 3D: 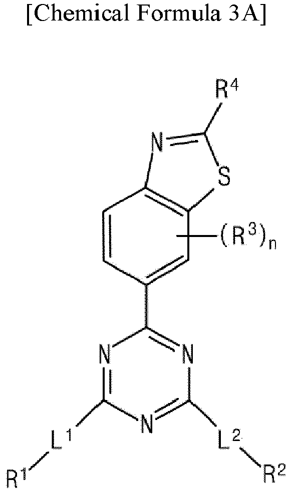 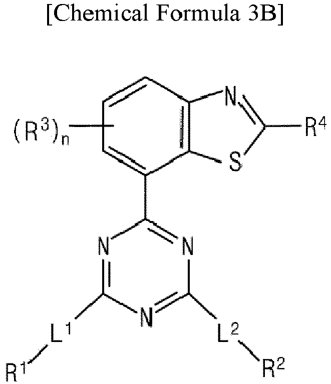 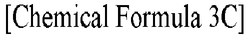 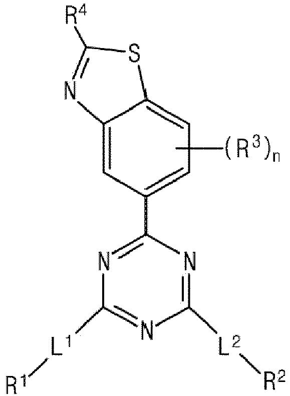 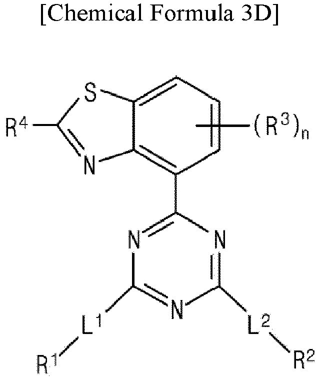 wherein, in the Chemical Formulae 3A, 3B, 3C and 3D,
each of R¹, R², R³, R⁴, L¹ and L² is a same as defined in Chemical Formula 1; and n is 0, 1, 2 or 3.

In another example embodiment, the organic compound can have the following structure of Chemical Formula 4: 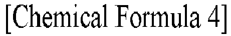 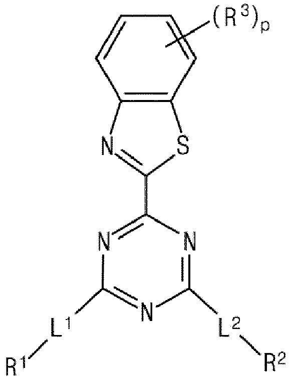 wherein, in the Chemical Formula 4,
each of R¹, R², R³, L¹ and L² is a same as defined in Chemical Formula 1; and p is 0, 1, 2, 3 or 4.

As an example, one of R¹ and R² in Chemical Formula 1 can be a carbazolyl group unsubstituted or substituted with deuterium or a C₁-C₂₀ alkyl group, where the C₁-C₂₀ alkyl group substituted to the carbazolyl group can be unsubstituted or substituted with deuterium, the other of R¹ and R² in Chemical Formula 1 can be selected from the group consisting of phenyl, biphenyl, pyrenyl, fluorenyl, dibenzofuranyl, dibenzothiophenyl and carbazolyl each of which is independently unsubstituted or substituted with at least one of deuterium, a C₁-C₂₀ alkyl group and a C₆-C₃₀ aryl group, where each of the C₁-C₂₀ alkyl group and the C₆-C₃₀ aryl group substituted to R² can be independently unsubstituted or further substituted with deuterium, R³ in Chemical Formula 1 can be deuterium or a C₁-C₂₀ alkyl group unsubstituted or substituted with deuterium, R⁴ in Chemical Formula 1 can be phenyl unsubstituted or substituted with deuterium or a C₁-C₂₀ alkyl group, where the C₁-C₂₀ alkyl group substituted to the phenyl can be unsubstituted with deuterium, and/or m can be 0, 1, 2, 3 or 4.

In a preferred embodiment, the organic compound can be at least one of the following compounds

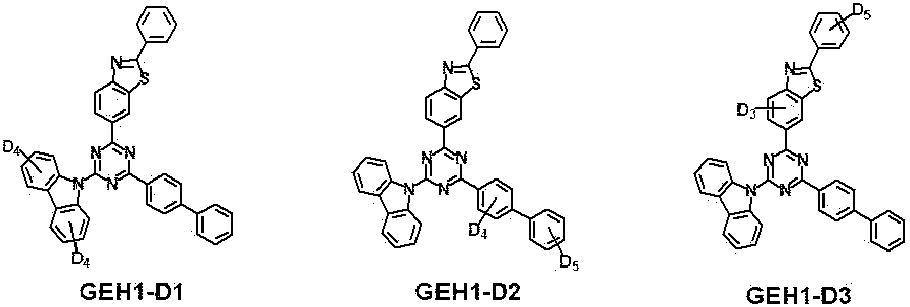

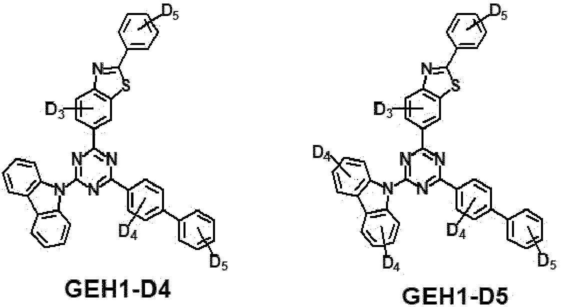

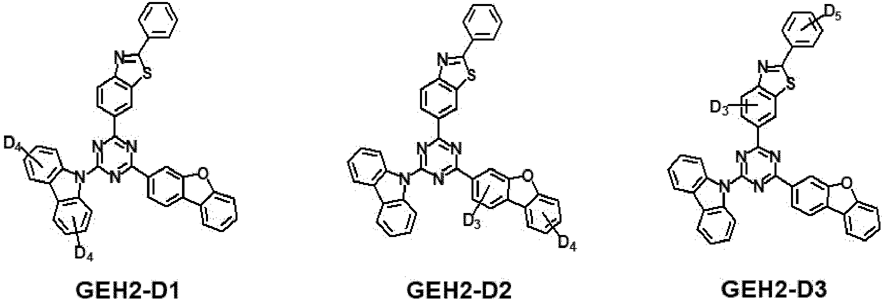

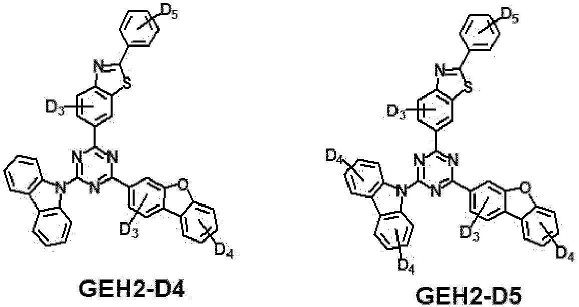

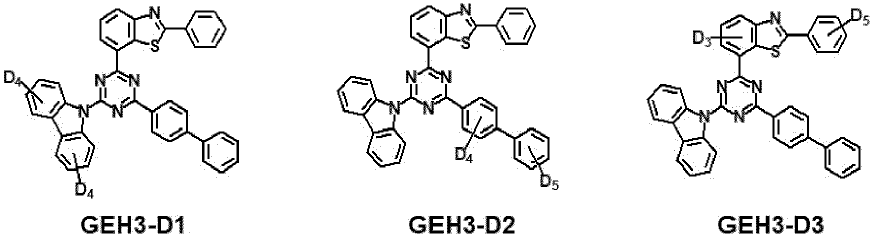

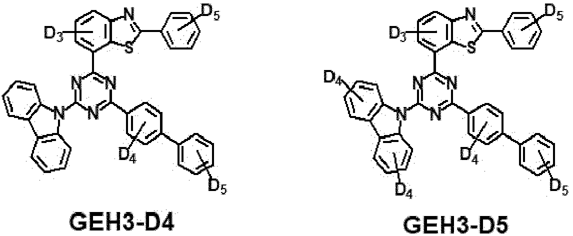

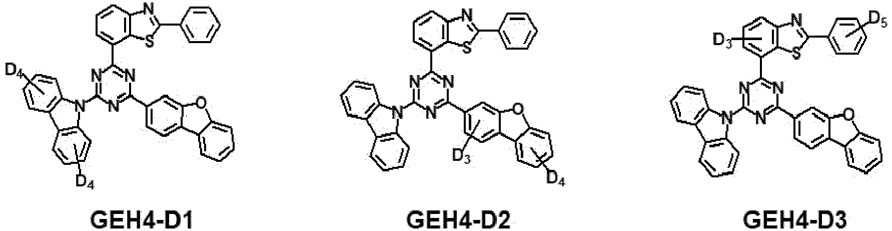

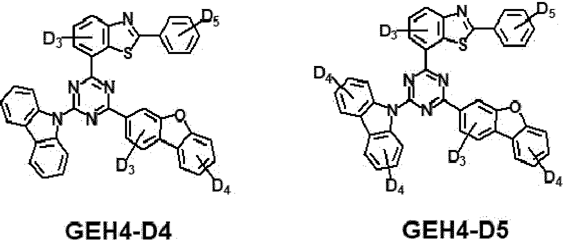

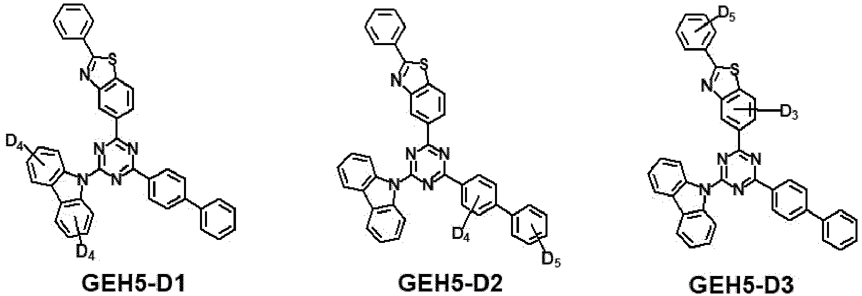

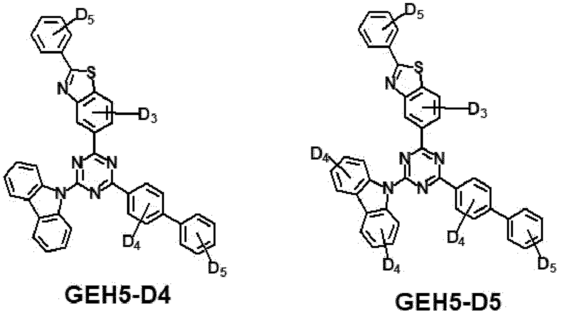

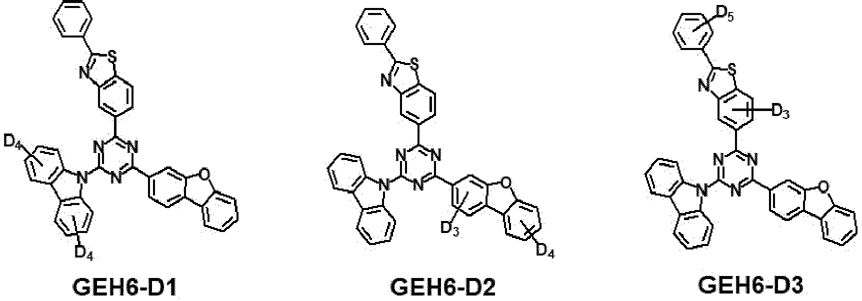

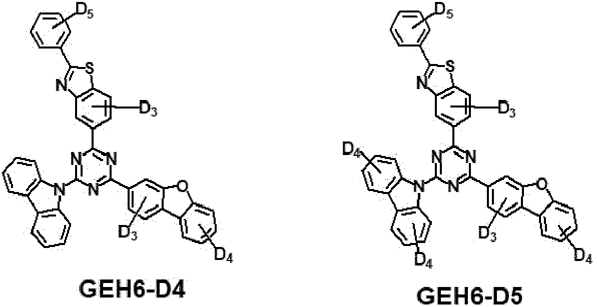

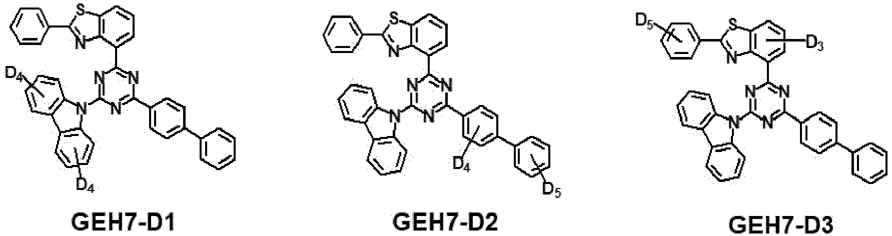

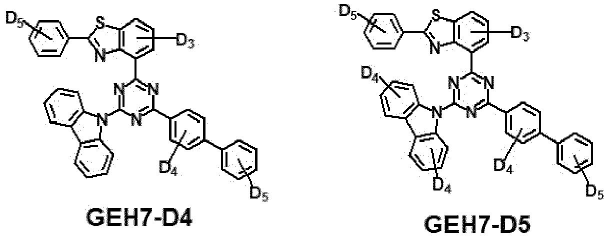

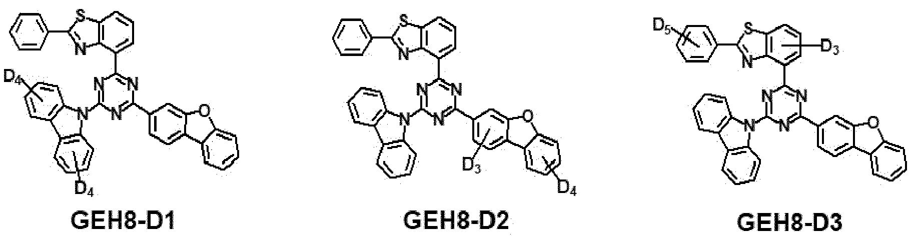

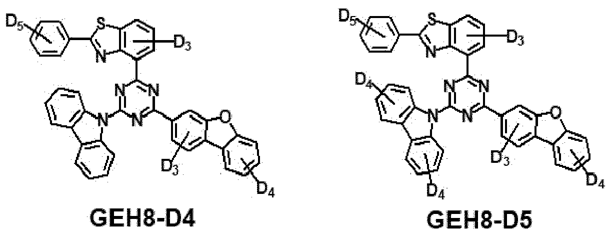

In another preferred embodiment, the organic compound can be at least one of the following compounds:

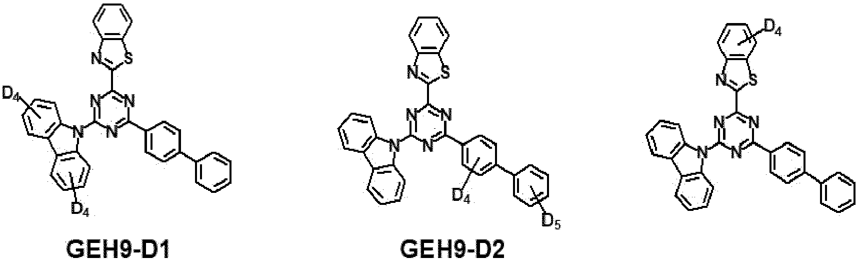

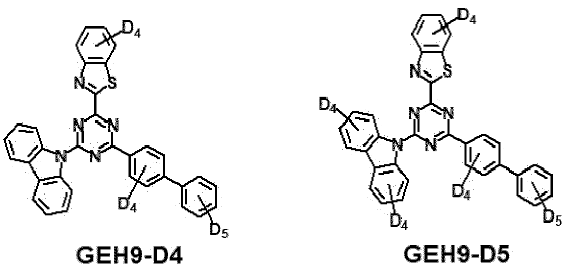

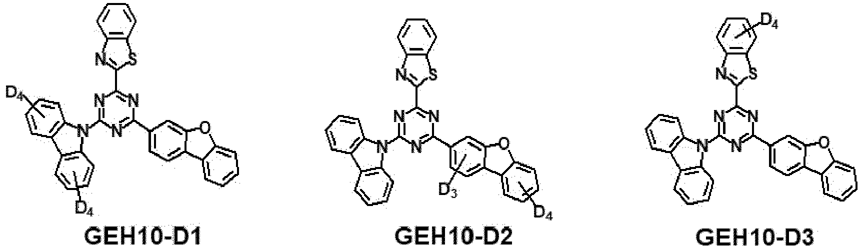

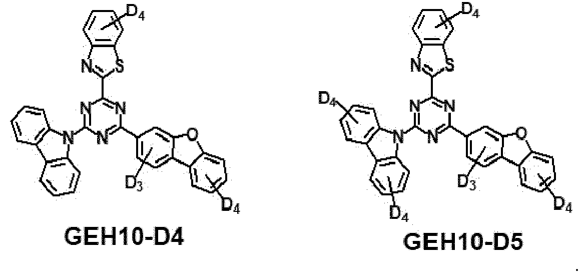

In another aspect, the present invention provides an organic light emitting device includes a first electrode; a second electrode facing the first electrode; and an emissive layer disposed between the first electrode and the second electrode, wherein the emissive layer includes an organic compound as described above or according to one of the preferred embodiments above or hereinafter.

The emissive layer can include at least one emitting material layer.

As an example, the at least one emitting material layer can include a first host (*e.g.* N-type host) and a dopant (emitter), and the first host can include the organic compound.

Optionally, the at least one emitting material layer can further include a second host (*e.g.* P-type host).

The emissive layer can have a single emitting part, or multiple emitting parts to form a tandem structure.

The emissive layer can include
a first emitting part disposed between the first electrode and the second electrode and including a first emitting material layer;
a second emitting part disposed between the first emitting part and the second electrode and including a second emitting material layer; and
a first charge generation layer disposed between the first emitting part and the second emitting part,
wherein at least one of the first emitting material layer and the second emitting material layer includes the organic compound.

The second emitting material layer can include
a first layer disposed between the first charge generation layer and the second electrode; and
a second layer disposed between the first layer and the second electrode,
wherein at least one of the first layer and the second layer includes the organic compound.

In an embodiment, one of the first layer and the second layer is a red emitting material layer and the other of the first layer and the second layer is a green emitting material layer, and wherein the green emitting material layer includes the organic compound.

In an embodiment, the second emitting material layer further includes a third layer disposed between the first layer and the second layer.

In an embodiment, the emissive layer further includes:
a third emitting part disposed between the second emitting part and the second electrode and including a third emitting material layer; and
a second charge generation layer disposed between the second emitting part and the third emitting part.

In yet another aspect, the present invention provides an organic light emitting device, for example, an organic light emitting display device or an organic light emitting illumination device, that includes a substrate and the organic light emitting diode as described above or according to one of the preferred embodiments above or hereinafter over the substrate.

The organic compound includes a at least one fused hetero aromatic moiety including at least one nitrogen atom linked directly or indirectly to a triazine moiety, and a benzothiazole moiety linked to the triazine moiety. The organic compound including the triazine moiety as an electron donor and the fused hetero aromatic moiety as an electron acceptor can accept both holes and electros rapidly.

The organic compound can be applied into the emissive layer in the organic light emitting diode. The organic compound has relatively higher singlet and triplet energy levels compared to an emitter, and wide energy bandgap between HOMO energy level and LUMO energy level. The organic compound has large dipole moment so that electron injections between the emitting material layer and the electron transport layer can be improved. Exciton energies can be transferred from the organic compound to the emitter in the emitting material layer by using the organic compound as the host in the emitting material layer.

In addition, the organic compound includes at least one deuterium and/or a deuterium-substituted aliphatic group, a deuterium-substituted aromatic group and a deuterium-substituted hetero aromatic group. The organic light emitting diode and the organic light emitting device with beneficial luminous efficiency and luminous lifetime can be realized by applying the organic compound into the diode and the device.

It is to be understood that both the foregoing general description and the following detailed description are exemplary and explanatory, and are intended to provide further explanation of the inventive concepts as claimed.

### BRIEF DESCRIPTION OF THE DRAWINGS

The accompanying drawings, which are included to provide a further understanding of the invention, are incorporated in and constitute a part of this application, illustrate embodiments of the invention and together with the description serve to explain principles of the invention.
FIG. 1 illustrates a schematic circuit diagram of an organic light emitting display device in accordance with the present invention.
FIG. 2 illustrates a cross-sectional view of an organic light emitting display device as an example of an organic light emitting device in accordance with an example embodiment of the present invention.
FIG. 3 illustrates a cross-sectional view of an organic light emitting diode having a single emitting part in accordance with an example embodiment of the present invention.
FIG. 4 illustrates a cross-sectional view of an organic light emitting display device in accordance with another example embodiment of the present invention.
FIG. 5 illustrates a cross-sectional view of an organic light emitting diode with two emitting parts forming a tandem structure in accordance with another example embodiment of the present invention.
FIG. 6 illustrates a cross-sectional view of an organic light emitting diode with three emitting parts forming a tandem structure in accordance with another example embodiment of the present invention.

### DETAILED DESCRIPTION

Reference will now be made in detail to aspects of the invention, examples of which are illustrated in the accompanying drawings. Wherever possible, the same reference numbers will be used throughout the drawings to refer to the same or like parts.

### [Organic Compound]

The organic compound of the present invention has beneficial luminous properties. The organic compound can have the following structure of Chemical Formula 1: 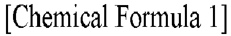 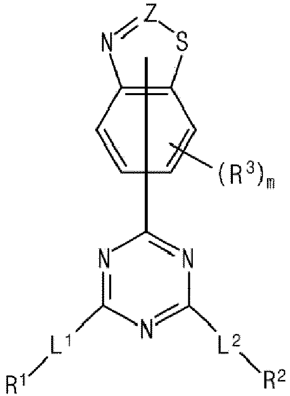 wherein, in the Chemical Formula 1,
each of R¹ and R² is independently an unsubstituted or substituted C₆-C₃₀ aryl group or an unsubstituted or substituted C₃-C₃₀ hetero aryl group;
R³ is independently deuterium, an unsubstituted or substituted C₁-C₂₀ alkyl group, an unsubstituted or substituted C₆-C₃₀ aryl group or an unsubstituted or substituted C₃-C₃₀ hetero aryl group, where each R³ is identical to or different from each other when m is 2, 3 or 4;
Z is CR⁴ or a carbon atom linked to a triazine moiety, where R⁴ is hydrogen, deuterium, an unsubstituted or substituted C₁-C₂₀ alkyl group, an unsubstituted or substituted C₆-C₃₀ aryl group or an unsubstituted or substituted C₃-C₃₀ hetero aryl group,
wherein at least one of R¹, R², R³ and R⁴ is deuterium, a deuterium-substituted C₁-C₂₀ alkyl group, a deuterium-substituted C₆-C₃₀ aryl group or a deuterium-substituted C₃-C₃₀ hetero aryl group, and at least one of R¹, R² and R⁴ is an unsubstituted or substituted C₁₀-C₃₀ fused hetero aryl group with at least one nitrogen atom;
each of L¹ and L² is independently a single bond or an unsubstituted or substituted C6-C30 arylene group; and
m is 0, 1, 2 or 3 when Z is CR⁴ and m is 0, 1, 2, 3 or 4 when Z is the carbon atom linked to the triazine moiety.

As used herein, the term "unsubstituted" means that hydrogen is directly linked to a carbon atom. "Hydrogen", as used herein, can refer to protium.

As used herein, "substituted" means that the hydrogen is replaced with a substituent. The substituent can comprise, but is not limited to, an unsubstituted or deuterium- or halogen-substituted C₁-C₂₀ alkyl group, an unsubstituted or deuterium- or halogen-substituted C₁-C₂₀ alkoxy, halogen, a cyano group, a hydroxyl group, a carboxylic group, a carbonyl group, an amino group, an unsubstituted or deuterium-substituted a C₁-C₁₀ alkyl amino group, an unsubstituted or deuterium-substituted C₆-C₃₀ aryl amino group, an unsubstituted or deuterium-substituted C₃-C₃₀ hetero aryl amino group, a nitro group, a hydrazyl group, a sulfonate group, an unsubstituted or deuterium- or halogen-substituted C₁-C₁₀ alkyl silyl group, an unsubstituted or deuterium- or halogen-substituted C₁-C₁₀ alkoxy silyl group, an unsubstituted or deuterium- or halogen-substituted C₃-C₂₀ cyclo alkyl silyl group, an unsubstituted or deuterium- or halogen-substituted C₆-C₃₀ aryl silyl group, an unsubstituted or deuterium-substituted C₃-C₃₀ hetero aryl silyl group, an unsubstituted or substituted C₆-C₃₀ aryl group, an unsubstituted or substituted C₃-C₃₀ hetero aryl group.

For example, each of the C₆-C₃₀ aryl group and the C₃-C₃₀ hetero aryl group can be substituted with at least one of deuterium, C₁-C₂₀ alkyl, C₆-C₃₀ aryl and C₃-C₃₀ hetero aryl.

As used herein, the term "hetero" in terms such as "a hetero aromatic group", "a hetero cyclo alkylene group", "a hetero arylene group", "a hetero aryl alkylene group", "a hetero aryl oxylene group", "a hetero cyclo alkyl group", "a hetero aryl group", "a hetero aryl alkyl group", "a hetero aryloxy group", "a hetero aryl amino group" and the likes means that at least one carbon atom, for example 1 to 5 carbons atoms, constituting an aliphatic chain, an alicyclic group or ring or an aromatic group or ring is substituted with at least one hetero atom selected from the group consisting of N, O, S and P.

As used herein, the C₆-C₃₀ aromatic group can comprise, but is not limited to, a C₆-C₃₀ aryl group, a C₇-C₃₀ aryl alkyl group, a C₆-C₃₀ aryloxy group and/or a C₆-C₃₀ aryl amino group, each of which can be independently unsubstituted or substituted. For example, the C₆-C₃₀ aryl group can include, but is not limited to, an unfused or fused aryl group such as phenyl, biphenyl, terphenyl, naphthyl, anthracenyl, pentalenyl, indenyl, indeno-indenyl, heptalenyl, biphenylenyl, indacenyl, phenalenyl, phenanthrenyl, benzo-phenanthrenyl, dibenzo-phenanthrenyl, azulenyl, pyrenyl, fluoranthenyl, triphenylenyl, chrysenyl, tetraphenylenyl, tetracenyl, pleiadenyl, picenyl, pentaphenylenyl, pentacenyl, fluorenyl, indeno-fluorenyl or spiro-fluorenyl. The C₆-C₃₀ arylene group can include, but is not limited to, any bivalent linking group corresponding to the above aryl group.

As used herein, the C₃-C₃₀ hetero aromatic group can comprise, but is not limited to, a C₃-C₃₀ hetero aryl group, a C₄-C₃₀ hetero aryl alkyl group, a C₃-C₃₀ hetero aryloxy group and/or a C₃-C₃₀ hetero aryl amino group, each of which can be independently unsubstituted or substituted. For example, the C₃-C₃₀ hetero aryl group can comprise, but is not limited to, an unfused or fused hetero aryl group such as pyrrolyl, pyridinyl, pyrimidinyl, pyrazinyl, pyridazinyl, triazinyl, tetrazinyl, imidazolyl, pyrazolyl, indolyl, iso-indolyl, indazolyl, indolizinyl, pyrrolizinyl, carbazolyl, benzo-carbazolyl, dibenzo-carbazolyl, indolo-carbazolyl, indeno-carbazolyl, benzo-furo-carbazolyl, benzo-thieno-carbazolyl, carbolinyl, quinolinyl, iso-quinolinyl, phthlazinyl, quinoxalinyl, cinnolinyl, quinazolinyl, quinolizinyl, purinyl, benzo-quinolinyl, benzo-iso-quinolinyl, benzo-quinazolinyl, benzo-quinoxalinyl, acridinyl, phenazinyl, phenoxazinyl, phenothiazinyl, phenanthrolinyl, perimidinyl, phenanthridinyl, pteridinyl, naphthyridinyl, furanyl, pyranyl, oxazinyl, oxazolyl, oxadiazolyl, triazolyl, dioxinyl, benzo-furanyl, dibenzo-furanyl, thiopyranyl, xanthenyl, chromenyl, iso-chromenyl, thioazinyl, thiophenyl, benzo-thiophenyl, dibenzo-thiophenyl, difuro-pyrazinyl, benzofuro-dibenzo-furanyl, benzothieno-benzo-thiophenyl, benzothieno-dibenzo-thiophenyl, benzothieno-benzo-furanyl, benzothieno-dibenzo-furanyl, xanthene-linked spiro acridinyl, dihydroacridinyl substituted with at least one C₁-C₁₀ alkyl and N-substituted spiro fluorenyl. The C₃-C₃₀ hetero arylene group can include, but is not limited to, any bivalent linking group corresponding to the hetero above aryl group.

As an example, each of the aromatic group (or aryl group) or the hetero aromatic group (or hetero aryl group) of R¹ to R⁴ in Chemical Formula 1 can consist of one to four aromatic and/or hetero aromatic rings. When the number of the aromatic and/or hetero aromatic rings of R¹ to R⁴ becomes more than four, conjugated structure among the within the whole molecule becomes too long, thus, the organometallic compound can have too narrow energy bandgap. For example, each of the aryl group or the hetero aryl group of R¹ to R⁴ can comprise independently, but is not limited to, phenyl, biphenyl, naphthyl, anthracenyl, pyrrolyl, triazinyl, imidazolyl, pyrazolyl, pyridinyl, pyrazinyl, pyrimidinyl, pyridazinyl, furanyl, benzo-furanyl, dibenzo-furanyl, thiophenyl, benzo-thiophenyl, dibenzo-thiophenyl, carbazolyl, acridinyl, carbolinyl, phenazinyl, phenoxazinyl, or phenothiazinyl.

As an example, the C₁₀-C₃₀ fused hetero aryl group with at least one nitrogen atom as R¹, R², R³ and/or R⁴ in Chemical Formula 1 can be selected from, but is not limited to, carbazolyl, benzo-carbazolyl, indeno-carbazolyl, indolo-carbazolyl, aza-carbazolyl (such as carbolinyl), acridinyl, phenazinyl, phenoxazinyl and phenothiazinyl each of which can be independently unsubstituted or substituted. For example, the C₁₀-C₃₀ fused hetero aryl group with at least one nitrogen atom can include, but is not limited to, carbazolyl, benzo-carbazolyl, indeno-carbazolyl and indolo-carbazolyl each of which is independently unsubstituted or substituted with at least one of deuterium, an unsubstituted or deuterium-substituted C₁-C₂₀ alkyl group, an unsubstituted or deuterium-substituted C₆-C₃₀ aryl group and an unsubstituted or deuterium-substituted C₃-C₃₀ hetero aryl group.

R¹ and R² is independently an unsubstituted or substituted C₆-C₃₀ aryl group or an unsubstituted or substituted C₃-C₃₀ hetero aryl group, preferably an unsubstituted or substituted C₆-C₁₈ or an unsubstituted or substituted C₆-C₁₈ hetero aryl group, more preferably an unsubstituted or substituted C₆-C₁₂ or an unsubstituted or substituted C₆-C₁₅ hetero aryl group, in particular phenyl, biphenyl, naphthyl, pyrrolyl, triazinyl, imidazolyl, pyrazolyl, pyridinyl, pyrimidinyl, benzo-furanyl, dibenzofuranyl, benzo-thiophenyl, dibenzothiophenyl, carbazolyl, or acridinyl, especially phenyl, biphenyl, dibenzofuranyl or carbazolyl.

R³ is independently deuterium, an unsubstituted or substituted C₁-C₂₀ alkyl group, an unsubstituted or substituted C₆-C₃₀ aryl group or an unsubstituted or substituted C₃-C₃₀ hetero aryl group, where each R³ is identical to or different from each other when m is 2, 3 or 4. Preferably R³ is independently deuterium, an unsubstituted or substituted C₁-C₄ alkyl group, an unsubstituted or substituted C₆-C₁₈ aryl group or an unsubstituted or substituted C₆-C₁₈ hetero aryl group, where each R³ is identical to or different from each other when m is 2, 3 or 4. In particular, R³ is deuterium.

R⁴ is hydrogen, deuterium, an unsubstituted or substituted C₁-C₂₀ alkyl group, an unsubstituted or substituted C₆-C₃₀ aryl group or an unsubstituted or substituted C₃-C₃₀ hetero aryl group, preferably hydrogen, deuterium, an unsubstituted or substituted C₁-C₄ alkyl group, an unsubstituted or substituted C₆-C₁₈ aryl group or an unsubstituted or substituted C₆-C₁₈ hetero aryl group, more preferably hydrogen, deuterium and an unsubstituted or substituted C₆-C₁₈ aryl group, in particular hydrogen, deuterium and phenyl.

Each of L¹ and L² is independently a single bond or an unsubstituted or substituted C₆-C₃₀ arylene group. In case that L¹ and/or L² is a single bond, R¹ and/or R² are bonded directly to the triazine moiety. Preferably each of L¹ and L² is independently a single bond or an unsubstituted or substituted C₆-C₁₈ arylene group, in particular L¹ and L² is independently a single bond or phenylene.

The organic compound having the structure of Chemical Formula 1 includes at least one fused hetero aromatic moiety linked to directly or via linking group (L¹ and/or L²) to the triazine moiety, and a benzothiazole moiety linked to the triazine moiety. The organic compound includes the triazine moiety of an electron acceptor and the fused hetero aromatic moiety of an electron donor moiety so as to accept both electrons and holes rapidly. In addition, the organic compound has higher singlet energy level (S₁) and triplet energy level (T₁) than those of an emitter, and relatively wide energy bandgap between HOMO (highest occupied molecular orbital) energy level and LUMO (lowest unoccupied molecular orbital) energy level.

In addition, the organic compound having the structure of Chemical Formula 1 has large dipole moment. As an example, the organic compound having the structure of Chemical Formula 1 can have about 0.2 or more, for example, between about 0.20 to about 2.5, of dipole moment.

When the organic compound having the structure of Chemical Formula 1 having large dipole moment is applied to a host in an emitting material layer, electrons can be injected from an electron transport layer to the emitting material layer through energy band bending caused by large dipole moment. Accordingly, an organic light emitting diode including the organic compound can lower its driving voltages and improve its luminous efficiency.

Moreover, the organic compound can include at least one of deuterium, a deuterium-substituted C₁-C₂₀ alkyl group, a deuterium-substituted C₆-C₃₀ aryl group and deuterium-substituted C₃-C₃₀ hetero aryl group linked to the core. The organic light emitting diode where the organic compound substituted with at least one of deuterium or deuterium-substituted groups are applied can improve its luminous lifetime.

In one example embodiment, the organic compound can be applied into an emissive layer, for example, an emitting material layer as a host so that an organic light emitting diode having beneficial luminous properties can be realized. The organic compound includes the benzothiazole moiety linked to the triazine moiety so as to improve further its electron transfer property. As an example, the organic compound can be applied into the emitting material layer as an N-type host, but is not limited thereto.

In one example embodiment, the benzothiazole moiety in Chemical Formula 1 can be linked to the triazine moiety through the benzene ring thereof. An organic compound such a molecular conformation can have the following structure of Chemical Formula 2: 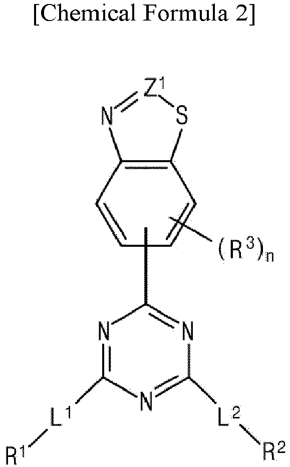 wherein, in the Chemical Formula 2,
each of R¹, R², R³, L¹ and L² is a same as defined in Chemical Formula 1;
Z¹ is CR⁴, where R⁴ is a same as defined in Chemical Formula 1; and
n is 0, 1, 2 or 3.

For example, the organic compound having the structure of Chemical Formula 2 can have the following structure of Chemical Formula 3A, Chemical Formula 3B, Chemical Formula 3C or Chemical Formula 3D: 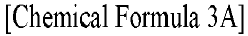 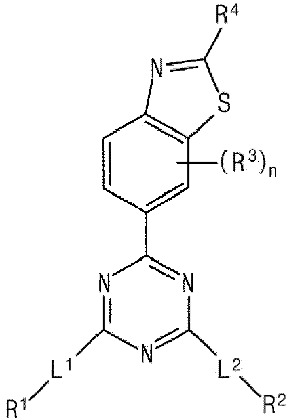 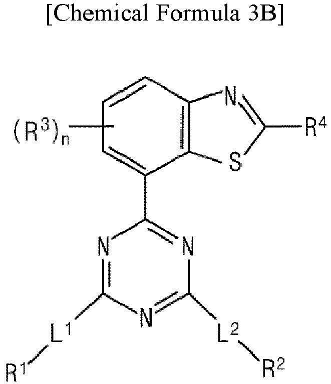 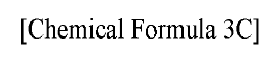 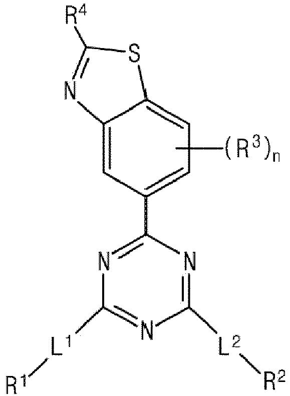 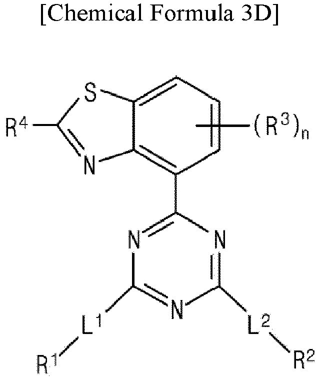 wherein, in the Chemical Formulae 3A, 3B, 3C and 3D,
each of R¹, R², R³, R⁴, L¹ and L² is a same as defined in Chemical Formula 1; and n is 0, 1, 2 or 3.

In another example embodiment, the benzothiazole moiety in Chemical Formula 1 can be linked to the triazine moiety through the thiazole ring thereof. The organic compound with such a molecular conformation can have the following structure of Chemical Formula 4: 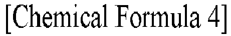 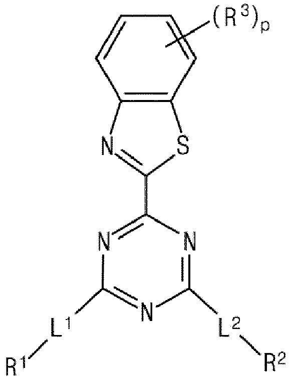 wherein, in the Chemical Formula 4,
each of R¹, R², R³, L¹ and L² is a same as defined in Chemical Formula 1; and p is 0, 1, 2, 3 or 4.

In another example embodiment, one of R¹ and R² in Chemical Formula 1 can be a carbazolyl group unsubstituted or substituted with deuterium or a C₁-C₂₀ alkyl group, where the C₁-C₂₀ alkyl group substituted to the carbazolyl group can be unsubstituted or substituted with deuterium, the other of R¹ and R² in Chemical Formula 1 can be selected from the group consisting of phenyl, biphenyl, pyrenyl, fluorenyl, dibenzofuranyl, dibenzothiophenyl and carbazolyl each of which can be independently unsubstituted or substituted with at least one of deuterium, a C₁-C₂₀ alkyl group and a C₆-C₃₀ aryl group, where each of the C₁-C₂₀ alkyl group and the C₆-C₃₀ aryl group substituted to R² can be independently unsubstituted or further substituted with deuterium, R³ in Chemical Formula 1 can be deuterium or a C₁-C₂₀ alkyl group unsubstituted or substituted with deuterium, R⁴ in Chemical Formula 1 can be phenyl unsubstituted or substituted with deuterium or a C₁-C₂₀ alkyl group, where the C₁-C₂₀ alkyl group substituted to the phenyl can be unsubstituted with deuterium, and m can be 0, 1, 2, 3 or 4.

More particularly, the organic compound having the structure of Chemical Formula 2A and 3A to 3D can be at least one of or selected from, but is not limited to the following compounds of Chemical Formula 5:

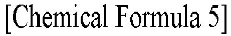

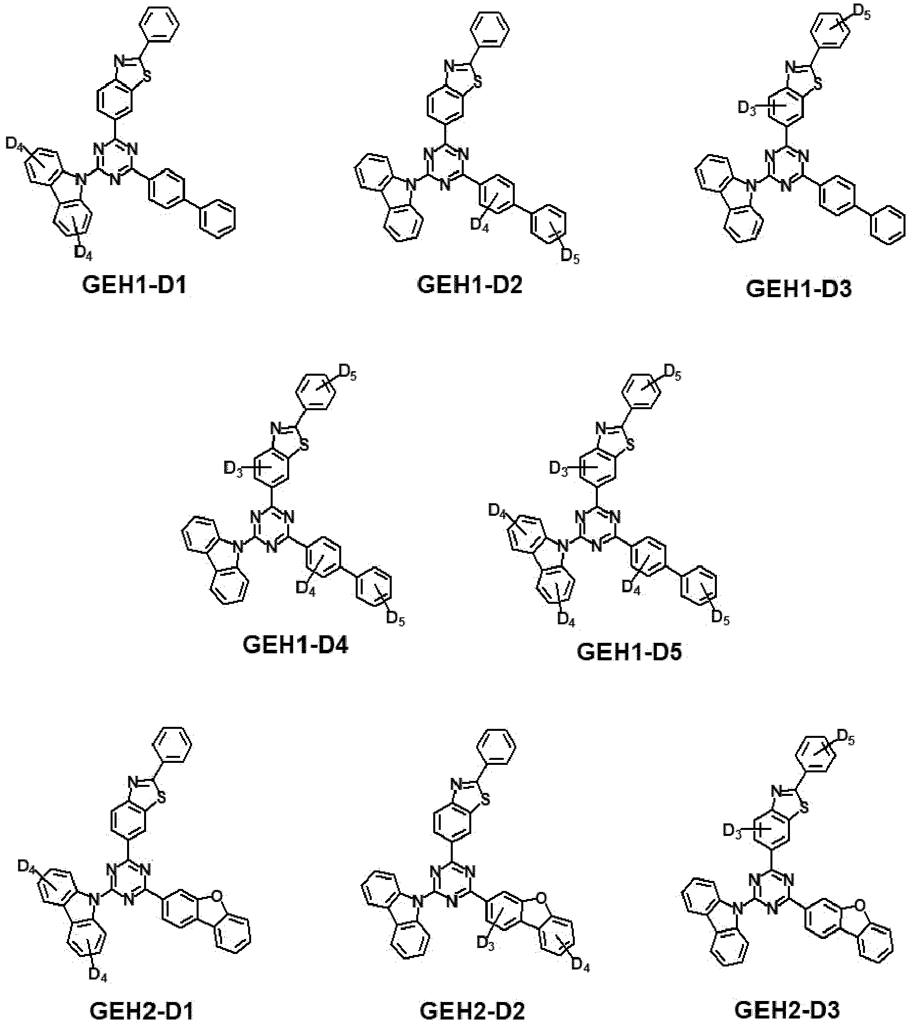

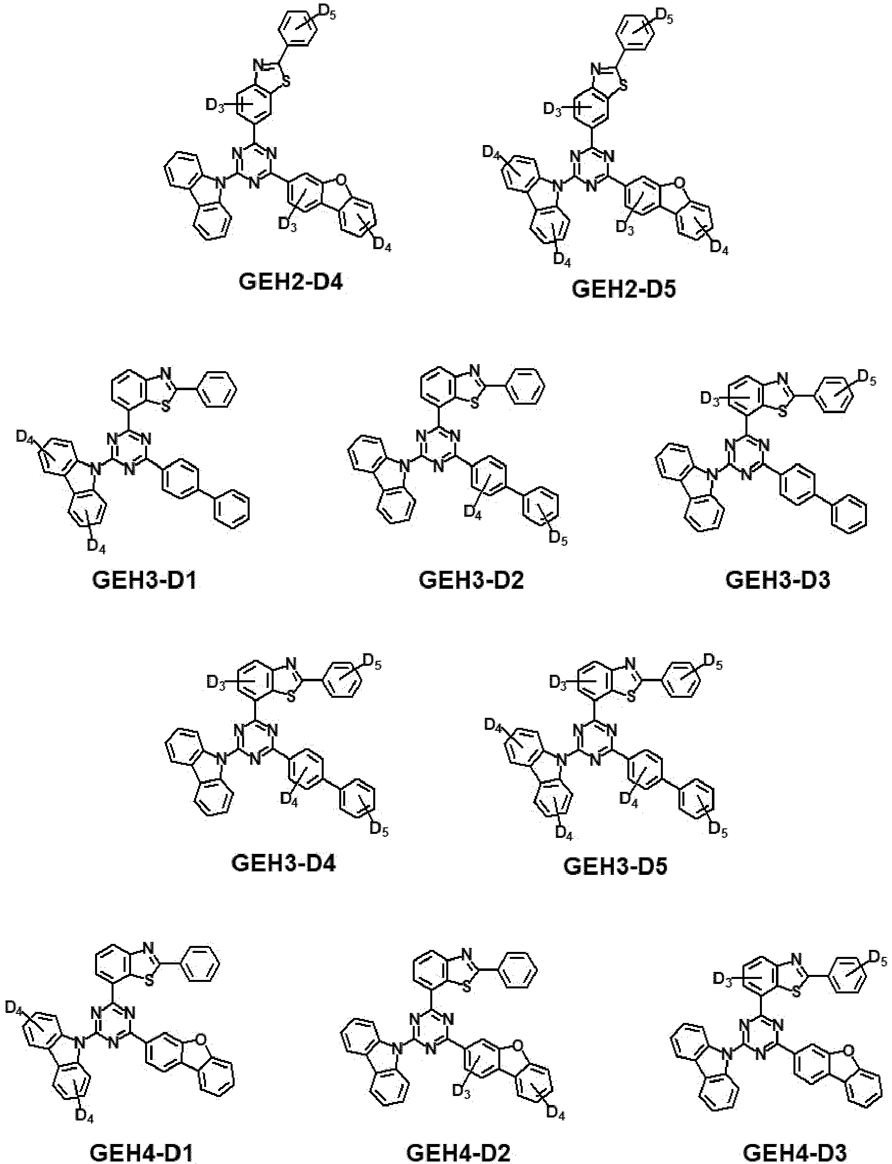

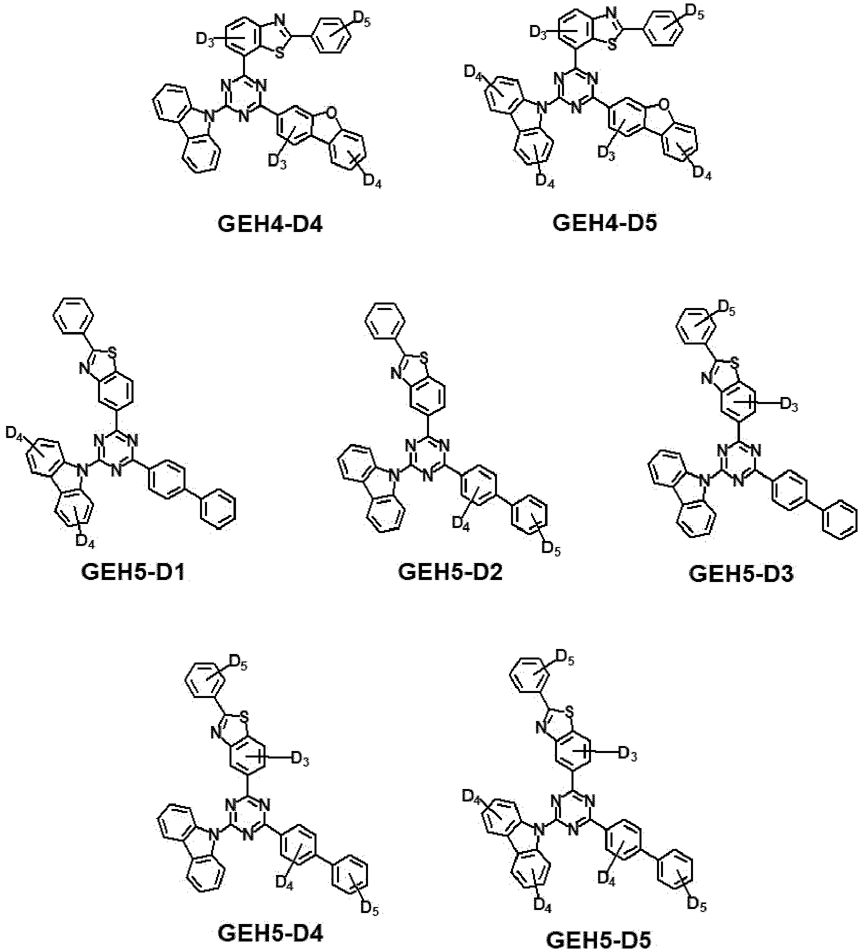

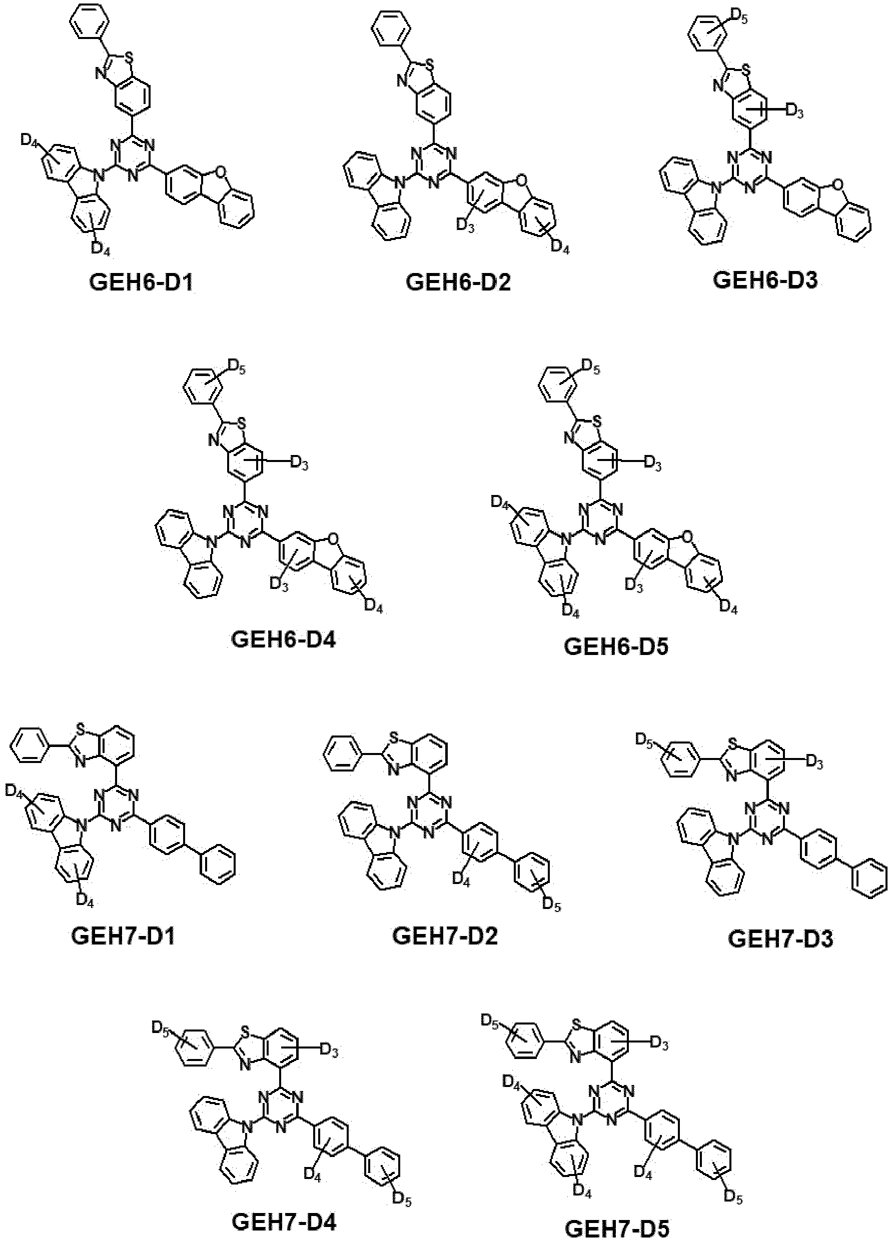

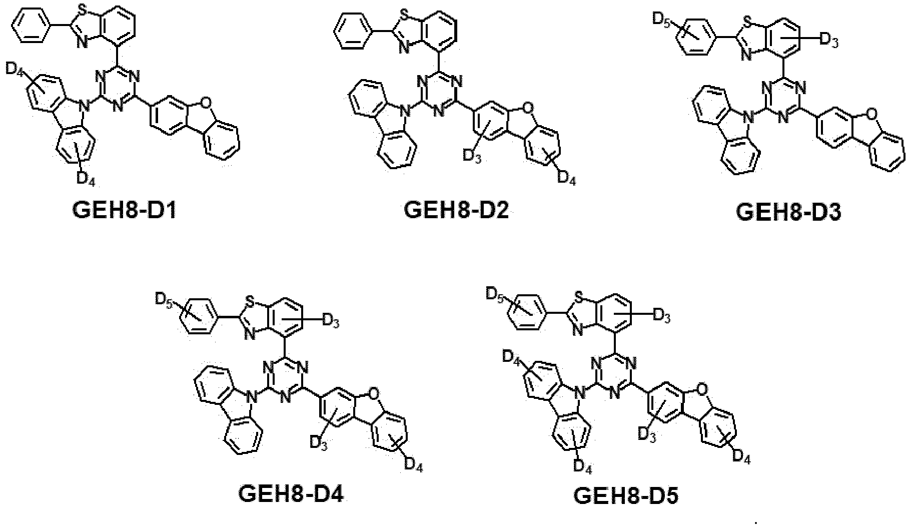

The organic compound having the structure of Chemical Formula 4 can be at least one of or selected from, but is not limited to, the following compounds of Chemical Formula 6:

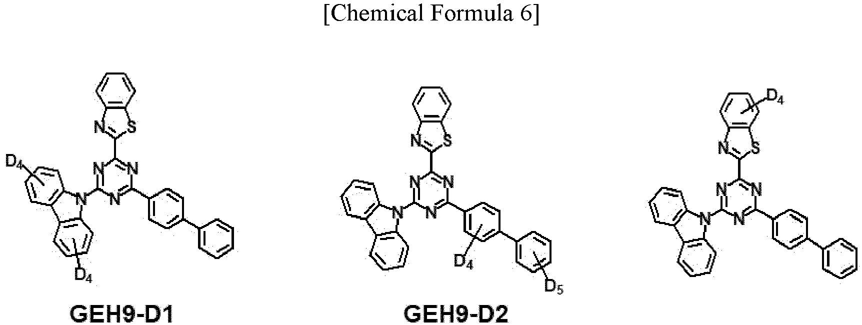

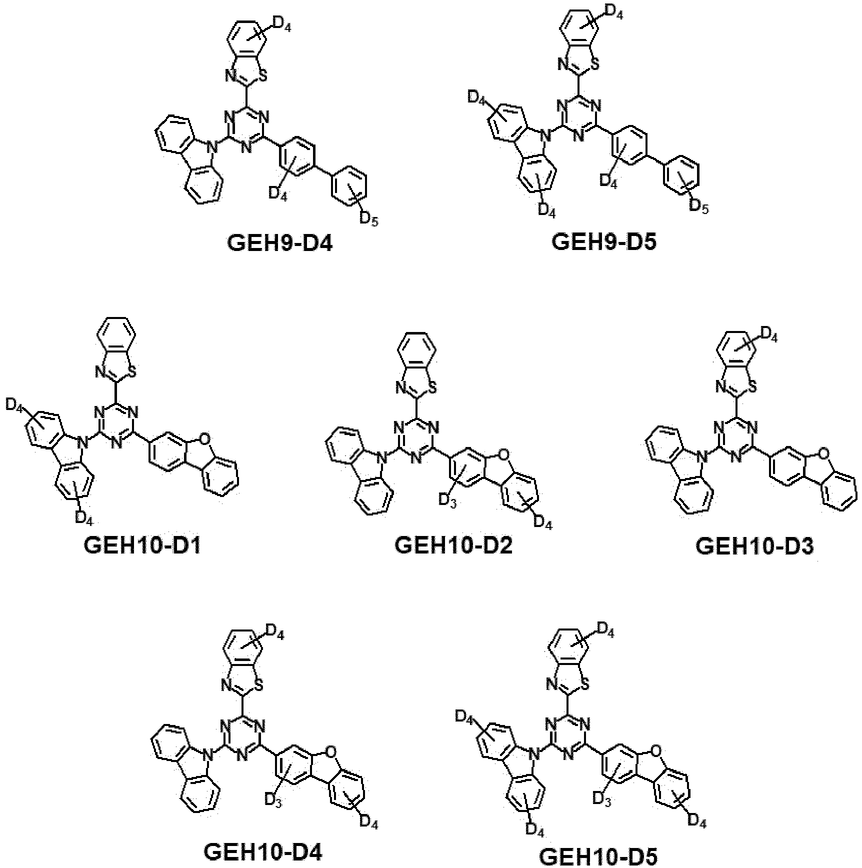

The organic compound having the structure of Chemical Formulae 1 to 6 includes the triazine moiety of electron donor and at least one fused hetero aromatic moiety of electron donor so that the organic compound can accept both electrons and holes rapidly. The benzothiazole moiety causes the dipole moment of the molecule to be improved, and thereby further improving electric transfer property of the molecule. In addition, as the organic compound includes at least one of deuterium and/or a deuterium-substituted group, the organic compound can have stable molecular conformation. Accordingly, it is possible to realize an organic light emitting diode with beneficial luminous efficiency and luminous lifetime by introducing the organic compound having the structure of Chemical Formulae 1 to 6 into an emissive layer.

### [Organic Light Emitting Diode and Organic Light Emitting Device]

The luminous efficiency and/or the luminous lifespan of an organic light emitting diode where the organic compound having the structure of Chemical Formulae 1 to 6 is applied to an emissive layer can be improved. As an example, the emissive layer including the organic compound having the structure of Chemical Formulae 1 to 6 can be applied to an organic light emitting diode with a single emitting part in a red pixel region, a green pixel region and/or a blue pixel region. Alternatively, the emissive layer including the organic compound having the structure of Chemical Formulae 1 to 6 can be applied to an organic light emitting diode of having a tandem structure where at least two emitting parts are stacked.

The organic light emitting diode where an emissive layer includes the organic compound having the structure of Chemical Formulae 1 to 6 can be applied to an organic light emitting device such as an organic light emitting display device or an organic light emitting illumination device. As an example, an organic light emitting display device will be described.

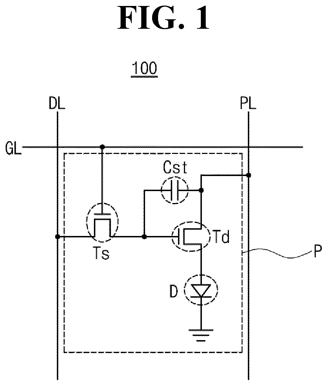

FIG. 1 illustrates a schematic circuit diagram of an organic light emitting display device in accordance with the present invention. As illustrated in FIG. 1, a gate line GL, a data line DL and power line PL, each of which crosses each other to define a pixel region P, in an organic light emitting display device 100. A switching thin film transistor Ts, a driving thin film transistor Td, a storage capacitor Cst and an organic light emitting diode D are disposed within the pixel region P. The pixel region P may include a red (R) pixel region, a green (G) pixel region and a blue (B) pixel region. However, embodiments of the present invention are not limited to such examples.

The switching thin film transistor Ts is connected to the gate line GL and the data line DL. The driving thin film transistor Td and the storage capacitor Cst are connected between the switching thin film transistor Ts and the power line PL. The organic light emitting diode D is connected to the driving thin film transistor Td. When the switching thin film transistor Ts is turned on by a gate signal applied to the gate line GL, a data signal applied to the data line DL is applied to a gate electrode of the driving thin film transistor Td and one electrode of the storage capacitor Cst through the switching thin film transistor Ts.

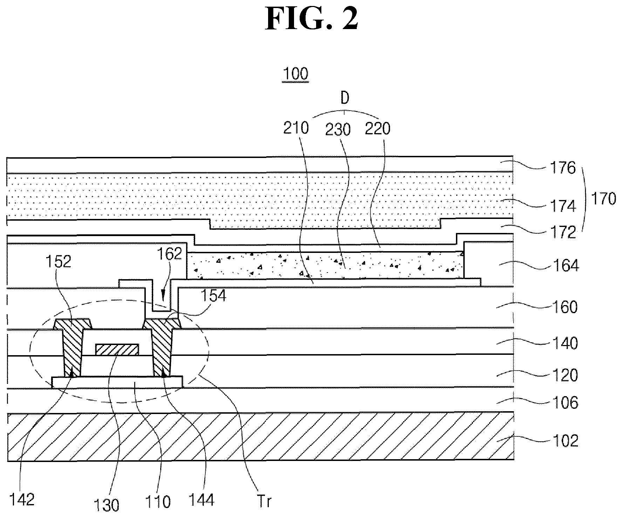

The driving thin film transistor Td is turned on by the data signal applied to the gate electrode 130 (FIG. 2) so that a current proportional to the data signal is supplied from the power line PL to the organic light emitting diode D through the driving thin film transistor Td. And then, the organic light emitting diode D emits light having a luminance proportional to the current flowing through the driving thin film transistor Td. In this case, the storage capacitor Cst is charged with a voltage proportional to the data signal so that the voltage of the gate electrode in the driving thin film transistor Td is kept constant during one frame. Therefore, the organic light emitting display device can display a desired image.

FIG. 2 illustrates a schematic cross-sectional view of an organic light emitting display device in accordance with an example embodiment of the present invention. As illustrated in FIG. 2, the organic light emitting display device 100 includes a substrate 102, a thin-film transistor Tr on the substrate 102, and an organic light emitting diode D connected to the thin film transistor Tr.

As an example, the substrate 102 can include a red pixel region, a green pixel region and a blue pixel region and an organic light emitting diode D can be located in each pixel region. Each of the organic light emitting diodes D emitting red, green and blue light, respectively, is located correspondingly in the red pixel region, the green pixel region and the blue pixel region.

The substrate 102 can include, but is not limited to, glass, thin flexible material and/or polymer plastics. For example, the flexible material can be selected from the group, but is not limited to, polyimide (PI), polyethersulfone (PES), polyethylenenaphthalate (PEN), polyethylene terephthalate (PET), polycarbonate (PC) and/or combinations thereof. The substrate 102, on which the thin film transistor Tr and the organic light emitting diode D are arranged, forms an array substrate.

A buffer layer 106 can be disposed on the substrate 102. The thin film transistor Tr can be disposed on the buffer layer 106. The buffer layer 106 can be omitted.

A semiconductor layer 110 is disposed on the buffer layer 106. In one example embodiment, the semiconductor layer 110 can include, but is not limited to, oxide semiconductor materials. In this case, a light-shield pattern may be disposed under the semiconductor layer 110, and the light-shield pattern can prevent light from being incident toward the semiconductor layer 110, and thereby, preventing or reducing the semiconductor layer 110 from being degraded by the light. Alternatively, the semiconductor layer 110 can include polycrystalline silicon. In this case, opposite edges of the semiconductor layer 110 can be doped with impurities.

A gate insulating layer 120 including an insulating material is disposed on the semiconductor layer 110. The gate insulating layer 120 can include, but is not limited to, an inorganic insulating material such as silicon oxide (SiOₓ, wherein 0 < x ≤ 2) or silicon nitride (SiNₓ, wherein 0 < x ≤ 2).

A gate electrode 130 made of a conductive material such as a metal is disposed on the gate insulating layer 120 so as to correspond to a center of the semiconductor layer 110. While the gate insulating layer 120 is disposed on a whole area of the substrate 102 as shown in FIG. 2, the gate insulating layer 120 may be patterned identically as the gate electrode 130.

An interlayer insulating layer 140 including an insulating material is disposed on the gate electrode 130 with and covers an entire surface of the substrate 102. The interlayer insulating layer 140 can include, but is not limited to, an inorganic insulating material such as silicon oxide (SiOₓ) or silicon nitride (SiNₓ), or an organic insulating material such as benzocyclobutene or photo-acryl.

The interlayer insulating layer 140 has first and second semiconductor layer contact holes 142 and 144 that expose or do not cover a portion of the surface nearer to the opposing ends than to a center of the semiconductor layer 110. The first and second semiconductor layer contact holes 142 and 144 are disposed on opposite sides of the gate electrode 130 and spaced apart from the gate electrode 130. The first and second semiconductor layer contact holes 142 and 144 are formed within the gate insulating layer 120 in FIG. 2. Alternatively, the first and second semiconductor layer contact holes 142 and 144 can be formed only within the interlayer insulating layer 140 when the gate insulating layer 120 is patterned identically as the gate electrode 130.

A source electrode 152 and a drain electrode 154, which are made of conductive material such as a metal, are disposed on the interlayer insulating layer 140. The source electrode 152 and the drain electrode 154 are spaced apart from each other on opposing sides of the gate electrode 130, and contact both sides of the semiconductor layer 110 through the first and second semiconductor layer contact holes 142 and 144, respectively.

The semiconductor layer 110, the gate electrode 130, the source electrode 152 and the drain electrode 154 constitute the thin film transistor Tr, which acts as a driving element. The thin film transistor Tr in FIG. 2 has a coplanar structure in which the gate electrode 130, the source electrode 152 and the drain electrode 154 are disposed on the semiconductor layer 110. Alternatively, the thin film transistor Tr can have an inverted staggered structure in which a gate electrode is disposed under a semiconductor layer and a source and drain electrodes are disposed on the semiconductor layer. In this case, the semiconductor layer can include amorphous silicon.

The gate line GL and the data line DL, which cross each other to define a pixel region P, and a switching element Ts, which is connected to the gate line GL and the data line DL, can be further formed in the pixel region P. The switching element Ts is connected to the thin film transistor Tr, which is a driving element. In addition, the power line PL is spaced apart in parallel from the gate line GL or the data line DL. The thin film transistor Tr may further include a storage capacitor Cst configured to constantly keep a voltage of the gate electrode 130 for one frame.

A passivation layer 160 is disposed on the source and drain electrodes 152 and 154. The passivation layer 160 covers the thin film transistor Tr on the whole substrate 102. The passivation layer 160 has a flat top surface and a drain contact hole 162 that exposes or does not cover the drain electrode 154 of the thin film transistor Tr. While the drain contact hole 162 is disposed on the second semiconductor layer contact hole 144, it may be spaced apart from the second semiconductor layer contact hole 144.

The organic light emitting diode (OLED) D includes a first electrode 210 that is disposed on the passivation layer 160 and connected to the drain electrode 154 of the thin film transistor Tr. The OLED D further includes an emissive layer 230 and a second electrode 220 each of which is disposed sequentially on the first electrode 210.

The first electrode 210 is disposed in each pixel region. The first electrode 210 can be an anode and include conductive material having relatively high work function value. For example, the first electrode 210 can include a transparent conductive oxide (TCO). More particularly, the first electrode 210 can include, but is not limited to, indium tin oxide (ITO), indium zinc oxide (IZO), indium tin zinc oxide (ITZO), tin oxide (SnO), zinc oxide (ZnO), indium cerium oxide (ICO), aluminum doped zinc oxide (AZO), and/or combinations thereof.

In one example embodiment, when the organic light emitting display device 100 is a bottom-emission type, the first electrode 210 can have a single-layered structure of the TCO. Alternatively, when the organic light emitting display device 100 is a top-emission type, a reflective electrode or a reflective layer may be disposed under the first electrode 210. For example, the reflective electrode or the reflective layer can include, but is not limited to, silver (Ag) or aluminum-palladium-copper (APC) alloy. In the OLED D of the top-emission type, the first electrode 210 can have a triple-layered structure of ITO/Ag/ITO or ITO/APC/ITO.

In addition, a bank layer 164 is disposed on the passivation layer 160 in order to cover edges of the first electrode 210. The bank layer 164 exposes or does not cover a center of the first electrode 210 corresponding to each pixel region. The bank layer 164 can be omitted.

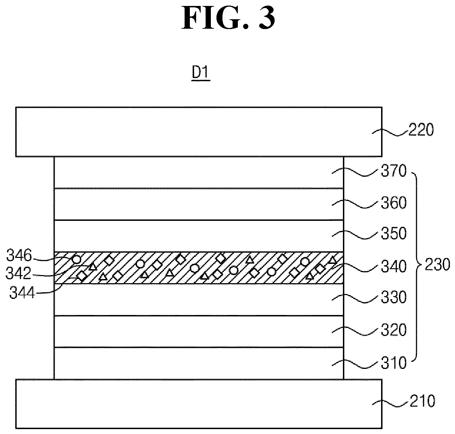

An emissive layer 230 is disposed on the first electrode 210. In one example embodiment, the emissive layer 230 can have a single-layered structure of an emitting material layer (EML). Alternatively, the emissive layer 230 can have a multiple-layered structure of a hole injection layer (HIL), a hole transport layer (HTL), an electron blocking layer (EBL), an EML, a hole blocking layer (HBL), an electron transport layer (ETL), an electron injection layer (EIL) and/or a charge generation layer (CGL) (FIG. 3). In one aspect, the emissive layer 230 can have a single emitting part. Alternatively, the emissive layer 230 can have multiple emitting parts to form a tandem structure. For example, the emissive layer 230 can be applied to an OLED with a single emitting part located each of the red pixel region, the green pixel region and the blue pixel region. Alternatively, the emissive layer 230 can be applied to a tandem-type OLED where at least two emitting parts are stacked.

The emissive layer 230 can include the organic compound having the structure of Chemical Formulae 1 to 6. The luminous efficiency and the luminous lifespan of the OLED D and the organic light emitting display device 100 can be improved by including the organic compound having the structure of Chemical Formulae 1 to 6.

The second electrode 220 is disposed on the substrate 102 above which the emissive layer 230 is disposed. The second electrode 220 can be disposed on a whole display area. The second electrode 220 can include a conductive material with a relatively low work function value compared to the first electrode 210. The second electrode 220 can be a cathode providing electrons. For example, the second electrode 220 can include at least one of, but is not limited to, aluminum (Al), magnesium (Mg), calcium (Ca), silver (Ag), alloy thereof and/or combinations thereof such as aluminum-magnesium alloy (Al-Mg). When the organic light emitting display device 100 is a top-emission type, the second electrode 220 is thin so as to have light-transmissive (semi-transmissive) property.

In addition, an encapsulation film 170 can be disposed on the second electrode 220 in order to prevent or reduce outer moisture from penetrating into the OLED D. The encapsulation film 170 can have, but is not limited to, a laminated structure of a first inorganic insulating film 172, an organic insulating film 174 and a second inorganic insulating film 176. The encapsulation film 170 can be omitted.

A polarizing plate can be attached onto the encapsulation film 170 to reduce reflection of external light. For example, the polarizing plate may be a circular polarizing plate. When the organic light emitting display device 100 is a bottom-emission type, the polarizing plate can be disposed under the substrate 102. Alternatively, when the organic light emitting display device 100 is a top-emission type, the polarizing plate can be disposed on the encapsulation film 170. In addition, a cover window can be attached to the encapsulation film 170 or the polarizing plate. In this case, the substrate 102 and the cover window may have a flexible property, thus the organic light emitting display device 100 may be a flexible display device.

The OLED D is described in more detail. FIG. 3 illustrates a schematic cross-sectional view of an organic light emitting diode having a single emitting part in accordance with an example embodiment of the present invention. As illustrated in FIG. 3, the organic light emitting diode (OLED) D1 in accordance with the present invention includes first and second electrodes 210 and 220 facing each other and an emissive layer 230 disposed between the first and second electrodes 210 and 220. The organic light emitting display device 100 includes a red pixel region, a green pixel region and a blue pixel region, and the OLED D1 can be disposed in the red pixel region, the green pixel region and the blue pixel region. As an example, the OLED D1 can be disposed in the green pixel region.

In an example embodiment, the emissive layer 230 includes an emitting material layer (EML) 340 disposed between the first and second electrodes 210 and 220. Also, the emissive layer 230 can include at least one of a hole transport layer (HTL) 320 disposed between the first electrode 210 and the EML 340 and an electron transport layer (ETL) 360 disposed between the second electrode 220 and the EML 340. In addition, the emissive layer 230 can further include at least one of a hole injection layer (HIL) 310 disposed between the first electrode 210 and the HTL 320 and an electron injection layer (EIL) 370 disposed between the second electrode 220 and the ETL 360. Alternatively, the emissive layer 230 can further comprise a first exciton blocking layer, *i.e.* an electron blocking layer (EBL) 330 disposed between the HTL 320 and the EML 340 and/or a second exciton blocking layer, *i.e.* a hole blocking layer (HBL) 350 disposed between the EML 340 and the ETL 360.

The first electrode 210 can be an anode that provides holes into the EML 340. The first electrode 210 can include a conductive material having a relatively high work function value, for example, a transparent conductive oxide (TCO). In an example embodiment, the first electrode 210 can include, but is not limited to, ITO, IZO, ITZO, SnO, ZnO, ICO, AZO, and/or combinations thereof.

The second electrode 220 can be a cathode that provides electrons into the EML 340. The second electrode 220 can include a conductive material having a relatively low work function values, i.e., a highly reflective material such as Al, Mg, Ca, Ag, and/or alloy thereof and/or combinations thereof such as Al-Mg.

The HIL 310 is disposed between the first electrode 210 and the HTL 320 and can improve an interface property between the inorganic first electrode 210 and the organic HTL 320. In one example embodiment, the HIL 310 can include, but is not limited to, 4,4',4"-Tris(3-methylphenylamino)triphenylamine (MTDATA), 4,4',4"-Tris(N,N-diphenyl-amino)triphenylamine (NATA), 4,4',4"-Tris(N-(naphthalene-1-yl)-N-phenyl-amino)triphenylamine (1T-NATA), 4,4',4"-Tris(N-(naphthalene-2-yl)-N-phenyl-amino)triphenylamine (2T-NATA), Copper phthalocyanine (CuPc), Tris(4-carbazoyl-9-yl-phenyl)amine (TCTA), N,N'-Diphenyl-N,N'-bis(1-naphthyl)-1,1'-biphenyl-4,4"-diamine (NPB; NPD), N,N'-Bis{4-[bis(3-methylphenyl)amino]phenyl}-N,N'-diphenyl-4,4'-biphenyldiamine (DNTPD), 1,4,5,8,9,11-Hexaazatriphenylenehexacarbonitrile (Dipyrazino[2,3-f:2'3'-h] quinoxaline-2,3,6,7,10,11-hexacarbonitrile; HAT-CN), 1,3,5-tris[4-(diphenylamino)phenyl]benzene (TDAPB), poly(3,4-ethylenedioxythiphene)polystyrene sulfonate (PEDOT/PSS), N-(biphenyl-4-yl)-9,9-dimethyl-N-(4-(9-phenyl-9H-carbazol-3-yl)phenyl)-9H-fluoren-2-amine, N,N'-diphenyl-N,N'-di[4-(N,N'-diphenyl-amino)phenyl]benzidine (NPNPB) and/or combinations thereof. The HIL 310 can be omitted in compliance of the OLED D1 property. Preferred are N,N'-Diphenyl-N,N'-bis(1-naphthyl)-1,1'-biphenyl-4,4"-diamine (NPB; NPD), and 1,4,5,8,9,11-Hexaazatriphenylenehexacarbonitrile (Dipyrazino[2,3-f:2'3'-h]quinoxaline-2,3,6,7,10,11-hexacarbonitrile; HAT-CN).

The HTL 320 is disposed adjacently to the EML 340 between the first electrode 210 and the EML 340. In one example embodiment, the HTL 320 can include, but is not limited to, N,N'-Diphenyl-N,N'-bis(3-methylphenyl)-1,1'-biphenyl-4,4'-diamine (TPD), NPB(NPD), DNTPD, 4,4'-bis(N-carbazolyl)-1,1' -biphenyl (CBP), Poly[N,N'-bis(4-butylpnehyl)-N,N' -bis(phenyl)-benzidine] (Poly-TPD), Poly[(9,9-dioctylfluorenyl-2,7-diyl)-co-(4,4'-(N-(4-sec-butylphenyl)diphenylamine))] (TFB), Di-[4-(N,N-di-p-tolyl-amino)-phenyl]cyclohexane (TAPC), 3,5-Di(9H-carbazol-9-yl)-N,N-diphenylaniline (DCDPA), N-(biphenyl-4-yl)-9,9-dimethyl-N-(4-(9-phenyl-9H-carbazol-3-yl)phenyl)-9H-fluoren-2-amine, N-(biphenyl-4-yl)-N-(4-(9-phenyl-9H-carbazol-3-yl)phenyl)biphenyl-4-amine), N-([1,1'-Biphenyl]-4-yl)-9,9-dimethyl-N-(4-(9-phenl-9H-carbazol-3-yl)phenyl)-9H-fluoren-2-amine and/or combinations thereof. Preferred is N,N'-Diphenyl-N,N'-bis(1-naphthyl)-1,1'-biphenyl-4,4"-diamine (NPB; NPD).

The EML 340 can include a host 342 and/or 346 and a dopant (emitter) 346. For example, the EML 340 includes a first host 342, a second host 346 and the dopant 346 where ultimate light emission occurs. The EML 340 can emit red, green and/or blue color light.

The first host 342 can be an N-type host (electron-type host) with relatively beneficial electron affinity compared to the second host 344. The first host 342 includes the organic compound having the structure of Chemical Formulae 1 to 6.

The second host 344 can be a P-type host (hole-type host) with relatively beneficial hole affinity compared to the first host 342. As an example, the second host 344 can include, but is not limited to, a biscarbazole-based organic compound, an aryl amine- or a hetero aryl amine-based organic compound with at least one fused aromatic and/or fused hetero aromatic moiety, and/or an aryl amine- or a hetero aryl amine-based organic compound with a spirofluorene moiety.

For example, the second host 344 which can be used together with the dopant 346 can include, but is not limited to, 9-(3-(9H-carbazol-9-yl)phenyl)-9H-carbazole-3-carbonitrile (mCP-CN), CBP, 3,3'-bis(N-carbazolyl)-1,1'-biphenyl (mCBP), 1,3-Bis(carbazol-9-yl)benzene (mCP), Bis [2-(diphenylphosphino)phenyl] ether oxide (DPEPO), 2,8-bis(diphenylphosphoryl)dibenzothiophene (PPT), 1,3,5-Tri[(3 -pyridyl)-phen-3 -yl] benzene (TmPyPB), 2,6-Di(9H-carbazol-9-yl)pyridine (PYD-2Cz), 2,8-di(9H-carbazol-9-yl)dibenzothiophene (DCzDBT), 3',5'-Di(carbazol-9-yl)-[1,1' -bipheyl] -3,5-dicarbonitrile (DCzTPA), 4'-(9H-carbazol-9-yl)biphenyl-3,5-dicarbonitrile (pCzB-2CN), 3'-(9H-carbazol-9-yl)biphenyl-3,5-dicarbonitrile (mCzB-2CN), Diphenyl-4-triphenylsilyl-phenylphosphine oxide (TSPO1), 9-(9-phenyl-9H-carbazol-6-yl)-9H-carbazole (CCP), 4-(3-(triphenylen-2-yl)phenyl)dibenzo[b,d]thiophene, 9-(4-(9H-carbazol-9-yl)phenyl)-9H-3,9'-bicarbazole, 9-(3-(9H-carbazol-9-yl)phenyl)-9H-3,9'-bicarbazole, 9-(6-(9H-carbazol-9-yl)pyridin-3-yl)-9H-3,9'-bicabazole, 9,9'-Diphenyl-9H,9'H-3,3'-bicarbazole (BCzPh), 9,9'-Di(4-biphenyl)-9H,9'H-3,3'-bicabazole (BCZ), 1,3,5-Tris(carbazole-9-yl)benzene (TCP), TCTA, 4,4'-Bis(carbazole-9-yl)-2,2'-dimethylbipheyl (CDBP), (2,7-Bis(carbazole-9-yl)-9,9-dimethylfluorene (DMFL-CBP), 2,2',7,7'-Tetrakis(carbazole-9-yl)-9,9-spiorofluorene (Spiro-CBP), 3,6-Bis(carbazole-9-yl)-9-(2-ethyl-hexyl)-9H-carbazole (TCz1) and/or combinations thereof. Preferred are 9,9'-Diphenyl-9H,9'H-3,3'-bicarbazole (BCzPh) and 9,9'-Di(4-biphenyl)-9H,9'H-3,3'-bicabazole (BCZ), in particular 9,9'-Di(4-biphenyl)-9H,9'H-3,3'-bicabazole (BCZ.)

In one example embodiment, the first host 342, which can be the organic compound haivng the structure of Chemical Formulae 1 to 6, can have a highest occupied molecualr orbital (HOMO) energy level lower than a HOMO energy level of the second host 344 that can be the P-type host.

The dopant 346 can include a blue dopant, a green dopant and/or a red dopant. The blue dopant can include at least one of a blue phorescent compound, a blue fluorescent compound and a blue delayed fluorescent compound. The green dopant can include a green phosphorescent compound, a green fluorescent compound and a green delayed fluorescent compound. The red doant can include a red phosphorescent compound, a red fluorescent compound and a red delayed fluorescent compound.

The contents of the host 342 and/or 344 in the EML 340 can be about 50 wt% to about 99 wt%, for example, about 80 wt% to about 95 wt%, and the contents of the dopant 346 in the EML 340 can be about 1 wt% to about 50 wt%, for example, about 5 wt% to about 20 wt%, but is not limited thereto. When the EML 340 includes both the first host 342 and the second host 344, the first host 342 and the second host 344 can be admixed, but is not limited to, with a weight ratio of about 4:1 to aobut 1:4, for example about 3:1 to about 1:3.

The ETL 360 and the EIL 370 can be laminated sequentially between the EML 340 and the second electrode 220. An electron transporting material included in the ETL 360 has high electron moibity so as to provide electrons stably with the EML 340 by fast electron transportation.

In one example embodiment, the ETL 360 can include at least one of oxadiazole-based compounds, triazole-based compounds, phenanthroline-based compounds, benzoxazole-based compounds, benzothiazole-based compounds, benzimidazole-baed compounds and traizine-based compounds.

More particularly, the ETL 360 can inlcude, but is not limited to, tris-(8-hydroxyquinoline aluminum (Alq₃), 2-biphenyl-4-yl-5-(4-t-butylphenyl)-1,3,4-oxadiazole (PBD), spiro-PBD, lithium quinolate (Liq), 1,3,5-Tris(N-phenylbenzimidazol-2-yl)benzene (TPBi), Bis(2-methyl-8-quinolinolato-N1,O8)-(1,1'-biphenyl-4-olato)aluminum (BAlq), 4,7-diphenyl-1,10-phenanthroline (Bphen), 2,9-Bis(naphthalene-2-yl)4,7-diphenyl-1,10-phenanthroline (NBphen), 2,9-Dimethyl-4,7-diphenyl-1,10-phenathroline (BCP), 3-(4-Biphenyl)-4-phenyl-5-tert-butylphenyl-1,2,4-triazole (TAZ), 4-(Naphthalen-1-yl)-3,5-diphenyl-4H-1,2,4-triazole (NTAZ), 1,3,5-Tri(p-pyrid-3-yl-phenyl)benzene (TpPyPB), 2,4,6-Tris(3'-(pyridin-3-yl)biphenyl-3-yl)1,3,5-triazine (TmPPPyTz), Poly[9,9-bis(3'-((N,N-dimethyl)-N-ethylammonium)-propyl)-2,7-fluorene]-alt-2,7-(9,9-dioctylfluorene)] (PFNBr), tris(phenylquinoxaline (TPQ), TSPO1, 2-[4-(9,10-Di-2-naphthalen2-yl-2-anthracen-2-yl)phenyl]-1-phenyl-1H-benzimidazole (ZADN), at least one electron transporting materials represented by Chemical Formula 7 and/or combinations thereof, in particular at least one electron transporting materials represented by Chemical Formula 7

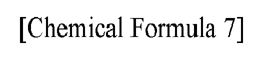

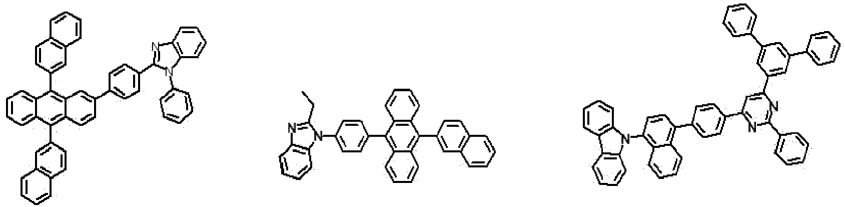

In addition, other benzimidazole-based electron transporting materials and/or other azine-based electron transporting materials can be used, but is not limited thereto.

In another example embodiment, the ETL 360 can include a first electron transporting material and a second electron transporting material. The first electron transporting material and the second electron transporting material in the ETL 360 can be admixed to, but is not limited to, a weight ratio of about 7:3 to about 3:7, for example, about 6:4 to about 4:6.

In still another example embodiment, the energy level bandgap between a lowest unoccupied molecular orbital (LUMO) energy level of the first host 342 of the organic compound having the structure of Chemical Formulae 1 to 6 in the EML 340 and a LUMO energy level of the electron transporting material can be about 0.5 eV or less, for example, about 0.4 eV or less. In this case, electrons can be injected rapidly from the ETL 360 to the EML 340.

As described above, the organic compound having the structure of Chemical Formulae 1 to 6 has large dipole moment. When the ETL 360 includes an electron transporting material with large dipole moment, electrons can be injected further rapidly from the ETL 360 to the EML 340 resulting from band-bending between the energy levels of the EML 340 and the ETL 360. As an example, the electron transporting material in the ETL 360 can have, but is not limited to, dipole moment between about 1.5 to about 9.0, for example, between about 1.5 to about 6.0.

In another example embodiment, the singlet and/or triplet energy level of the electron transporting material can be higher than the singlet and/or triplet energy level of the dopant 346. For example, when the EML 340 includes a phosphorescent dopant 346 that can use triplet exciton energy, the electron transporting material can have triplet energy level higher than the triplet energy level of the phosphorescent dopant 346.

The exciton generated in the EML 340 is defined with the EML 340, and therefore, exciton energy can be transferred from the host 342 and/or 346 to only dopant 346. As the exciton energy transfer path is defined, there is little possibility of exciton energy quenching through other transfer paths (e.g., from dopant to host, EML to other common layers). The generated excitons are emitted at the dopant 346 so that the OLED D1 can implement highly beneficial luminous efficiency.

In another example embodiment, the HOMO energy level of the electron transporting material can be lower than HOMO energy levels of the second host 344 of the P-type host and/or the dopant 346. The HOMO energy level of the first host 342 having the structure of Chemical Formulae 1 to 6 can be lower than the HOMO energy level of the second host 344, as described above.

The holes injected into the EML 340 can be defined with the EML 340 by designing the HOMO energy levels of the first host 342 and electron transporting material to be lower than the HOMO energy levels of the second host 344 and the dopant 346. It is possible to minimize non-radiative quenching of excitons by preventing holes from leaking to the adjacent common layers.

The EII, 370 is disposed between the second electrode 220 and the ETL 360, and can improve physical properties of the second electrode 220 and therefore, can enhance the lifespan of the OLED D1. In one example embodiment, the EIL 370 can include, but is not limited to, an alkali metal halide or an alkaline earth metal halide such as LiF, CsF, NaF, BaF₂ and the like, and/or an organometallic compound such as Liq, lithium benzoate, sodium stearate, and the like. Alternatively, the EIL 370 can be omitted.

When holes are transferred to the second electrode 220 via the EML 340 and/or electrons are transferred to the first electrode 210 via the EML 340, the OLED D1 can have short lifespan and reduced luminous efficiency. In order to prevent those phenomena, the OLED D1 in accordance with this aspect of the present invention can have at least one exciton blocking layer adjacent to the EML 340.

As an example, the OLED D1 can include the EBL 330 between the HTL 320 and the EML 340 so as to control and prevent electron transfers. In one example embodiment, the EBL 330 can include, but is not limited to, TCTA, Tris[4-(diethylamino)phenyl]amine, N-(biphenyl-4-yl)-9,9-dimethyl-N-(4-(9-phenyl-9H-carbazol-3-yl)phenyl)-9H-fluorene-2-amine, TAPC, MTDATA, mCP, mCBP, CuPc, DNTPD, TDAPB, DCDPA, 2,8-bis(9-phenyl-9H-carbazol-3-yl)dibenzo[b,d]thiophene and/or combinations thereof.

In addition, the OLED D1 can further include the HBL 350 as a second exciton blocking layer between the EML 340 and the ETL 360 so that holes cannot be transferred from the EML 340 to the ETL 360. In one example embodiment, the HBL 350 can include, but is not limited to, at least one of oxadiazole-based compounds, triazole-based compounds, phenanthroline-based compounds, benzoxazole-based compounds, benzothiazole-based compounds, benzimidazole-based compounds, and triazine-based compounds.

For example, the HBL 350 can include material having a relatively low HOMO energy level compared to the luminescent materials in EML 340. The HBL 350 can include, but is not limited to, BCP, BAlq, Alq₃, PBD, spiro-PBD, Liq, Bis-4,5-(3,5-di-3-pyridylphenyl)-2-methylpyrimidine (B3PYMPM), DPEPO, 9-(6-(9H-carbazol-9-yl)pyridine-3-yl)-9H-3,9'-bicarbazole, TSPO1 and/or combinations thereof.

As described above, the EML 340 includes a host 342 and/or 346 and a dopant 342, and the first host 342 can include an organic compound having the structure of Chemical Formulae 1 to 6.

The organic compound having the structure of Chemical Formulae 1 to 6 has excellent affinity to both holes and electrons. The organic compound having the structure of Chemical Formulae 1 to 6 with the benzothiazole moiety has further improved electron affinity. The organic compound has higher single energy level and triplet energy level and wider energy bandgap between HOMO energy level and the LUMO energy level compared to the dopant 346.

Also, the organic compound with large dipole moment shows excellent affinity to charges. In addition, the organic compound includes at least one of deuterium and/or a deuterium-substituted group so as to maintain stable chemical conformation. Accordingly, it is possible to improve the luminous efficiency and luminous lifetime of the OLED D1 by applying the organic compound having the structure of Chemical Formulae 1 to 6 into the first host 342 in the EML 340.

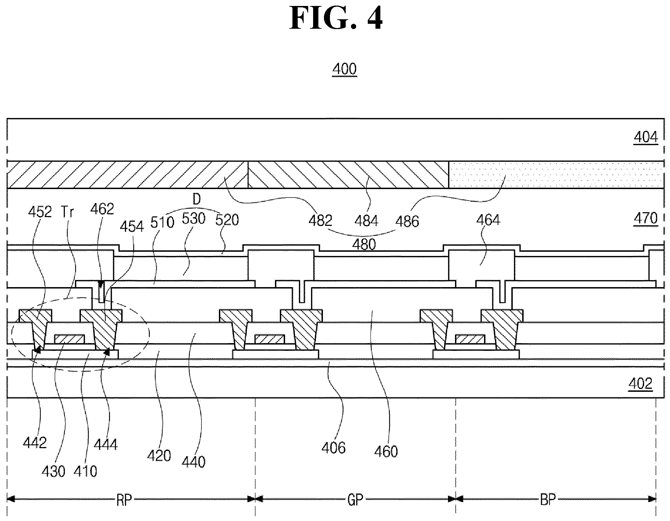

The organic light emitting device and the OLED D1 with a single emitting part are shown in FIGS. 2 and 3. In another example embodiment, an organic light emitting display device can implement full-color including white color. FIG. 4 illustrates a schematic cross-sectional view of an organic light emitting display device in accordance with another example embodiment of the present invention.

As illustrated in FIG. 4, the organic light emitting display device 400 includes a first substrate 402 that defines each of a red pixel region RP, a green pixel region GP and a blue pixel region BP, a second substrate 404 facing the first substrate 402, a thin film transistor Tr on the first substrate 402, an OLED D disposed between the first and second substrates 402 and 404 and emitting white (W) light and a color filter layer 480 disposed between the OLED D and the second substrate 404.

Each of the first and second substrates 402 and 404 can include, but is not limited to, glass, flexible material and/or polymer plastics. For example, each of the first and second substrates 402 and 404 can be made of PI, PES, PEN, PET, PC and/or combinations thereof. The second substrate 404 can be omitted. The first substrate 402, on which a thin film transistor Tr and the OLED D are arranged, forms an array substrate.

A buffer layer 406 can be disposed on the first substrate 402. The thin film transistor Tr is disposed on the buffer layer 406 correspondingly to each of the red pixel region RP, the green pixel region GP and the blue pixel region BP. The buffer layer 406 can be omitted.

A semiconductor layer 410 is disposed on the buffer layer 406. The semiconductor layer 410 can be made of or include oxide semiconductor material or polycrystalline silicon.

A gate insulating layer 420 including an insulating material, for example, inorganic insulating material such as silicon oxide (SiOₓ, wherein 0 < x ≤ 2) or silicon nitride (SiNₓ, wherein 0 < x ≤ 2) is disposed on the semiconductor layer 410.

A gate electrode 430 made of a conductive material such as a metal is disposed over the gate insulating layer 420 so as to correspond to a center of the semiconductor layer 410. An interlayer insulating layer 440 including an insulating material, for example, inorganic insulating material such as SiOₓ or SiNₓ (wherein 0 < x ≤ 2), or an organic insulating material such as benzocyclobutene or photo-acryl, is disposed on the gate electrode 430.

The interlayer insulating layer 440 has first and second semiconductor layer contact holes 442 and 444 that expose or do not cover a portion of the surface nearer to the opposing ends than to a center of the semiconductor layer 410. The first and second semiconductor layer contact holes 442 and 444 are disposed on opposite sides of the gate electrode 430 with spacing apart from the gate electrode 430.

A source electrode 452 and a drain electrode 454, which are made of or include a conductive material such as a metal, are disposed on the interlayer insulating layer 440. The source electrode 452 and the drain electrode 454 are spaced apart from each other with respect to the gate electrode 430. The source electrode 452 and the drain electrode 454 contact both sides of the semiconductor layer 410 through the first and second semiconductor layer contact holes 442 and 444, respectively.

The semiconductor layer 410, the gate electrode 430, the source electrode 452 and the drain electrode 454 constitute the thin film transistor Tr, which acts as a driving element.

Although not shown in FIG. 4, the gate line GL and the data line DL, which cross each other to define the pixel region P, and a switching element Ts, which is connected to the gate line GL and the data line DL, can be further formed in the pixel region P. The switching element Ts is connected to the thin film transistor Tr, which is a driving element. In addition, the power line PL is spaced apart in parallel from the gate line GL or the data line DL, and the thin film transistor Tr can further include the storage capacitor Cst configured to constantly keep a voltage of the gate electrode 430 for one frame.

A passivation layer 460 is disposed on the source electrode 452 and the drain electrode 454 and covers the thin film transistor Tr over the whole first substrate 402. The passivation layer 460 has a drain contact hole 462 that exposes or does not cover the drain electrode 454 of the thin film transistor Tr.

The OLED D is located on the passivation layer 460. The OLED D includes a first electrode 510 that is connected to the drain electrode 454 of the thin film transistor Tr, a second electrode 520 facing the first electrode 510 and an emissive layer 530 disposed between the first and second electrodes 510 and 520.

The first electrode 510 formed for each pixel region RP, GP or BP can be an anode and can include a conductive material having relatively high work function value. For example, the first electrode 510 can include, but is not limited to, ITO, IZO, ITZO, SnO, ZnO, ICO, AZO, and/or combinations thereof. Alternatively, a reflective electrode or a reflective layer can be disposed under the first electrode 510. For example, the reflective electrode or the reflective layer can include, but is not limited to, Ag or APC alloy.

A bank layer 464 is disposed on the passivation layer 460 in order to cover edges of the first electrode 510. The bank layer 464 exposes or does not cover a center of the first electrode 510 corresponding to each of the red pixel RP, the green pixel GP and the blue pixel BP. The bank layer 464 can be omitted.

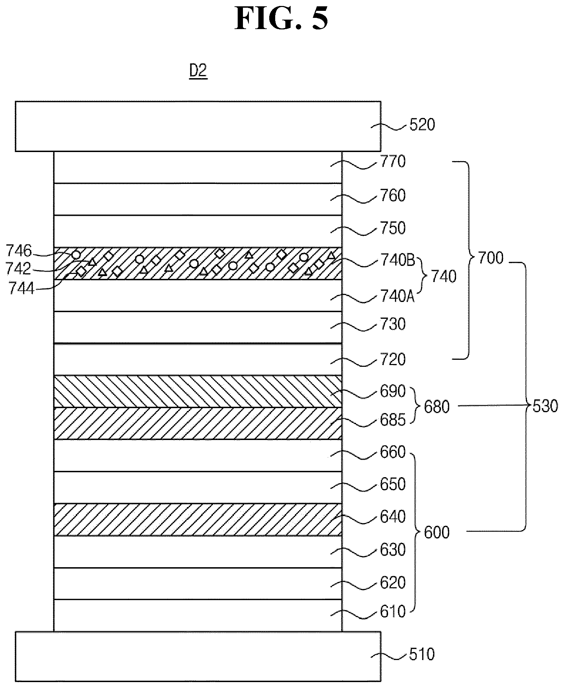

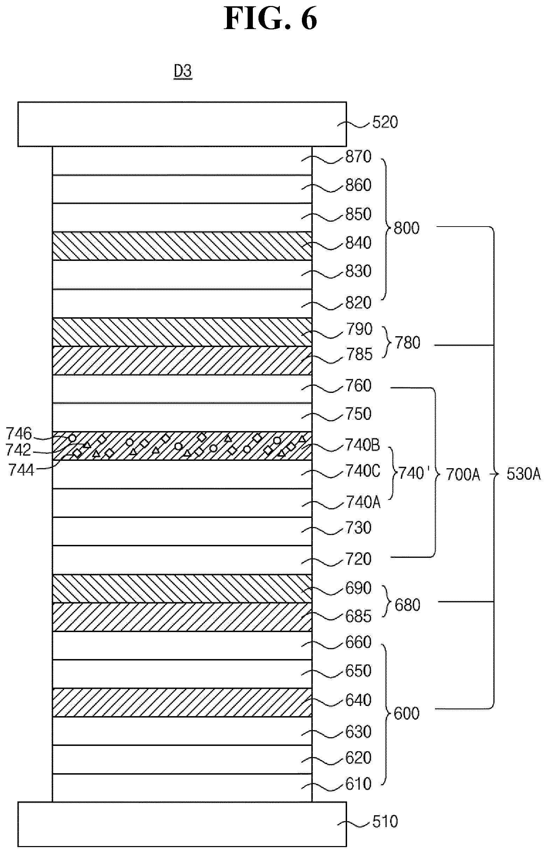

An emissive layer 530 that can include multiple emitting parts is disposed on the first electrode 510. As illustrated in FIGS. 5 and 6, the emissive layer 530 can include multiple emitting parts 600, 700, 700A, and 800 and at least one charge generation layer 680 and 780. Each of the emitting parts 600, 700, 700A and 800 includes at least one emitting material layer and can further include an HIL, an HTL, an EBL, an HBL, an ETL and/or an EIL.

The second electrode 520 can be disposed on the first substrate 402 above which the emissive layer 530 can be disposed. The second electrode 520 can be disposed over a whole display area, can include a conductive material with a relatively low work function value compared to the first electrode 510, and can be a cathode. For example, the second electrode 520 can include, but is not limited to, Al, Mg, Ca, Ag, alloy thereof, and/or combinations thereof such as Al-Mg.

Since the light emitted from the emissive layer 530 is incident to the color filter layer 480 through the second electrode 520 in the organic light emitting display device 400 in accordance with the second embodiment of the present invention, the second electrode 520 has a thin thickness so that the light can be transmitted.

The color filter layer 480 is disposed on the OLED D and includes a red color filter pattern 482, a green color filter pattern 484 and a blue color filter pattern 486 each of which is disposed correspondingly to the red pixel RP, the green pixel GP and the blue pixel BP, respectively. Although not shown in FIG. 4, the color filter layer 480 can be attached to the OLED D through an adhesive layer. Alternatively, the color filter layer 480 can be disposed directly on the OLED D.

In addition, an encapsulation film 470 can be disposed on the second electrode 520 in order to prevent or reduce outer moisture from penetrating into the OLED D. The encapsulation film 470 can have, but is not limited to, a laminated structure including a first inorganic insulating film, an organic insulating film and a second inorganic insulating film (170 in FIG. 2). In addition, a polarizing plate can be attached onto the second substrate 404 to reduce reflection of external light. For example, the polarizing plate can be a circular polarizing plate.

In FIG. 4, the light emitted from the OLED D is transmitted through the second electrode 520 and the color filter layer 480 is disposed on the OLED D. In this case, the organic light emitting display device 400 can be a top-emission type. Alternatively, when the organic light emitting display device 400 is a bottom-emission type, the light emitted from the OLED D is transmitted through the first electrode 510 and the color filter layer 480 can be disposed between the OLED D and the first substrate 402.

In addition, a color conversion layer may be formed or disposed between the OLED D and the color filter layer 480. The color conversion layer may include a red color conversion layer, a green color conversion layer and a blue color conversion layer each of which is disposed correspondingly to each pixel (RP, GP and BP), respectively, so as to convert the white (W) color light to each of a red, green and blue color lights, respectively. Alternatively, the organic light emitting display device 400 can comprise the color conversion film instead of the color filter layer 480.

As described above, the white (W) color light emitted from the OLED D is transmitted through the red color filter pattern 482, the green color filter pattern 484 and the blue color filter pattern 486 each of which is disposed correspondingly to the red pixel region RP, the green pixel region GP and the blue pixel region BP, respectively, so that red, green and blue color lights are displayed in the red pixel region RP, the green pixel region GP and the blue pixel region BP.

An OLED that can be applied into the organic light emitting display device will be described in more detail. FIG. 5 illustrates a schematic cross-sectional view of an organic light emitting diode having a tandem structure of two emitting parts.

As illustrated in FIG. 5, the OLED D2 in accordance with the example embodiment of the present invention includes first and second electrodes 510 and 520 facing each other and an emissive layer 530 disposed between the first and second electrodes 510 and 520. The emissive layer 530 includes a first emitting part 600 disposed between the first and second electrodes 510 and 520, a second emitting part 700 disposed between the first emitting part 600 and the second electrode 520 and a charge generation layer (CGL) 680 disposed between the first and second emitting parts 600 and 700.

The first electrode 510 can be an anode and can include a conductive material having relatively high work function value such as TCO. For example, the first electrode 510 can include, but is not limited to, ITO, IZO, ITZO, SnO, ZnO, ICO, AZO, and/or combinations thereof. The second electrode 520 can be a cathode and can include a conductive material with a relatively low work function value. For example, the second electrode 520 can include, but is not limited to, highly reflective material such as Al, Mg, Ca, Ag, alloy thereof and/or combinations thereof such as Al-Mg.

The first emitting part 600 includes a first EML (EML1) 640. The first emitting part 600 can further include at least one of an HIL 610 disposed between the first electrode 510 and the EMI,1 640, a first HTL (HTL1) 620 disposed between the HIL 610 and the EMI,1 640, and a first ETL (ETL1) 660 disposed between the EML1 640 and the CGL 680. Alternatively, the first emitting part 600 can further include a first EBL (EBL1) 630 disposed between the HTL1 620 and the EMI,1 640 and/or a first HBL (HBL1) 650 disposed between the EMI,1 640 and the ETL1 660.

The second emitting part 700 includes a second EML (EML2) 740. The second emitting part 700 can further include at least one of a second HTL (HTL2) 720 disposed between the CGL 680 and the EML2 740, a second ETL (ETL2) 760 disposed between the second electrode 520 and the EMI,2 740 and an EII, 770 disposed between the second electrode 520 and the ETL2 760. Alternatively, the second emitting part 700 can further include a second EBL (EBL2) 730 disposed between the HTL2 720 and the EMI,2 740 and/or a second HBL (HBL2) 750 disposed between the EMI,2 740 and the ETL2 760.

One of the EMI,1 640 and the EMI,2 740 can include the organic compound having the structure of Chemical Formulae 1 to 6 so that it can emit red to green color light, and the other of the EML1 640 and the EM2 740 can emit blue color light, so that the OLED D2 can realize white (W) emission. Hereinafter, the OLED D2 where the EMI,2 740 includes the organic compound having the structure of Chemical Formulae 1 to 6 will be described in detail.

The HIL 610 is disposed between the first electrode 510 and the HTL1 620 and improves an interface property between the inorganic first electrode 510 and the organic HTL1 620. In one exemplary embodiment, the HIL 610 can include, but is not limited to, MTDATA, NATA, 1T-NATA, 2T-NATA, CuPc, TCTA, NPB (NPD), DNTPD, HAT-CN, TDAPB, PEDOT/PSS, N-(biphenyl-4-yl)-9,9-dimethyl-N-(4-(9-phenyl-9H-carbazol-3-yl)phenyl)-9H-fluoren-2-amine, NPNPB and/or combinations thereof. The HIL 610 can be omitted in compliance of the OLED D2 property.

In one example embodiment, each of the HTL1 620 and the HTL2 720 can independently include, but is not limited to, TPD, NPB (NPD), DNTPD, CBP, poly-TPD, TFB, TAPC, DCDPA, N-(biphenyl-4-yl)-9,9-dimethyl-N-(4-(9-phenyl-9H-carbazol-3-yl)phenyl)-9H-fluoren-2-amine, N-(biphenyl-4-yl)-N-(4-(9-phenyl-9H-carbazol-3-yl)phenyl)biphenyl-4-amine, N-([1,1'-Biphenyl]-4-yl)-9,9-dimethyl-N-(4-(9-phenl-9H-carbazol-3-yl)phenyl)-9H-fluoren-2-amine and/or combinations thereof.

Each of the ETL1 660 and the ETL2 760 facilitates electron transportation in each of the first emitting part 600 and the second emitting part 700, respectively. As an example, each of the ETL1 660 and the ETL2 760 can include at least one of oxadiazole-based compounds, triazole-based compounds, phenanthroline-based compounds, benzoxazole-based compounds, benzothiazole-based compounds, benzimidazole-based compound and triazine-based compounds. For example, each of the ETL1 660 and the ETL2 760 can include, but is not limited to, Alq₃, PBD, spiro-PBD, Liq, TPBi, BAlq, Bphen, NBphen, BCP, TAZ, NTAZ, TpPyPB, TmPPPyTz, PFNBr, TPQ, TSPO1, ZADN, at least one electron transporting materials of Chemical Formula 7 and/or combinations thereof.

The EII, 770 is disposed between the second electrode 520 and the ETL2 760, and can improve physical properties of the second electrode 520 and therefore, can enhance the lifespan of the OLED D2. In one example embodiment, the EII, 770 can include, but is not limited to, an alkali metal halide or an alkaline earth metal halide such as LiF, CsF, NaF, BaF₂ and the like, and/or an organometallic Compound such as Liq, lithium benzoate, sodium stearate, and the like.

Each of the EBL1 630 and the EBL2 730 can independently include, but is not limited to, TCTA, Tris[4-(diethylamino)phenyl]amine, N-(biphenyl-4-yl)-9,9-dimethyl-N-(4-(9-phenyl-9H-carbazol-3-yl)phenyl)-9H-fluorene-2-amine, TAPC, MTDATA, mCP, mCBP, CuPc, DNTPD, TDAPB, DCDPA, 2,8-bis(9-phenyl-9H-carbazol-3-yl)dibenzo[b,d]thiophene and/or combinations thereof, respectively.

Each of the HBL1 650 and the HBL2 750 can include, but is not limited to, at least one of oxadiazole-based compounds, triazole-based compounds, phenanthroline-based compounds, benzoxazole-based compounds, benzothiazole-based compounds, benzimidazole-based compounds, and triazine-based compounds. For example, each of the HBL1 650 and the HBL2 750 can independently include, but is not limited to, BCP, BAlq, Alq₃, PBD, spiro-PBD, Liq, B3PYMPM, DPEPO, 9-(6-(9H-carbazol-9-yl)pyridine-3-yl)-9H-3,9'-bicarbazole, TSPO1 and/or combinations thereof, respectively.

The CGL 680 is disposed between the first emitting part 600 and the second emitting part 700. The CGL 680 includes an N-type CGL (N-CGL) 685 disposed adjacently to the first emitting part 600 and a P-type CGL (P-CGL) 690 disposed adjacently to the second emitting part 700. The N-CGL 685 injects electrons to the EMI,1 640 of the first emitting part 600 and the P-CGL 690 injects holes to the EMI,2 740 of the second emitting part 700.

The N-CGL 685 can be an organic layer doped with an alkali metal such as Li, Na, K and Cs and/or an alkaline earth metal such as Mg, Sr, Ba and Ra. For example, the host in the N-CGL 685 can include, but is not limited to, Bphen and MTDATA. The contents of the alkali metal or the alkaline earth metal in the N-CGL 685 can be between about 0.01 wt% and about 30 wt%.

The P-CGL 690 can include, but is not limited to, inorganic material selected from the group consisting of tungsten oxide (WOₓ), molybdenum oxide MoOₓ), beryllium oxide (Be₂O₃), vanadium oxide (V₂O ) and/or combinations thereof, and/or organic material selected from the group consisting of NPD, DNTPD, HAT-CN, 2,3,5,6-tetrafluoro-7,7,8,8-tetracyanoquinodimethane (F4-TCNQ), 1,3,4,5,7,8-hexafluorotetracyanonapththoquinodimethane (F6-TCNNQ), TPD, N,N,N',N'-tetranaphthalenyl-benzidine (TNB), TCTA, N,N'-dioctyl-3,4,9,10-perylenedicarboximide (PTCDI-C8) and/or combinations thereof.

The EML1 640 can be a blue EML. In this case, the EMI,1 640 can be a blue EML, a sky-blue EML or a deep-blue EML. The EMI,1 640 can include a blue host and a blue dopant. The EMI,1 640 can include a blue host and blue dopant.

The blue host can include at least one of a P-type blue host and an N-type blue host. For example, the blue host can include, but is not limited to, mCP, mCP-CN, mCBP, CBP-CN, 9-(3-(9H-Carbazol-9-yl)phenyl)-3-(diphenylphosphoryl)-9H-carbazole (mCPPO1) 3,5-Di(9H-carbazol-9-yl)biphenyl (Ph-mCP), TSPO1, 9-(3'-(9H-carbazol-9-yl)-[1,1'-biphenyl]-3-yl)-9H-pyrido[2,3-b]indole (CzBPCb), Bis(2-methylphenyl)diphenylsilane (UGH-1), 1,4-Bis(triphenylsilyl)benzene (UGH-2), 1,3-Bis(triphenylsilyl)benzene (UGH-3), 9,9-Spiorobifluoren-2-yl-diphenyl-phosphine oxide (SPPO1), 9,9'-(5-(Triphenylsilyl)-1,3-phenylene)bis(9H-carbazole) (SimCP) and/or combinations thereof. Alternatively, the blue host can include the organic compound having the structure of Chemical Formulae 1 to 6.

The blue dopant can include at least one of blue phosphorescent material, blue fluorescent material and blue delayed fluorescent material. As an example, the blue dopant can include, but is not limited to, perylene, 4,4'-Bis[4-(di-p-tolylamino)styryl]biphenyl (DPAVBi), 4-(Di-p-tolylamino)-4-4'-[(di-p-tolylamino)styryl]stilbene (DPAVB), 4,4'-Bis[4-(diphenylamino)styryl]biphenyl (BDAVBi), 2,7-Bis(4-diphenylamino)styryl)-9,9-spiorfluorene (spiro-DPVBi), [1,4-bis[2-[4-[N,N-di(p-tolyl)amino]phenyl]vinyl] benzene (DSB), 1-4-di-[4-(N,N-diphenyl)amino]styryl-benzene (DSA), 2,5,8,11-Tetra-tetr-butylperylene (TBPe), Bis(2-hydroxylphenyl)-pyridine)beryllium (Bepp₂), 9-(9-Phenylcarbazole-3-yl)-10-(naphthalene-1-yl)anthracene (PCAN), mer-Tris(1-phenyl-3-methylimidazolin-2-ylidene-C,C(2)'iridium(III) (mer-Ir(pmi)₃), fac-Tris(1,3-diphenyl-benzimidazolin-2-ylidene-C,C(2)'iridium(III) (fac-Ir(dpbic)₃), Bis(3,4,5-trifluoro-2-(2-pyridyl)phenyl-(2-carboxypyridyl)iridium(III) (Ir(tfpd)₂pic), tris(2-(4,6-difluorophenyl)pyridine))iridium(III) (Ir(Fppy)₃), Bis[2-(4,6-difluorophenyl)pyridinato-C²,N](picolinato)iridium(III) (FIrpic) and/or combinations thereof.

The contents of the blue host in the EMI,1 640 can be about 50 wt% to about 99 wt%, for example, about 80 wt% to about 95 wt%, and the contents of the blue dopant in the EMI,1 640 can be about 1 wt% to about 50 wt%, for example, about 5 wt% to about 20 wt%, but is not limited thereto. When the EMI,1 640 includes both the P-type blue host and the N-type blue host, the P-type blue host and the N-type blue host can be admixed, but is not limited to, with a weight ratio of about 4: 1 to aobut 1:4, for example about 3:1 to about 1:3.

The EML2 740 can include a lower EML (first layer) 740A disposed between the EBL2 730 and the HBL2 750 and an upper EML (second layer) 740B disposed between the lower EML 740A and the HBL2 750. One of the first layer 740A and the second layer 740B can emit red color light and the other of the first layer 740A and the second layer 740B can emit green color light. Hereinafter, the EMI,1 740 where the first layer 740A emits a red color light and the second layer 740B emits a green color light will be described in detail.

The first layer 740A includes a red host and red dopant. As an example, the red host can include at least one of a P-type red host and an N-type red host. For example, the red host can include, but is not limited to, mCP-CN, CBP, mCBP, mCP, DPEPO, PPT, TmPyPB, PYD-2Cz, DCzDBT, DCzTPA, pCzB-2CN, mCzB-2CN, TSPO1, CCP, 4-(3-(triphenylen-2-yl)phenyl)dibenzo[b,d]thiophene, 9-(4-(9H-carbazol-9-yl)phenyl)-9H-3,9'-bicarbazole, 9-(3-(9H-carbazol-9-yl)phenyl)-9H-3,9'-bicarbazole, 9-(6-(9H-carbazol-9-yl)pyridin-3-yl)-9H-3,9'-bicabazole, BCzPh, BCZ, TCP, TCTA, CDBP, DMFL-CBP, Spiro-CBP, TCz1 and/or combinations thereof. Alternatively, the red host can include the organic compound having the structure of Chemical Formulae 1 to 6. Preferred are BCzPh and BCZ, in particular

The red dopant can include at least one of a red fluorescent compound, a red fluorescent compound and a red delayed fluorescent compound. As an example, the red dopant can include, but is not limited to, Bis[2-(4,6-dimethyl)phenylquinoline)](2,2,6,6-tetramethylheptane-3,5-dionate)iridium(III), Bis[2-(4-n-hexylphenyl)quinoline](acetylacetonate)iridium(III) (Hex-Ir(phq)₂(acac)), Tris[2-(4-n-hexylphenyl)quinoline]iridium(III) (Hex-Ir(phq)₃), Tris[2-phenyl-4-methylquinoline]iridium(III) (Ir(Mphq)₃), Bis(2-phenylquinoline)(2,2,6,6-tetramethylheptene-3,5-dionate)iridium(III) (Ir(dpm)PQ₂), Bis(phenylisoquinoline)(2,2,6,6-tetramethylheptene-3,5-dionate)iridium(III) (Ir(dpm)(piq)₂), Bis(1-phenylisoquinoline)(acetylacetonate)iridium(III) (Ir(piq)₂(acac)), Bis[(4-n-hexylphenyl)isoquinoline](acetylacetonate)iridium(III) (Hex-Ir(piq)₂(acac)), Tris[2-(4-n-hexylphenyl)quinoline]iridium(III) (Hex-Ir(piq)₃), Tris(2-(3-methylphenyl)-7-methyl-quinolato)iridium (Ir(dmpq)₃), Bis[2-(2-methylphenyl)-7-methylquinoline](acetylacetonate)iridium(III) (Ir(dmpq)₂(acac)), Bis[2-(3,5-dimethylphenyl)-4-methylquinoline](acetylacetonate)iridium(III) (Ir(mphmq)₂(acac)), Tris(dibenzoylmethane)mono(1,10-phenanthroline)europium(III) (Eu(dbm)₃(phen)) and/or combinations thereof.

The contents of the red host in the first layer 740A can be about 50 wt% to about 99 wt%, for example, about 80 wt% to about 95 wt%, and the contents of the red dopant in the first layer 740A can be about 1 wt% to about 50 wt%, for example, about 5 wt% to about 20 wt%, but is not limited thereto. When the first layer 740A includes both the P-type red host and the N-type red host, the P-type red host and the N-type red host can be admixed, but is not limited to, with a weight ratio of about 4:1 to aobut 1:4, for example about 3:1 to about 1:3.

The second layer 740B can include a first host 742, optionally a second host 744, and a green dopant 746. The first host 742 can be an N-type host and includes the organic compound having the structure of Chemical Formulae 1 to 6. The second host 744 can be a P-type host and can include, but is not limited to, a biscarbazole-based organic compound, an aryl amine- or a hetero aryl amine-based organic compound with at least one fused aromatic and/or fused hetero aromatic moiety, and/or an aryl amine- or a hetero aryl amine-based organic compound with a spirofluorene moiety. For example, the second host 744 can include, but is not limited to, mCP-CN, CBP, mCBP, mCP, DPEPO, PPT, TmPyPB, PYD-2Cz, DCzDBT, DCzTPA, pCzB-2CN, mCzB-2CN, TSPO1, CCP, 4-(3-(triphenylen-2-yl)phenyl)dibenzo[b,d]thiophene, 9-(4-(9H-carbazol-9-yl)phenyl)-9H-3,9'-bicarbazole, 9-(3-(9H-carbazol-9-yl)phenyl)-9H-3,9'-bicarbazole, 9-(6-(9H-carbazol-9-yl)pyridin-3-yl)-9H-3,9'-bicabazole, BCzPh, BCZ, TCP, TCTA, CDBP, DMFL-CBP, Spiro-CBP, TCz1 and/or combinations thereof.

The green dopant 746 can include at least one of a green phosphorescnet compound, a green fluorescent compound and a green delayed fluorescent compound. As an example, the green dopant 746 can include, but is not limited to, [Bis(2-phenylpyridine)](pyridyl-2-benzofuro[2,3-b]pyridine)iridium, Tris[2-phenylpyridine]iridium(III) (Ir(ppy)₃), fac-Tris(2-phenylpyridine)iridium(III) (fac-Ir(ppy)₃), Bis(2-phenylpyridine)(acetylacetonate)iridium(III) (Ir(ppy)₂(acac)), Tris[2-(p-tolyl)pyridine]iridium(III) (Ir(mppy)₃), Bis(2-(naphthalene-2-yl)pyridine)(acetylacetonate)iridium(III) (Ir(npy)₂acac), Tris(2-phenyl-3-methyl-pyridine)iridium (Ir(3mppy)₃), fac-Tris(2-(3-p-xylyl)phenyl)pyridine iridium(III) (TEG) and/or combinations thereof. Preferred are Tris[2-phenylpyridine]iridium(III) (Ir(ppy)₃), fac-Tris(2-phenylpyridine)iridium(III) (fac-Ir(ppy)₃, in particular Tris[2-phenylpyridine]iridium(III) (Ir(ppy)₃).

The contents of the host 742 and/or 744 in the second layer 740B can be about 50 wt% to about 99 wt%, for example, about 80 wt% to about 95 wt%, and the contents of the dopant 746 in the second layer 740B can be about 1 wt% to about 50 wt%, for example, about 5 wt% to about 20 wt%, but is not limited thereto. When the second layer 740B includes both the first host 742 and the second host 744, the first host 742 and the second host 744 can be admixed, but is not limited to, with a weight ratio of about 4:1 to aobut 1:4, for example about 3:1 to about 1:3.

Alternatively, the EMI,2 740 can further include a third layer (740C in FIG. 6) that can emit yellow-green color light and can be disposed between the first layer 740A of the red EML and the second layer 740B of the green EML.

The OLED D2 with a tandem structure in accordance with this embodiment includes the organic compound having the structure of Chemical Formulae 1 to 6. The organic compound having the structure of Chemical Formulae 1 to 6 has higher single energy level and triplet energy level and wider energy bandgap between HOMO energy level and the LUMO energy level compared to the emitter. The luminous properties of the OLED D2 where the emissive layer includes the organic compound with excellent affinity to both holes and electrons can be improved. Also, the organic compound with large dipole moment shows excellent affinity to electrons. In addition, the organic compound includes at least one of deuterium and/or a deuterium-substituted group so as to maintain stable chemical conformation. Accordingly, it is possible to implement white color light with beneficial luminous efficiency and luminous lifetime in the OLED D2 including the organic compound and two emitting parts.

An OLED can have three or more emitting parts to form a tandem structure. FIG. 6 is a schematic cross-sectional view illustrating an organic light emitting diode in accordance with yet another example embodiment of the present invention.

As illustrated in FIG. 6, the OLED D3 includes first and second electrodes 510 and 520 facing each other and an emissive layer 530A disposed between the first and second electrodes 510 and 520. The emissive layer 530A includes a first emitting part 600 disposed between the first and second electrodes 510 and 520, a second emitting part 700A disposed between the first emitting part 600 and the second electrode 520, a third emitting part 800 disposed between the second emitting part 700A and the second electrode 520, a first charge generation layer (CGL1) 680 disposed between the first and second emitting parts 600 and 700A, and a second charge generation layer (CGL2) 780 disposed between the second and third emitting parts 700A and 800.

The first emitting part 600 includes a first EML (EML1) 640. The first emitting part 600 can further include at least one of an HIL 610 disposed between the first electrode 510 and the EMI,1 640, a first HTL (HTL1) 620 disposed between the HIL 610 and the EMI,1 640, a first ETL (ETL1) 660 disposed between the EMI,1 640 and the CGL1 680. Alternatively, the first emitting part 600 can further comprise a first EBL (EBL1) 630 disposed between the HTL1 620 and the EMI,1 640 and/or a first HBL (HBL1) 650 disposed between the EMI,1 640 and the ETL1 660.

The second emitting part 700A includes a second EML (EML2) 740'. The second emitting part 700A can further include at least one of a second HTL (HTL2) 720 disposed between the CGL1 680 and the EMI,2 740' and a second ETL (ETL2) 760 disposed between the EML2 740' and the CGL2 780. Alternatively, the second emitting part 700A can further include a second EBL (EBL2) 730 disposed between the HTL2 720 and the EMI,2 740' and/or a second HBL (HBL2) 750 disposed between the EMI,2 740' and the ETL2 760.

The third emitting part 800 includes a third EML (EML3) 840. The third emitting part 800 can further include at least one of a third HTL (HTL3) 820 disposed between the CGL2 780 and the EML3 840, a third ETL (ETL3) 860 disposed between the second electrode 520 and the EML3 840 and an EII, 870 disposed between the second electrode 520 and the ETL3 860. Alternatively, the third emitting part 800 can further comprise a third EBL (EBL3) 830 disposed between the HTL3 820 and the EML3 840 and/or a third HBL (HBL3) 850 disposed between the EML3 840 and the ETL3 860.

The CGL1 680 is disposed between the first emitting part 600 and the second emitting part 700A and the CGL2 780 is disposed between the second emitting part 700A and the third emitting part 800. The CGL1 680 includes a first N-type CGL (N-CGL1) 685 disposed adjacently to the first emitting part 600 and a first P-type CGL (P-CGL1) 690 disposed adjacently to the second emitting part 700A. The CGL2 780 includes a second N-type CGL (N-CGL2) 785 disposed adjacently to the second emitting part 700A and a second P-type CGL (P-CGL2) 790 disposed adjacently to the third emitting part 800. Each of the N-CGL1 685 and the N-CGL2 785 injects electrons to the EMI,1 640 of the first emitting part 600 and the EMI,2 740' of the second emitting part 700A, respectively, and each of the P-CGL1 690 and the P-CGL2 790 injects holes to the EMI,2 740' of the second emitting part 700A and the EML3 840 of the third emitting part 800, respectively.

The materials included in the HIL 610, the HTL1 to the HTL3 620, 720 and 820, the EBL1 to the EBL3 630, 730 and 830, the HBL1 to the HBL3 650, 750 and 850, the ETL1 to the ETL3 660, 760 and 860, the EIL 870, the CGL1 680, and the CGL2 780 can be identical to the materials with referring to FIGS. 3 and 5.

At least one of the EMI,1 640, the EML2 740' and the EML3 840 can include the organic compound having the structure of Chemical Formulae 1 to 6. For example, one of the EMI,1 640, the EML2 740' and the EML3 840 can emit red to green color light, and the other of the EML1 640, the EML2 740' and the EML3 840 can emit blue color light so that the OLED D3 can realize white (W) emission. Hereinafter, the OLED where the EMI,2 740' includes the organic compound having the structure of Chemical Formulae 1 to 6 and emits red to green color light, and each of the EMI,1 640 and the EML3 840 emits blue color light will be described in detail.

Each of the EMI,1 640 and the EML3 840 can be independently a blue EML. In this case, each of the EMI,1 640 and the EML3 840 can be independently a blue EML, a sky-blue EML or a deep-blue EML. Each of the EMI,1 640 and the EML3 840 can independently include a blue host and a blue dopant. Each of the blue host and the blue dopant can be identical to the blue host and the blue dopant with referring to FIG. 5. For example, the blue dopant can include at least one of blue phosphorescent material, blue fluorescent material and blue delayed fluorescent material. Alternatively, the blue dopant in the EMI,1 640 can be identical to or different from the blue dopant in the EML3 840 in terms of color and/or luminous efficiency.

The EMI,2 740' can include a lower EML (first layer) 740A disposed between the EBL2 730 and the HBL2 750, an upper EML (second layer) 740B disposed between the first layer 740A and the HBL2 750, and a middle EML (third layer) 740C disposed between the first layer 740A and the second layer 740B. One of the first layer 740A and the second layer 740B can emit red color and the other of the first layer 740A and the second layer 740B can emit green color. Hereinafter, the EMI,2 740' where the first layer 740A emits a red color and the second layer 740B emits a green color will be described in detail.

The first layer 740A can include a red host and a red dopant. The red host can include at least one of a P-type red host and an N-type red host. As an example, the red host and the red dopant can be identical to the corresponding materials with referring to FIG. 5.

The second layer 740B can include a first host 742, optionally a second host 744, and a green dopant 746. The first host 742, the second host 744 and the green dopant 746 can be identical to the corresponding materials with referring to FIG. 5.

The third layer 740C can be a yellow-green emitting material layer. The third layer 740C can include a yellow-green host and a yellow-green dopant. The yellow-green host can include at least one of a P-type yellow-green host and an N-type yellow-green host. As an example, the yellow-green host can include at least one of the blue host, the green host and the red host. Alternatively, the yellow-green host can include the organic compound having the structure of Chemical Formulae 1 to 6.

The yellow-green dopant can include at least one of a yellow-green fluorescent compound, a yellow-green phosphorescent compound and a yellow-green delayed fluorescent compound. For example, the yellow-green dopant can include, but is not limited to, 5,6,11,12-Tetraphenylnaphthalene (Rubrene), 2,8-Di-tert-butyl-5,11-bis(4-tert-butylphenyl)-6,12-diphenyltetracene (TBRb), Bis(2-phenylbenzothiazolato)(acetylacetonate)irdium(III) (Ir(BT)₂(acac)), Bis(2-(9,9-diethytl-fluoren-2-yl)-1-phenyl-1H-benzo[d]imdiazolato)(acetylacetonate)iridium(III) (Ir(fbi)₂(acac)), Bis(2-phenylpyridine)(3-(pyridine-2-yl)-2H-chromen-2-onate)iridium(III) (fac-Ir(ppy)₂Pc), Bis(2-(2,4-difluorophenyl)quinoline)(picolinate)iridium(III) (FPQIrpic), Bis(4-phenylthieno[3,2-c]pyridinato-N,C2') (acetylacetonate) iridium(III) (PO-01) and/or combinations thereof.

The contents of the yellow-green host in the third layer 740C can be about 50 wt% to about 99 wt%, for example, about 80 wt% to about 95 wt%, and the contents of the yellow-green dopant in the third layer 740C can be about 1 wt% to about 50 wt%, for example, about 5 wt% to about 20 wt%, but is not limited thereto. When the third layer 740C includes both the P-type yellow-green host and the N-type yellow-green host, the P-type yellow-green host and the N-type yellow-green host can be admixed, but is not limited to, with a weight ratio of about 4:1 to aobut 1:4, for example about 3:1 to about 1:3.

The OLED D3 with a tandem structure in accordance with this embodiment includes the organic compound having the structure of Chemical Formulae 1 to 6. The organic compound having the structure of Chemical Formulae 1 to 6 has higher single energy level and triplet energy level and wider energy bandgap between HOMO energy level and the LUMO energy level compared to the emitter. The luminous properties of the OLED D3 where the emissive layer includes the organic compound with excellent affinity to both holes and electrons can be improved. Also, the organic compound with large dipole moment shows excellent affinity to electrons. In addition, the organic compound includes at least one of deuterium and/or a deuterium-substituted group so as to maintain stable chemical conformation. Accordingly, it is possible to implement white color light with beneficial luminous efficiency and luminous lifetime in the OLED D2 including the organic compound and three emitting parts.

### Synthesis Example 1: Synthesis of Compound GEH1-D1

### (1) Syntheis of Intermediate A-i

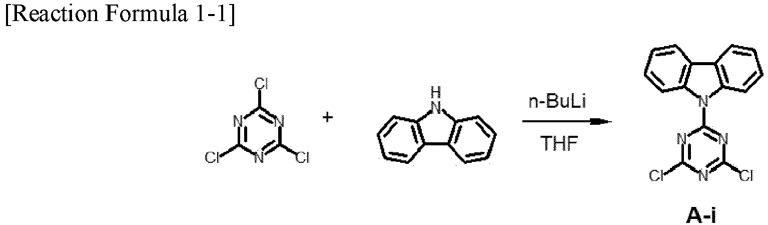

Carbazole (81 g, 0.48 mol) in THF (1000 ml) was added into a round bottom flask with one neck, then the solution was subsitituted with nitorgen and cooled to -78°C. 2.5 M n-BuLi solution (190 ml, 0.48 mol) was added slowly into the flask, 2,4,6-trichloro-1,3,5-triazine (100 g, 0.54 mol) was adeed into the flask 30 minutes later, and the solution was raised to a room temperature to proceed with a reaction for 3 hours. After the reaction was complete, the product was completely precipitated using methahol, filtered, dissolved methylene chloride (MC) again, and proceed with column chormatography (eluent: hexane (Hex) /MC). The obtained product was concentrated, subejcted to acetone slurry and filtered to give an Intermedate A-1 (127 g, 83%).

### (2) Syntheis of Intermediate A

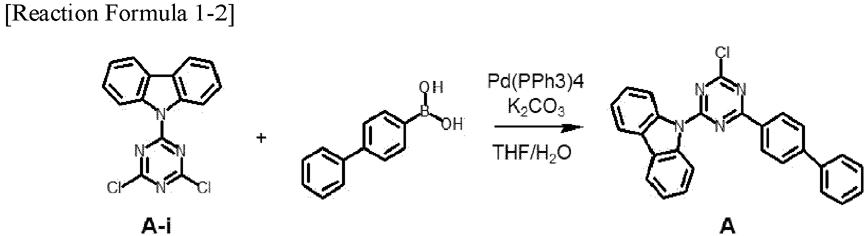

The Intermediate A-i (50 g, 0.16 mol), [1,1'-biphenyl]-4-ylboronic acid (28.0 g, 0.135 mol), Pd(PPh3)4 (Palladium-tetrakis(triphenylphosphine), 9 g, 0.009 mol) and K₂CO₃ (44 g, 0.315 mol) in a mixed solvent (Tetrahydrofuran (THF) 500 ml, water 100 ml) were added into a round bottom flask with one neck, then the solution was refluxed at 80°C for 4 hours. After the reaction solution was cooled to a room temperature, excessive methanol was poured into the flask to preicipiate a product completely and then the product was filtered. The preicipitated product was heated with DCB (1,2-Dichlorobenzene) to be dissoved completly, and then subjected to silica fliter using CF (chloroform). The filtered solution was concentrated, subejcted to methanol slurry and filrtered to give an Intermediate A (40.5 g, 59%).

### (3) Synthesis of Intermediate C-1

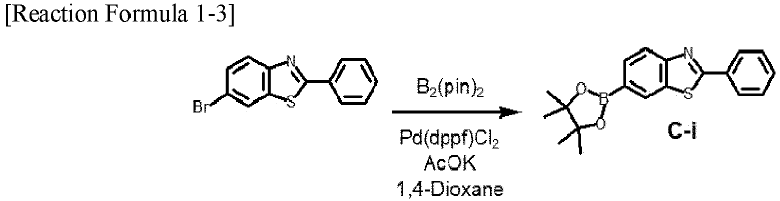

6-bormo-2-phenyl-1,3-benzothiazole (20 g, 0.072 mol), Pd(dppf)Cl₂ (1,1'-Bis(dipheneylphosphino)ferrocene)palladium(II), 2.6 g, 0.004 mol), Potassium acetate (14.3 g, 0.14 mol) and B₂(pin)₂ (Bis(pinacolato)diborn, 27.7 g, 0.11 mol) in 1,4-Dioxane (250 ml) were added into a round bottom flask with one neck, then the solution was refluxed for 3 hours. Afther the reaction solution was cooled to a room temperature sufficiently, the solution was filtered, washed with MC (methylene chloride) and concentrated. The concentrated product was dissolved in MC again, and subjected to silica gel filter using MC. The filtered solution was concentrated and subscted to methanol slurry to precipitat a solid as an Intermediate C-1 (21 g, 90%).

### (4) Synthesis of Intermediate A-1-ii

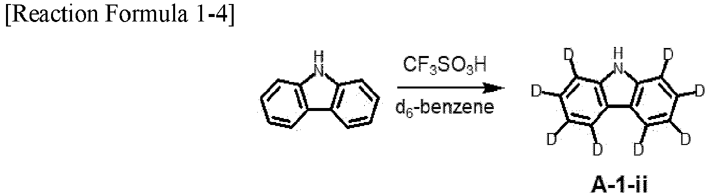

Carbazole (10 g, 0.060 mol) in a mixed solvent (DCB 50 mL, d₆-benzne 50 ml) was added into a round bottom flask with one neck, and then trifluoro methane sulfonic acid

(CF₃SO₃H, 40 ml) was added slowly into the flask. The solution was neutralized with K₃PO₄ saturation solution for 3 horus and then extracted with MC/H₂O twice. The extracted solution was concentrated and subjected to methanol slurry to give an Intermeidate A-1-ii (9.5 g, 90.7%).

### (5) Synthesis of Intermediate A-1-i

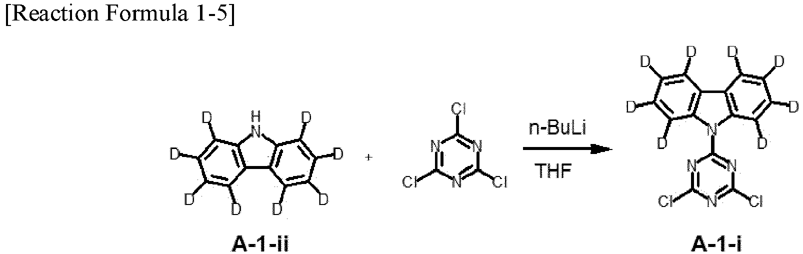

The Intermeidate A-1-ii (9.5 g, 0.054 mol) in THF (100 ml) was added into a round bottom flask with one neck, then the solution was was substituted with nitrogen and cooled to - 78°C. 2.5 M n-BuLi slution (22 ml, 0.055 mol) was added slowly into the flask, 1,3,5-chlorotriazine (11 g, 0.060 mol) was added into the flask 30 minutes later, and the solution was raised to a room temperature to proceed with a reaction for 3 hours. After the reaction was complete, the product was completely precipitated using methanol, filtered, dissolved with MC again, and proceed with colum choromatography (eluent: Hex / MC). The obtained product was concentrated, subject to acetone slurry and filtered to give an Intermediate A-1-i (13.0 g, 74.5%).

### (6) Synthesis of Intermediate A-1

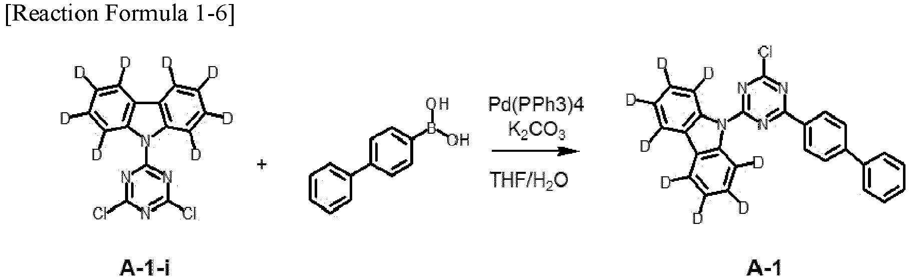

The Intermediate A-1-i (100 g, 0.32 mol), [1,1'-biphenyl]-4-ylboronic acid (56.0 g, 0.27 mol), Pd(PPh3)4 (18 g, 0.018 mol) and K₂CO₃ (88 g, 0.63 mol) in a mixed solvent (THF 1000 ml, water 200 ml) were added into a round bottom flask with one neck, then the solution was refluxed at 70°C for 2 hours. After the reaction solution was cooled to a room temperature, excessive methanol was poured into the flask to precipitate a procut completely, and the product was filtered. The precipitated product was heated with DCB to be dissolved completely, and then subjected to silica filter using CF. The filtered solution was concentrated, subject to methanol slurry and filtered to give an Intermediate A-1 (81.0 g, 59%).

### (7) Synthesis of Compound GEH1-D1

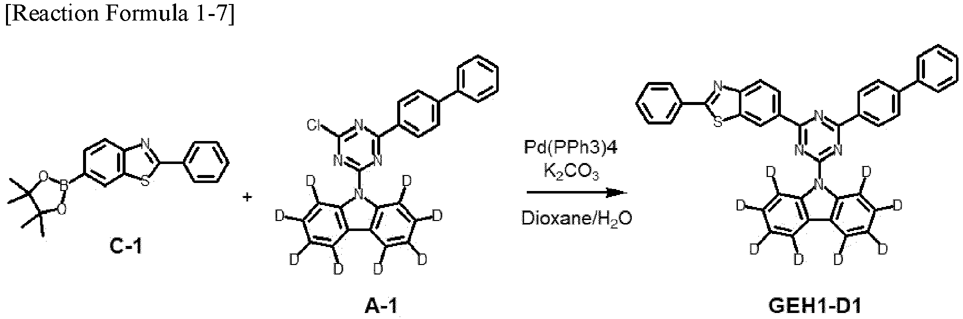

The Intermediate A-1 (10.0 g, 0.022 mol), the Intermediate C-1 (7.8 g, 0.024 mol), Pd(PPh3)4 (1.2 g, 0.0011 mol) and K₂CO₃ (6.1 g, 0.044 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH1-D1 (8.7 g, 62.3%).

### Synthesis Example 2: Synthesis of Compound GEH1-D2

### (1) Synthesis of Intermediate D-i

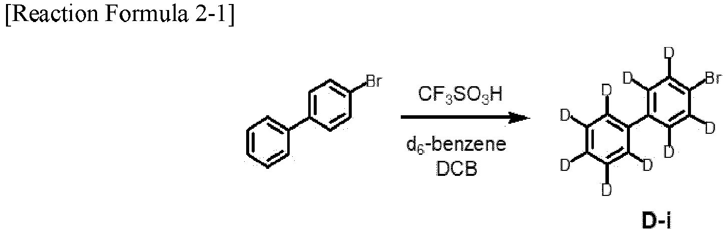

4-borombiphenyl (30 g, 0.13 mol) in DCB (300 ml) was added into a round bottom flask with one neck, then d₆-benzene (300 ml) was added into the flask. Trifluoro methane sulfonic acid (84 ml, 0.91 mol) was added slowly into the flast, then the raction was performed at room temperature for 3 hours. The reaction solution was cooled sufficeintly using ice bath and K₃PO₄ aqeuous solution was slowly into the flask to terminate the reaction. The solution was extrcted with MC/H₂O twice, and the extracted solution was concentrated. The concentrated product was dissolved in MC again and subjected to silca gel filter (eluent: Hex: MC = 1:1). The filtered solution was concentrated and dried to give an Intermediate D-i (30 g, 95%).

### (2) Synthesis of Intermediate D

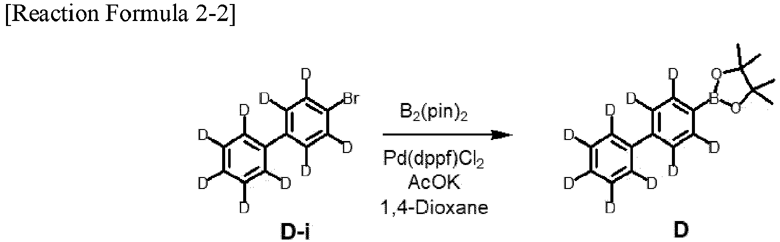

The Intermediate D-i (30 g, 0.12 mol), Pd(dppf)Cl₂ (4.5 g, 0.006 mol), Potassium acetate (24.3 g, 0.25 mol) and B₂(pin)₂ (47 g, 0.185 mol) in 1,4-Dioxane (300 ml) were added into a round bottom flask with one neck, then the solution was refluxed for 3 hours. Afther the reaction solution was cooled to a room temperature sufficiently, the solution was fitered, washed with MC and concentrated. The concentrated product was dissolved in MC again, and subjected to silica gel filter using MC. The filtered solution was concentrated and then subjected to methanol slurry to precipitate a solid as an Intermediate D (27 g, 75%).

### (3) Synthesis of Intermediate A-2

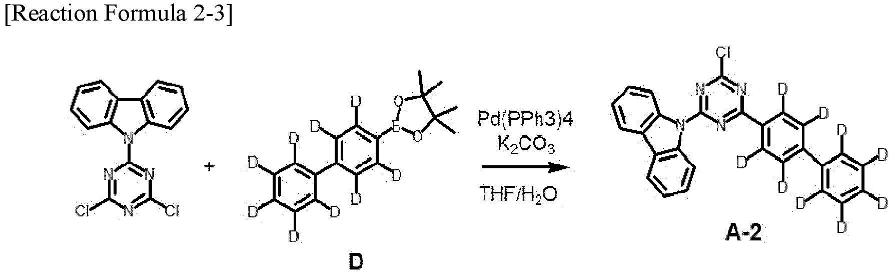

9-(4,6-dichloro-1,3,5-triazin-2-yl)carbazole (100 g, 0.32 mol), the Intemediate D (78.1 g, 0.27 mol), Pd(PPh3)4 (19 g, 0.016 mol) and K₂CO₃ (90 g, 0.64 mol) in a mixed solvent (THF 1000 ml, water 200 ml) were added into a round bottom flask with one neck, then the solution was refluxed at 70°C for 2 hours. After the reaction solution was cooled to a room temperature, excessive methanol was poured into the flask to preicipiate a product completely and then the product was filtered. The preicipitated product was heated with DCB to be dissoved completly, and then subjected to silica fliter using CF. The filtered solution was concentrated, subejcted to methanol slurry and filrtered to give an Intermediate A-2 (81.0 g, 57%).

### (4) Synthesis of Compound GEH1-D2

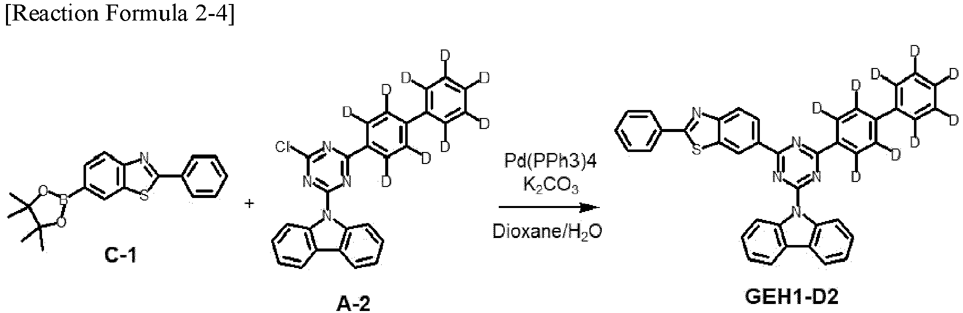

The Intermediate A-2 (10.0 g, 0.022 mol), the Intermediate C-1 (7.8 g, 0.024 mol), Pd(PPh3)4 (1.2 g, 0.0011 mol) and K₂CO₃ (6.1 g, 0.044 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH1-D2 (9.7 g, 69.4%).

### Synthesis Example 3: Synthesis of Compound GEH1-D3

### (1) Synthesis of Intermediate C-2-i

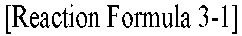

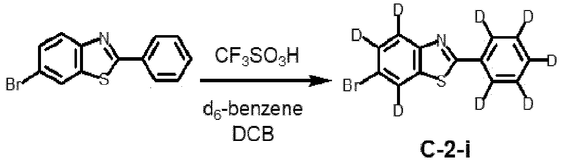

6-borom-2-phenyl-1,3-benzothiazole (30 g, 0.10 mol) in DCB (300 ml) was added into a round bottom flask with one neck, then d₆-benzene (300 ml) was added into the flask. Trifluoro methane sulfonic acid (120 ml, 1.3 mol) was added slowly into the flast, then the raction was performed at room temperature at 60°C for 3 hours. The reaction solution was cooled sufficeintly using ice bath and K₃PO₄ aqeuous solution was slowly into the flask to terminate the reaction. The solution was extrcted with MC/H₂O twice, and the extracted solution was concentrated. The concentrated product was dissolved in MC again and subjected to silca gel filter (eluent: Hex: MC = 1:1). The filtered solution was concentrated and dried to give an Intermediate C-2-i (27 g, 88%).

### (2) Synthesis of Intermediate C-2

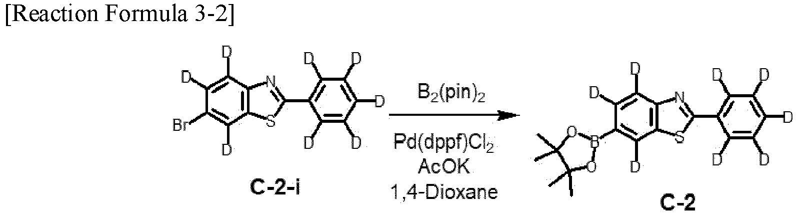

The Intermediate C-2-i (27.0 g, 0.09 mol), Pd(dppf)Cl₂ (3.3 g, 0.0045 mol), Potassium acetate (17.8 g, 0.18 mol) and B₂(pin)₂ (35 g, 0.135 mol) in 1,4-Dioxane (300 ml) were added into a round bottom flask with one neck, then the solution was refluxed for 4 hours. Afther the reaction solution was cooled to a room temperature sufficiently, the solution was fitered, washed with MC and concentrated. The concentrated product was dissolved in MC again, and subjected to silica gel filter using MC. The filtered solution was concentrated and then subjected to H₂O/MeOH slurry to precipitate a solid as an Intermediate C-2 (23 g, 74%).

### (3) Synthesis of Compound GEH1-D3

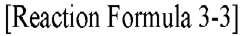

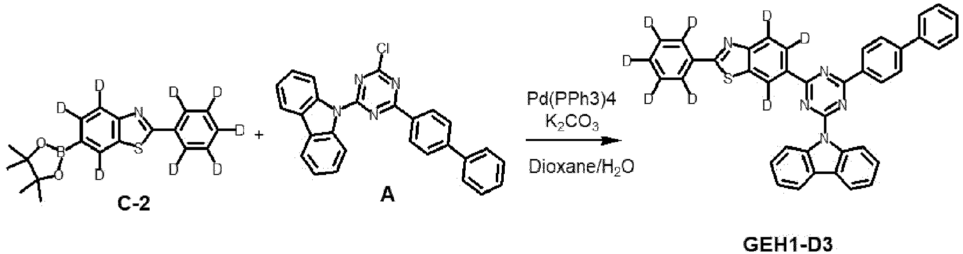

The Intermediate A (10.0 g, 0.023 mol), the Intermediate C-2 (7.8 g, 0.025 mol), Pd(PPh3)4 (1.4 g, 0.0012 mol) and K₂CO₃ (6.3 g, 0.045 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH1-D3 (10.0 g, 70.3%).

### Synthesis Example 4: Synthesis of Compound GEH1-D4

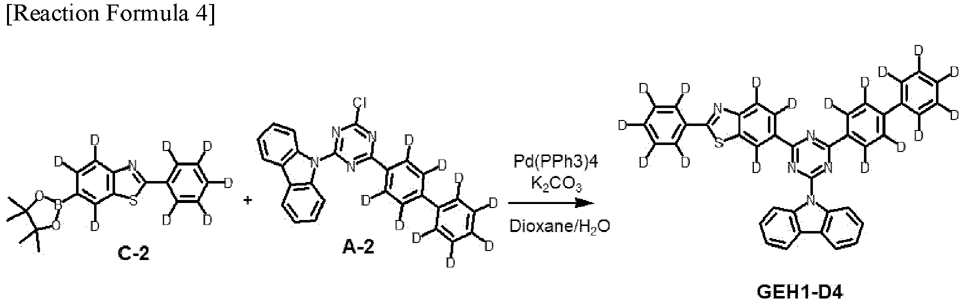

The Intermediate A-2 (10.0 g, 0.022 mol), the Intermediate C-2 (8.3 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH1-D4 (11.5 g, 81.8%).

### Synthesis Example 5: Synthesis of Compound GEH1-D5

### (1) Synthesis of Intermediate A-3

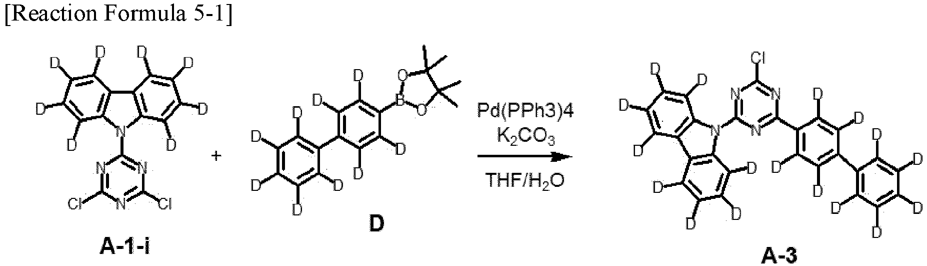

The Intermediate A-1-i (100 g, 0.32 mol), the Intemediate D (78.1 g, 0.27 mol), Pd(PPh3)4 (18 g, 0.018 mol) and K₂CO₃ (88 g, 0.63 mol) in a mixed solvent (THF 1000 ml, water 200 ml) were added into a round bottom flask with one neck, then the solution was refluxed at 70°C for 2 hours. After the reaction solution was cooled to a room temperature, excessive methanol was poured into the flask to preicipiate a product completely and then the product was filtered. The preicipitated product was heated with DCB to be dissoved completly, and then subjected to silica fliter using CF. The filtered solution was concentrated, subejcted to methanol slurry and filrtered to give an Intermediate A-3 (89.0 g, 65%).

### (2) Synthesis of Compound GEH1-D5

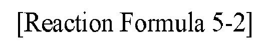

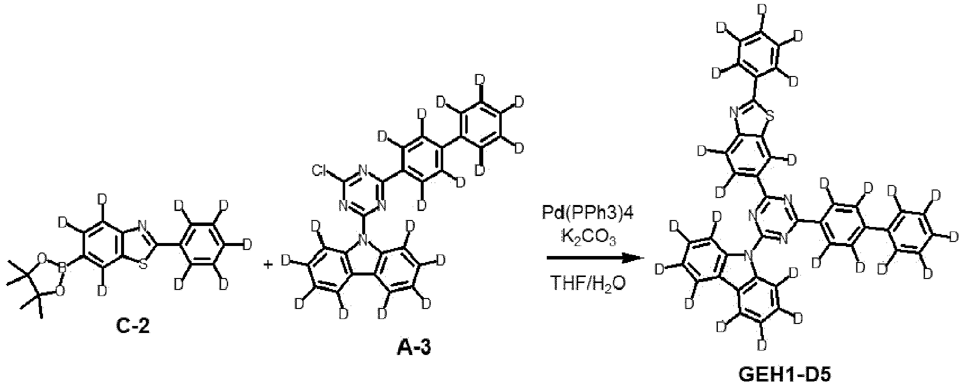

The Intermediate A-3 (10.0 g, 0.022 mol), the Intermediate C-2 (8.5 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH1-D5 (10.0 g, 71.1%).

### Synthesis Example 6: Synthesis of Compound GEH2-D1

### (1) Synthesis of Intermediate B-1

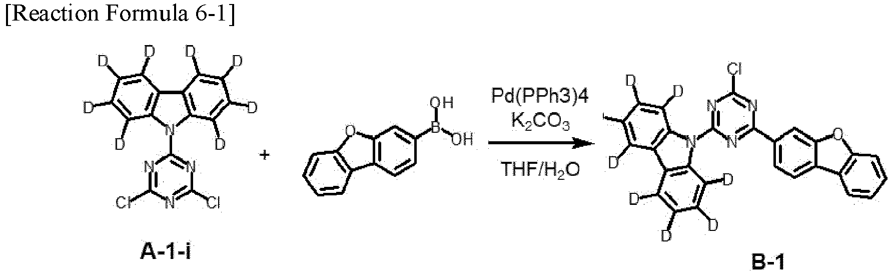

The Intermediate A-1-i (100 g, 0.32 mol), Dibenzofuran-3-ylboronic acid (63.0 g, 0.27 mol), Pd(PPh3)4 (18 g, 0.018 mol) and K₂CO₃ (88 g, 0.63 mol) in a mixed solvent (THF 2000 ml, water 400 ml) were added into a round bottom flask with one neck, then the solution was refluxed at 70°C for 2 hours. After the reaction solution was cooled to a room temperature, excessive methanol was poured into the flask to precipitate a procut completely, and the product was filtered. The precipitated product was heated with DCB to be dissolved completely, and then subjected to silica filter using CF. The filtered solution was concentrated, subject to methanol slurry and filtered to give an Intermediate B-1 (94 g, 69%).

### (2) Synthesis of Compound GEH2-D1

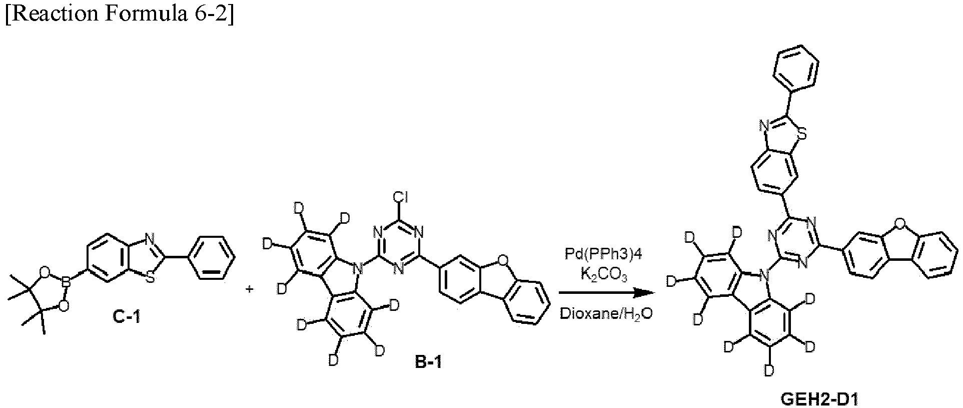

The Intermediate B-1 (10.0 g, 0.022 mol), the Intermediate C-1 (7.8 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH2-D1 (11.2 g, 80.9%).

### Synthesis Example 7: Synthesis of Compound GEH2-D2

### (1) Synthesis of Intermediate E-1

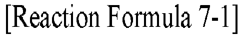

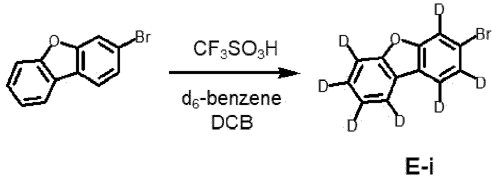

3-boromodibenzo[b,d]furan (32.1 g, 0.13 mol) in DCB (300 ml) was added into a round bottom flask with one neck, then d₆-benzene (300 ml) was added into the flask. Trifluoro methane sulfonic acid (84 ml, 0.91 mol) was added slowly into the flast, then the raction was performed at room temperature for 3 hours. The reaction solution was cooled sufficeintly using ice bath and K₃PO₄ aqeuous solution was slowly into the flask to terminate the reaction. The solution was extrcted with MC/H₂O twice, and the extracted solution was concentrated. The concentrated product was dissolved in MC again and subjected to silca gel filter (eluent: Hex: MC = 1: 1). The filtered solution was concentrated and dried to give an Intermediate E-i (30 g, 95%).

### (2) Synthesis of Intermediate E

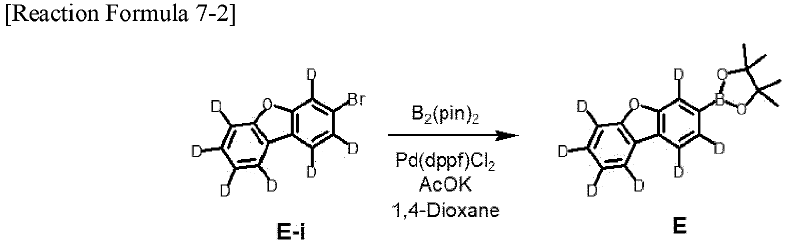

The Intermediate E-i (32.1 g, 0.12 mol), Pd(dppf)Cl₂ (4.5 g, 0.006 mol), Potassium acetate (24.3 g, 0.25 mol) and B₂(pin)₂ (47 g, 0.185 mol) in 1,4-Dioxane (300 ml) were added into a round bottom flask with one neck, then the solution was refluxed for 3 hours. Afther the reaction solution was cooled to a room temperature sufficiently, the solution was fitered, washed with MC and concentrated. The concentrated product was dissolved in MC again, and subjected to silica gel filter using MC. The filtered solution was concentrated and then subjected to methanol slurry to precipitate a solid as an Intermediate E (27.3 g, 75%).

### (3) Synthesis of Intermediate B-2

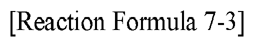

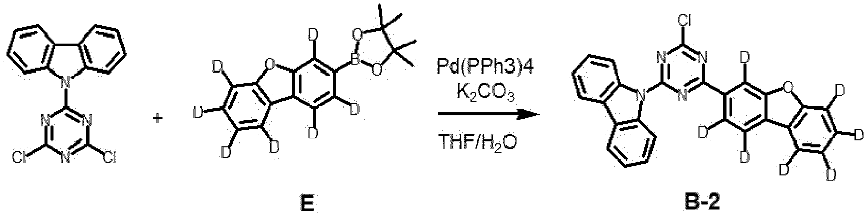

9-(4,6-dichloro-1,3-5-triazin-2-yl)carbazole (100 g, 0.32 mol), the Intemediate E (81.3 g, 0.27 mol), Pd(PPh3)4 (17 g, 0.016 mol) and K₂CO₃ (90 g, 0.64 mol) in a mixed solvent (THF 1000 ml, water 200 ml) were added into a round bottom flask with one neck, then the solution was refluxed at 70°C for 2 hours. After the reaction solution was cooled to a room temperature, excessive methanol was poured into the flask to preicipiate a product completely and then the product was filtered. The preicipitated product was heated with DCB to be dissoved completly, and then subjected to silica fliter using CF. The filtered solution was concentrated, subejcted to methanol slurry and filrtered to give an Intermediate B-2 (91.0 g, 67%).

### (4) Synthesis of Compound GEH2-D2

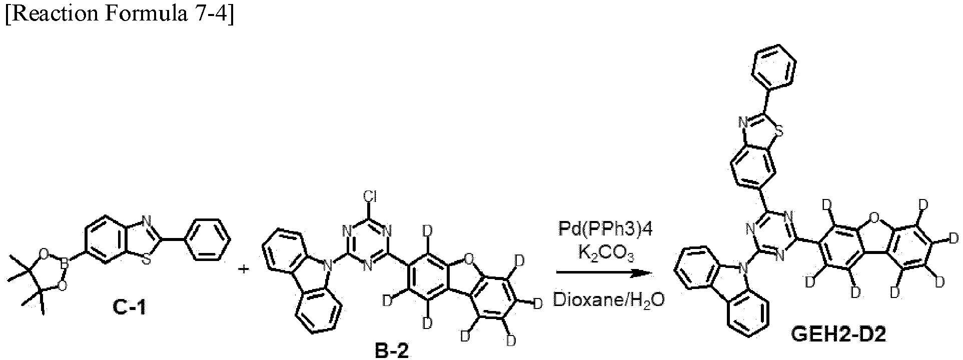

The Intermediate B-2 (10.0 g, 0.022 mol), the Intermediate C-1 (7.8 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH2-D2 (8.9 g, 69.7%).

### Synthesis Example 8: Synthesis of Compound GEH2-D3

### (1) Synthesis of Intermediate B

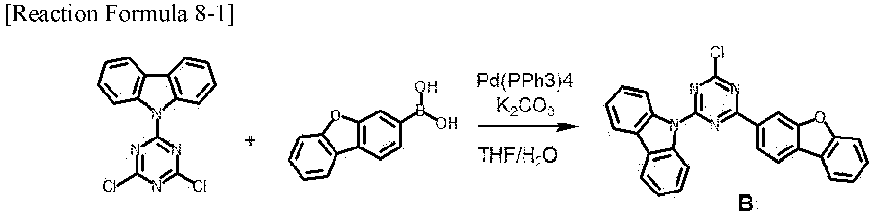

9-(4,6-dichloro-1,3,5-triazin-2-yl)carbazole (50 g, 0.16 mol), Dibenzofuran-3-ylboronic acid (31.5 g, 0.145 mol), Pd(PPh3)4 (9 g, 0.009 mol) and K₂CO₃ (44 g, 0.315 mol) in a mixed solvent (THF 1000 ml, water 200 ml) were added into a round bottom flask with one neck, then the solution was refluxed at 70°C for 2 hours. After the reaction solution was cooled to a room temperature, excessive methanol was poured into the flask to preicipiate a product completely and then the product was filtered. The preicipitated product was heated with DCB to be dissoved completly, and then subjected to silica fliter using CF. The filtered solution was concentrated, subejcted to methanol slurry and filrtered to give an Intermediate B (47 g, 69%).

### (2) Synthesis of Compound GEH2-D3

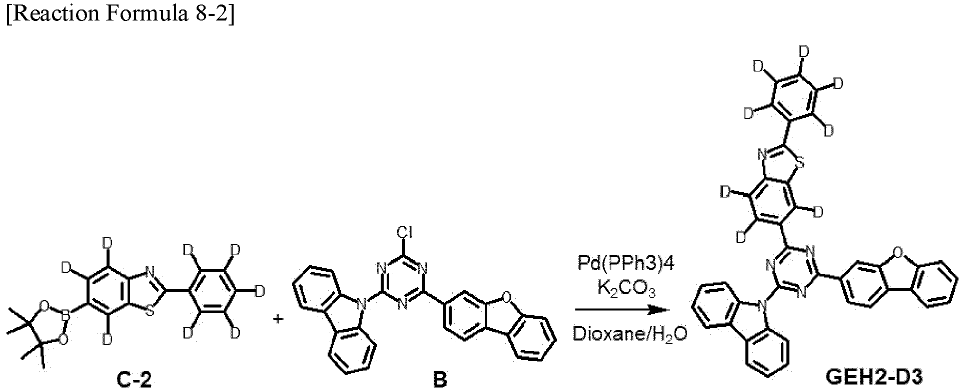

The Intermediate B (10.0 g, 0.022 mol), the Intermediate C-2 (7.9 g, 0.024 mol), Pd(PPh3)4 (1.3 g, 0.0012 mol) and K₂CO₃ (5.9 g, 0.043 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH2-D3 (10.0 g, 71.0%).

### Synthesis Example 9: Synthesis of Compound GEH2-D4

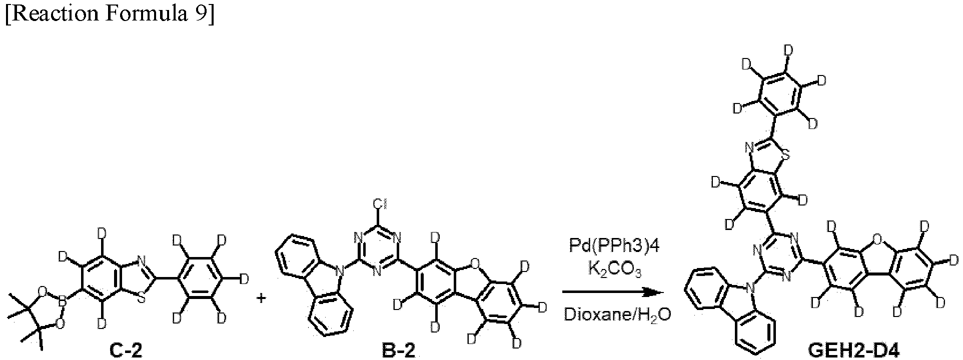

The Intermediate B-2 (10.0 g, 0.022 mol), the Intermediate C-2 (7.8 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH2-D4 (11.2 g, 80.9%).

### Synthesis Example 10: Synthesis of Compound GEH2-D5

### (1) Synthesis of Intermediate B-3

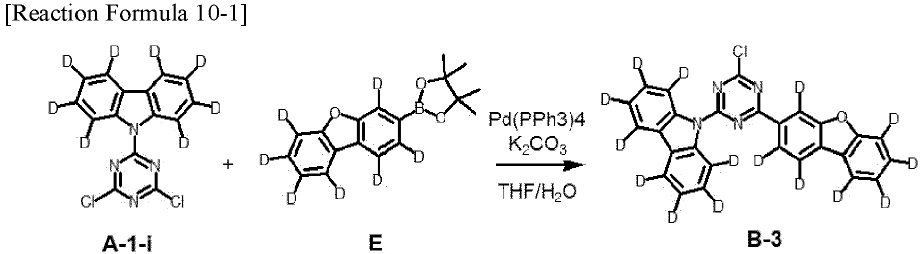

The Intermediate A-1-i (100 g, 0.32 mol), the Intermediate E (81.3 g, 0.27 mol), Pd(PPh3)4 (17 g, 0.016 mol) and K₂CO₃ (90 g, 0.64 mol) in a mixed solvent (THF 1000 ml, water 200 ml) were added into a round bottom flask with one neck, then the solution was refluxed at 70°C for 2 hours. After the reaction solution was cooled to a room temperature, excessive methanol was poured into the flask to precipitate a procut completely, and the product was filtered. The precipitated product was heated with DCB to be dissolved completely, and then subjected to silica filter using CF. The filtered solution was concentrated, subject to methanol slurry and filtered to give an Intermediate B-3 (84.0 g, 62%).

### (2) Synthesis of Compound GEH2-D5

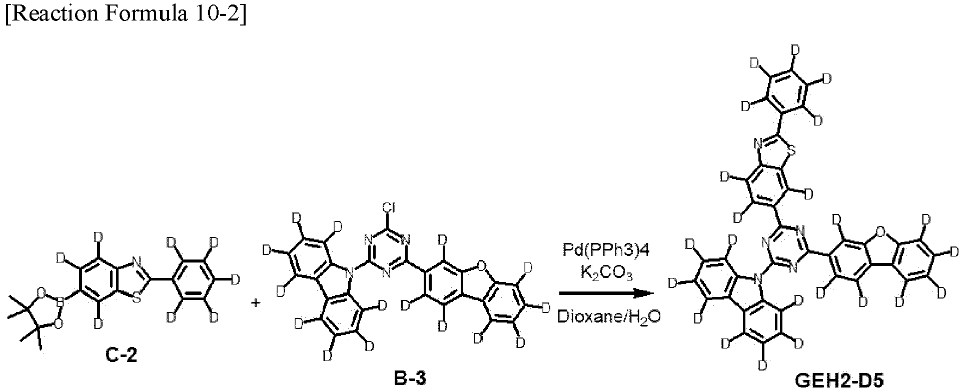

The Intermediate B-3 (10.0 g, 0.0215 mol), the Intermediate C-2 (8.2 g, 0.023 mol), Pd(PPh3)4 (1.2 g, 0.0011 mol) and K₂CO₃ (5.4 g, 0.04 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH2-D5 (11.2 g, 80.2%).

### Synthesis Example 11: Synthesis of Compound GEH3-D1

### (1) Synthesis of Intermediate C-3

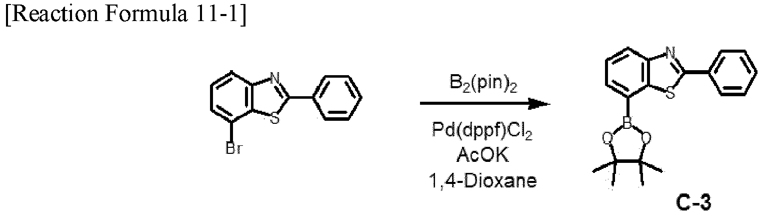

7-bromo-2-phenyl-1,3-benzothiazole (20 g, 0.072 mol), Pd(dppf)Cl₂ (2.6 g, 0.004 mol), Potassium acetate (14.3 g, 0.14 mol) and B₂(pin)₂ (27.7 g, 0.11 mol) in 1,4-Dioxane (250 ml) were added into a round bottom flask with one neck, then the solution was refluxed for 3 hours. Afther the reaction solution was cooled to a room temperature sufficiently, the solution was fitered, washed with MC and concentrated. The concentrated product was dissolved in MC again, and subjected to silica gel filter using MC. The filtered solution was concentrated and then subjected to methanol slurry to precipitate a solid as an Intermediate C-3 (21 g, 90%).

### (2) Synthesis of Compound GEH3-D1

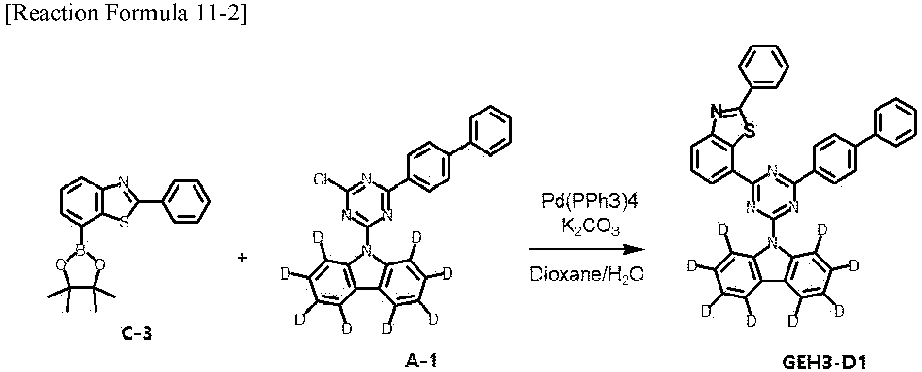

The Intermediate A-1 (10.0 g, 0.022 mol), the Intermediate C-3 (7.8 g, 0.024 mol), Pd(PPh3)4 (1.2 g, 0.0011 mol) and K₂CO₃ (6.1 g, 0.044 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH3-D1 (9.1 g, 65.2%).

### Synthesis Example 12: Synthesis of Compound GEH3-D2

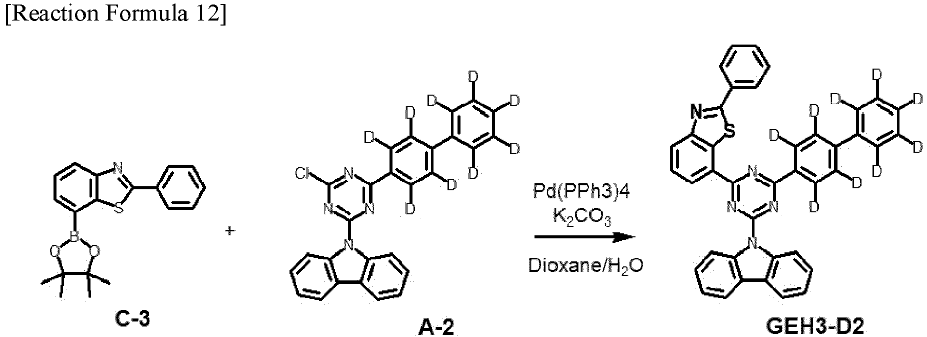

The Intermediate A-2 (10.0 g, 0.022 mol), the Intermediate C-3 (7.8 g, 0.024 mol), Pd(PPh3)4 (1.2 g, 0.0011 mol) and K₂CO₃ (6.1 g, 0.044 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH3-D2 (9.1 g, 65.2%).

### Synthesis Example 13: Synthesis of Compound GEH3-D3

### (1) Synthesis of Intermediate C-4-i

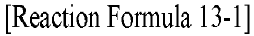

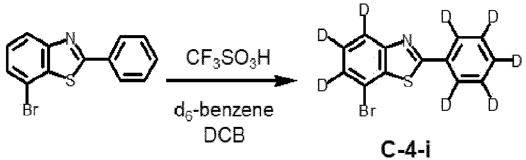

7-boromo-2-phenyl-1,3-benzothiazole (30 g, 0.10 mol) in DCB (300 ml) was added into a round bottom flask with one neck, then d₆-benzene (300 ml) was added into the flask. Trifluoro methane sulfonic acid (120 ml, 1.3 mol) was added slowly into the flast, then the raction was performed at 60°C for 12 hours. The reaction solution was cooled sufficeintly using ice bath and K₃PO₄ aqeuous solution was slowly into the flask to terminate the reaction. The solution was extrcted with MC/H₂O twice, and the extracted solution was concentrated. The concentrated product was dissolved in MC again and subjected to silca gel filter (eluent: Hex: MC = 1:1). The filtered solution was concentrated and dried to give an Intermediate C-4-i (28 g, 92%).

### (2) Synthesis of Intermediate C-4

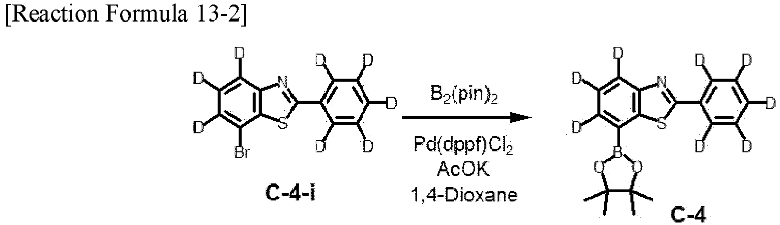

The Intermediate C-4-i (28.0 g, 0.09 mol), Pd(dppf)Cl₂ (3.3 g, 0.0045 mol), Potassium acetate (17.8 g, 0.18 mol) and B₂(pin)₂ (35 g, 0.135 mol) in 1,4-Dioxane (300 ml) were added into a round bottom flask with one neck, then the solution was refluxed for 3 hours. Afther the reaction solution was cooled to a room temperature sufficiently, the solution was fitered, washed with MC and concentrated. The concentrated product was dissolved in MC again, and subjected to silica gel filter using MC. The filtered solution was concentrated and then subjected to H₂O/MeOH slurry to precipitate a solid as an Intermediate C-4 (21.2 g, 68%).

### (3) Synthesis of Compound GEH3-D3

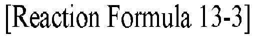

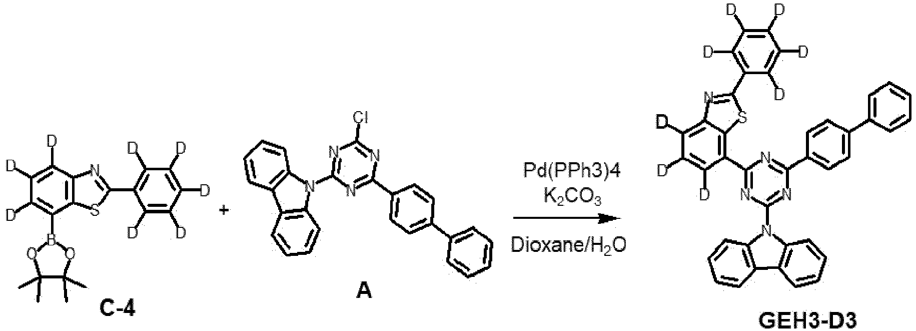

The Intermediate A (10.0 g, 0.022 mol), the Intermediate C-4 (7.8 g, 0.024 mol), Pd(PPh3)4 (1.2 g, 0.0011 mol) and K₂CO₃ (6.1 g, 0.044 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH3-D3 (9.1 g, 65.2%).

### Synthesis Example 14: Synthesis of Compound GEH3-D4

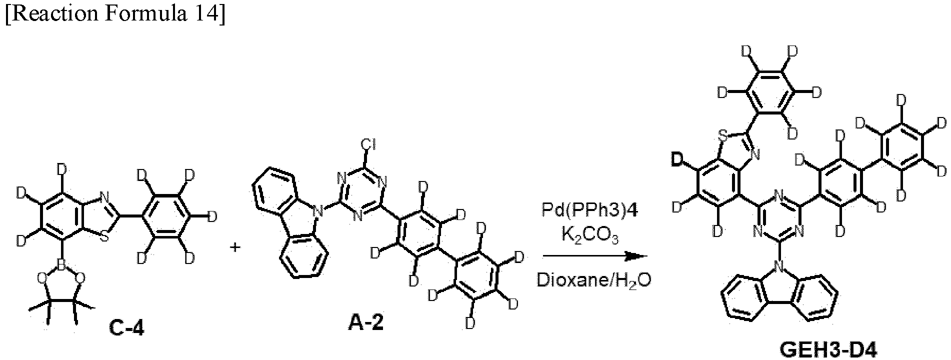

The Intermediate A-2 (10.0 g, 0.022 mol), the Intermediate C-4 (8.3 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH3-D4 (11.0 g, 78.2%).

### Synthesis Example 15: Synthesis of Compound GEH3-D5

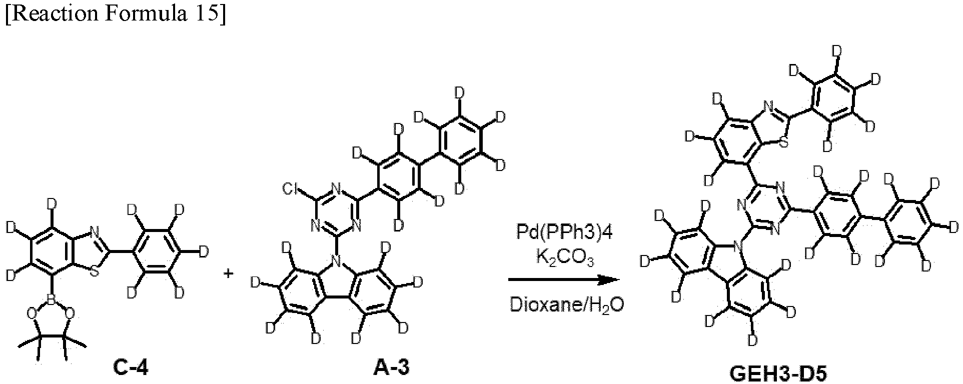

The Intermediate A-3 (10.0 g, 0.022 mol), the Intermediate C-4 (8.5 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH3-D5 (9.5 g, 67.5%).

### Synthesis Example 16: Synthesis of Compound GEH4-D1

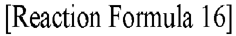

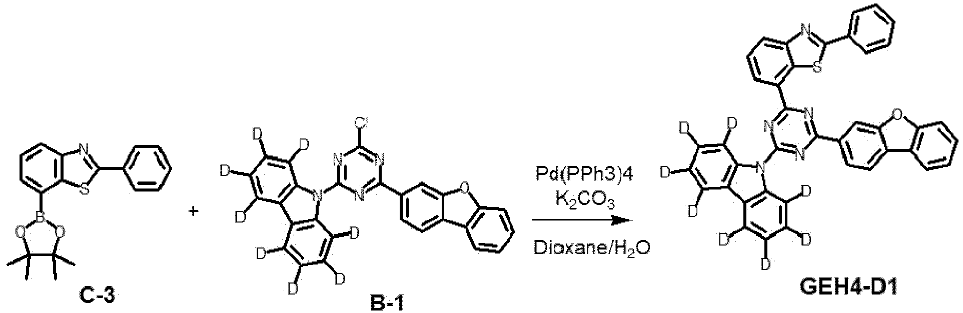

The Intermediate B-1 (10.0 g, 0.022 mol), the Intermediate C-3 (7.8 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH4-D1 (10.6 g, 83.0%).

### Synthesis Example 17: Synthesis of Compound GEH4-D2

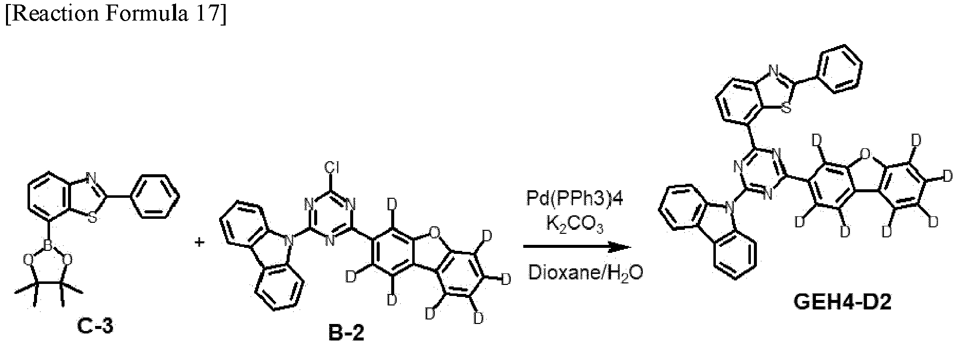

The Intermediate B-2 (10.0 g, 0.022 mol), the Intermediate C-3 (7.8 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH4-D2 (8.9 g, 69.7%).

### Synthesis Example 18: Synthesis of Compound GEH4-D3

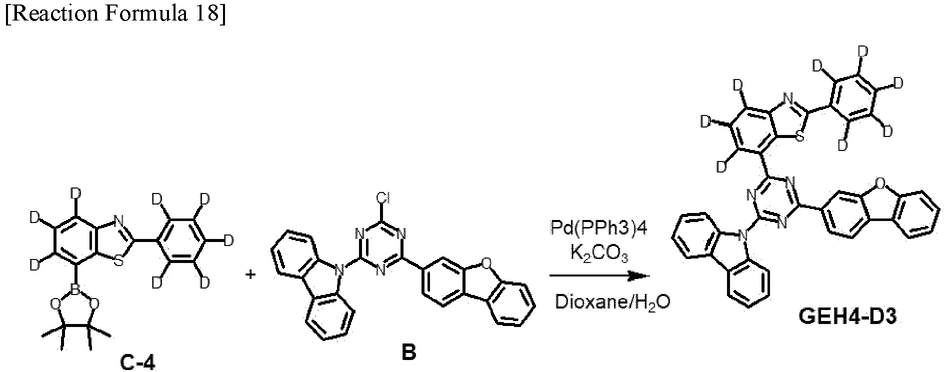

The Intermediate B (10.0 g, 0.022 mol), the Intermediate C-4 (7.9 g, 0.024 mol), Pd(PPh3)4 (1.2 g, 0.0011 mol) and K₂CO₃ (6.0 g, 0.043 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH4-D3 (12.2 g, 86.3%).

### Synthesis Example 19: Synthesis of Compound GEH4-D4

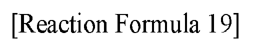

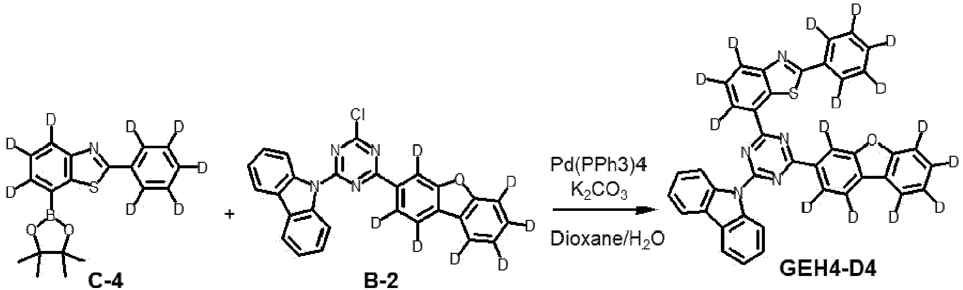

The Intermediate B-2 (10.0 g, 0.022 mol), the Intermediate C-4 (7.9 g, 0.024 mol), Pd(PPh3)4 (1.2 g, 0.0011 mol) and K₂CO₃ (6.0 g, 0.043 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH4-D4 (12.2 g, 86.3%).

### Synthesis Example 20: Synthesis of Compound GEH4-D5

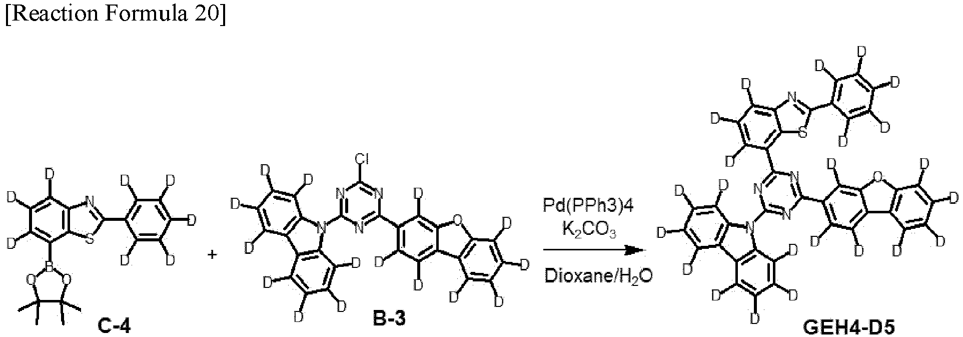

The Intermediate B-3 (10.0 g, 0.0215 mol), the Intermediate C-4 (8.2 g, 0.023 mol), Pd(PPh3)4 (1.2 g, 0.0011 mol) and K₂CO₃ (5.4 g, 0.04 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH4-D5 (10.4 g, 74.5%).

### Synthesis Example 21: Synthesis of Compound GEH5-D1

### (1) Synthesis of Intermediate C-5

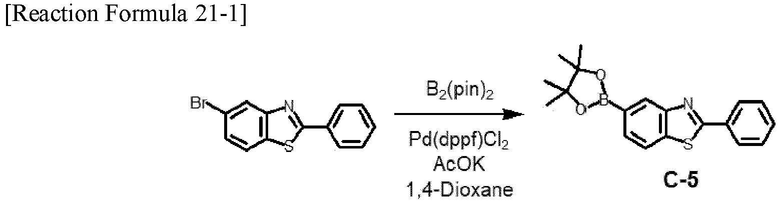

5-bromo-2-phenyl-1,3-benzothiazole (20 g, 0.072 mol), Pd(dppf)Cl₂ (2.6 g, 0.004 mol), Potassium acetate (14.3 g, 0.14 mol) and B₂(pin)₂ (27.7 g, 0.11 mol) in 1,4-Dioxane (250 ml) were added into a round bottom flask with one neck, then the solution was refluxed for 3 hours. Afther the reaction solution was cooled to a room temperature sufficiently, the solution was fitered, washed with MC and concentrated. The concentrated product was dissolved in MC again, and subjected to silica gel filter using MC. The filtered solution was concentrated and then subjected to methanol slurry to precipitate a solid as an Intermediate C-5 (21 g, 90%).

### (2) Synthesis of Compound GEH5-D1

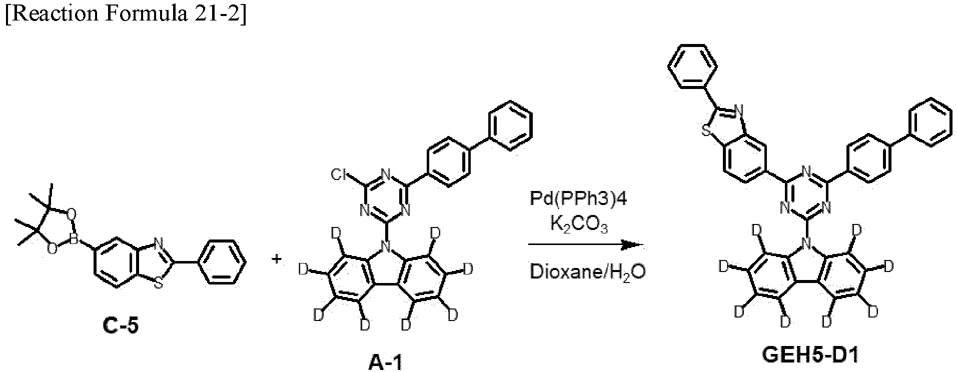

The Intermediate A-1 (10.0 g, 0.022 mol), the Intermediate C-5 (7.8 g, 0.024 mol), Pd(PPh3)4 (1.2 g, 0.0011 mol) and K₂CO₃ (6.1 g, 0.044 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH5-D1 (11.2 g, 86.1%).

### Synthesis Example 22: Synthesis of Compound GEH5-D2

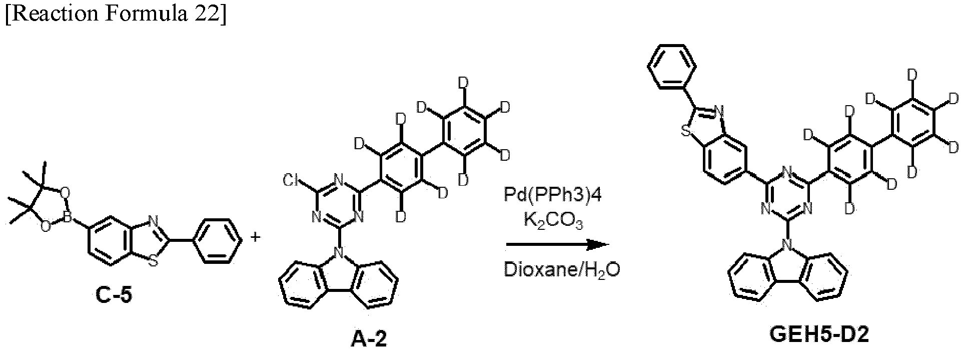

The Intermediate A-2 (10.0 g, 0.022 mol), the Intermediate C-5 (7.8 g, 0.024 mol), Pd(PPh3)4 (1.2 g, 0.0011 mol) and K₂CO₃ (6.1 g, 0.044 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH5-D2 (9.8 g, 70.0%).

### Synthesis Example 23: Synthesis of Compound GEH5-D3

### (1) Synthesis of Intermediate C-6-i

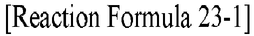

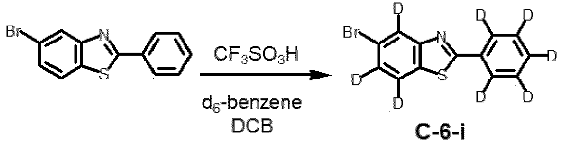

5-boromo-2-phenyl-1,3-benzothiazole (30 g, 0.10 mol) in DCB (300 ml) was added into a round bottom flask with one neck, then d₆-benzene (300 ml) was added into the flask. Trifluoro methane sulfonic acid (120 ml, 1.3 mol) was added slowly into the flast, then the raction was performed at 60°C for 12 hours. The reaction solution was cooled sufficeintly using ice bath and K₃PO₄ aqeuous solution was slowly into the flask to terminate the reaction. The solution was extrcted with MC/H₂O twice, and the extracted solution was concentrated. The concentrated product was dissolved in MC again and subjected to silca gel filter (eluent: Hex: MC = 1:1). The filtered solution was concentrated and dried to give an Intermediate C-6-i (29 g, 90%).

### (2) Synthesis of Intermediate C-6

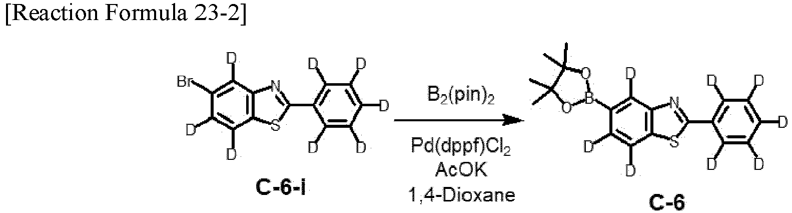

The Intermediate C-6-i (29.0 g, 0.10 mol), Pd(dppf)Cl₂ (3.5 g, 0.005 mol), Potassium acetate (18.8 g, 0.20 mol) and B₂(pin)₂ (40 g, 0.15 mol) in 1,4-Dioxane (300 ml) were added into a round bottom flask with one neck, then the solution was refluxed for 4 hours. Afther the reaction solution was cooled to a room temperature sufficiently, the solution was fitered, washed with MC and concentrated. The concentrated product was dissolved in MC again, and subjected to silica gel filter using MC. The filtered solution was concentrated and then subjected to H₂O/MeOH slurry to precipitate a solid as an Intermediate C-6 (25 g, 75%).

### (3) Synthesis of Compound GEH5-D3

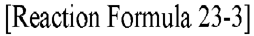

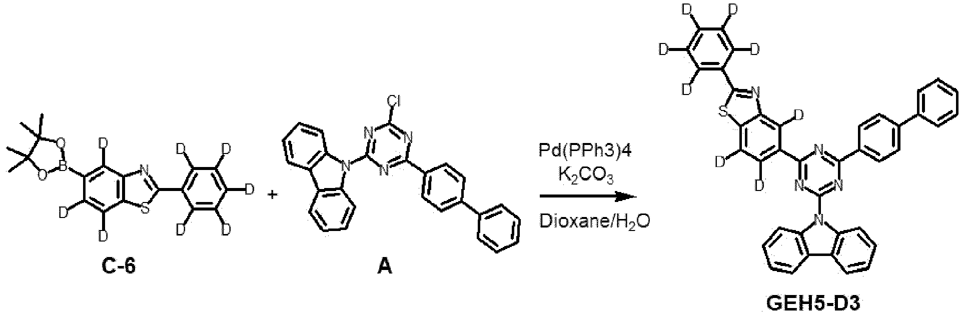

The Intermediate A (10.0 g, 0.023 mol), the Intermediate C-6 (8.7 g, 0.025 mol), Pd(PPh3)4 (1.4 g, 0.0012 mol) and K₂CO₃ (6.3 g, 0.045 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH5-D3 (10.8 g, 75.9%).

### Synthesis Example 24: Synthesis of Compound GEH5-D4

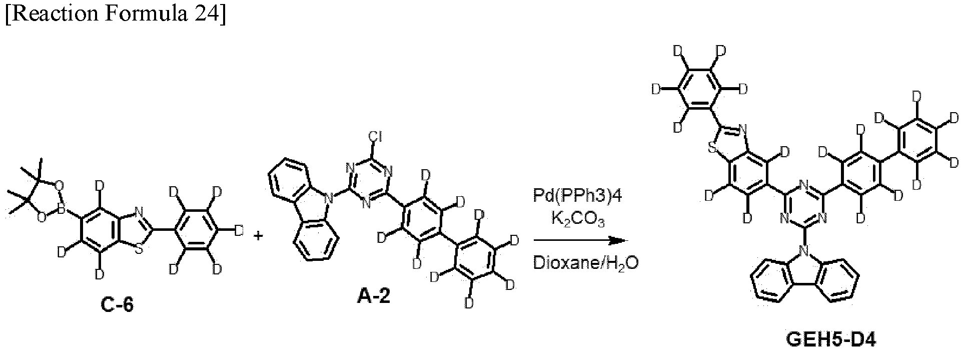

The Intermediate A-2 (10.0 g, 0.022 mol), the Intermediate C-6 (8.3 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH5-D4 (11.7 g, 83.2%).

### Synthesis Example 25: Synthesis of Compound GEH5-D5

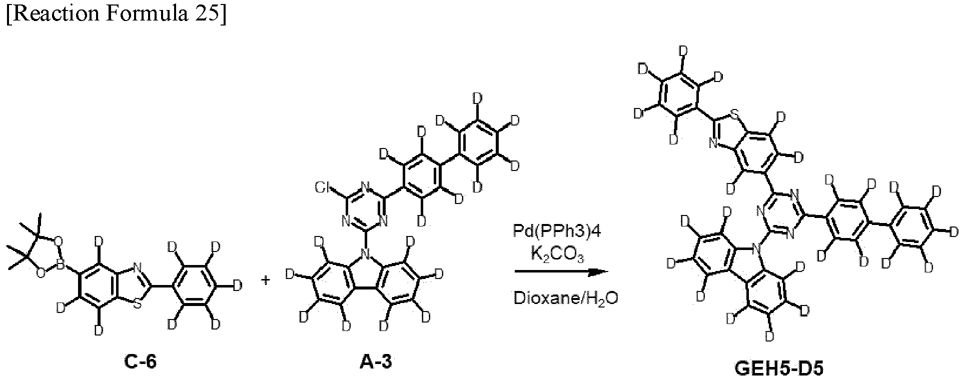

The Intermediate A-3 (10.0 g, 0.022 mol), the Intermediate C-6 (8.5 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH5-D5 (8.2 g, 58.3%).

### Synthesis Example 26: Synthesis of Compound GEH6-D1

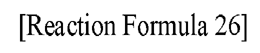

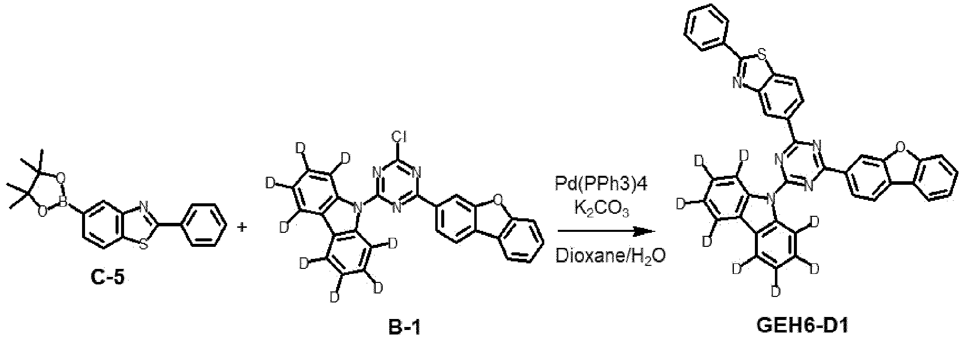

The Intermediate B-1 (10.0 g, 0.022 mol), the Intermediate C-5 (7.8 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH6-D1 (10.1 g, 73.0%).

### Synthesis Example 27: Synthesis of Compound GEH6-D2

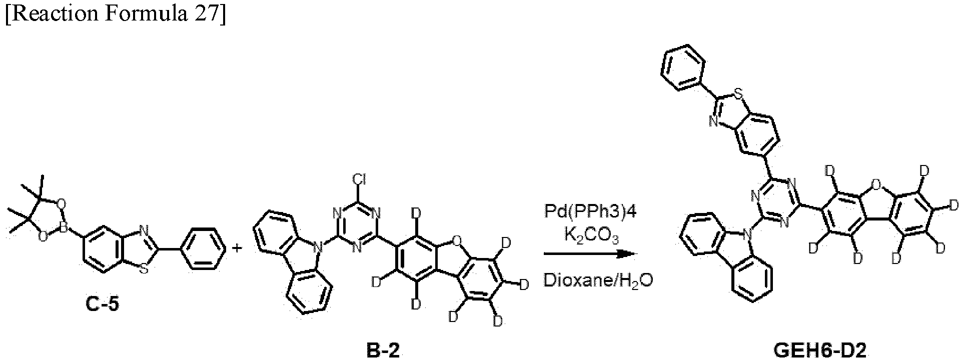

The Intermediate B-2 (9.4 g, 0.021 mol), the Intermediate C-5 (7.68 g, 0.023 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH6-D2 (10.6 g, 76.6%).

### Synthesis Example 28: Synthesis of Compound GEH6-D3

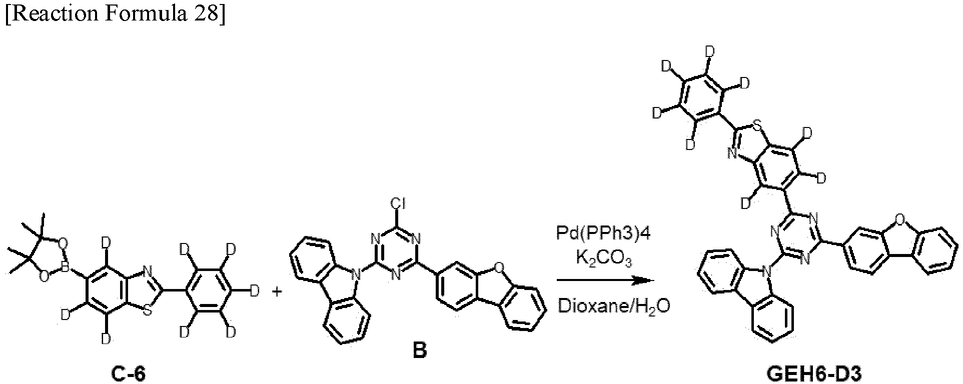

The Intermediate B (10.0 g, 0.022 mol), the Intermediate C-6 (7.9 g, 0.024 mol), Pd(PPh3)4 (1.2 g, 0.0011 mol) and K₂CO₃ (6.0 g, 0.043 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH6-D3 (9.7 g, 68.7%).

### Synthesis Example 29: Synthesis of Compound GEH6-D4

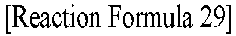

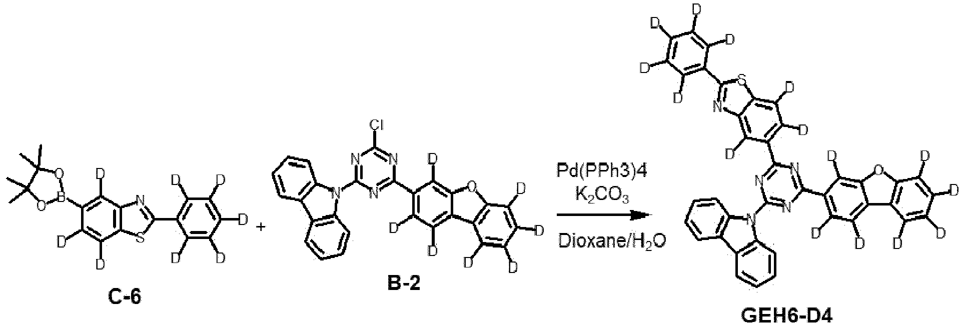

The Intermediate B-2 (10.0 g, 0.022 mol), the Intermediate C-6 (7.68 g, 0.023 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH6-D4 (10.6 g, 83.0%).

### Synthesis Example 30: Synthesis of Compound GEH6-D5

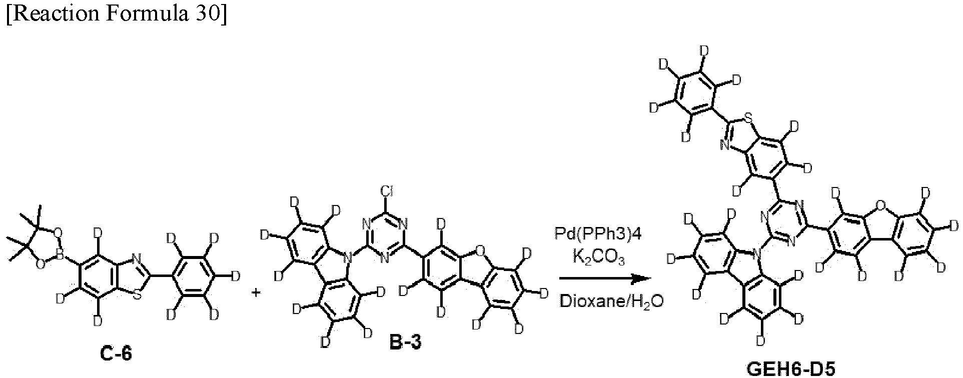

The Intermediate B-3 (10.0 g, 0.0215 mol), the Intermediate C-6 (8.2 g, 0.023 mol), Pd(PPh3)4 (1.2 g, 0.0011 mol) and K₂CO₃ (5.4 g, 0.04 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH6-D5 (11.2 g, 83.2%).

### Synthesis Example 31: Synthesis of Compound GEH7-D1

### (1) Synthesis of Intermediate C-7

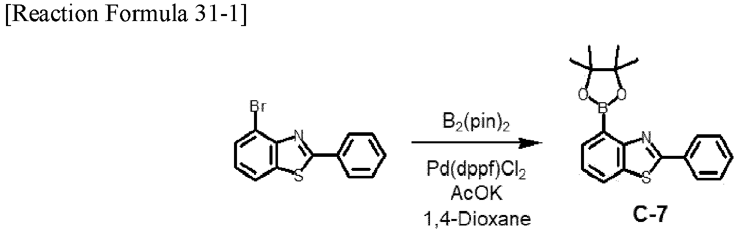

4-bromo-2-phenyl-1,3-benzothiazole (20 g, 0.072 mol), Pd(dppf)Cl₂ (2.6 g, 0.004 mol), Potassium acetate (14.3 g, 0.14 mol) and B₂(pin)₂ (27.7 g, 0.11 mol) in 1,4-Dioxane (250 ml) were added into a round bottom flask with one neck, then the solution was refluxed for 3 hours. Afther the reaction solution was cooled to a room temperature sufficiently, the solution was fitered, washed with MC and concentrated. The concentrated product was dissolved in MC again, and subjected to silica gel filter using MC. The filtered solution was concentrated and then subjected to methanol slurry to precipitate a solid as an Intermediate C-7 (21 g, 90%).

### (2) Synthesis of Compound GEH7-D1

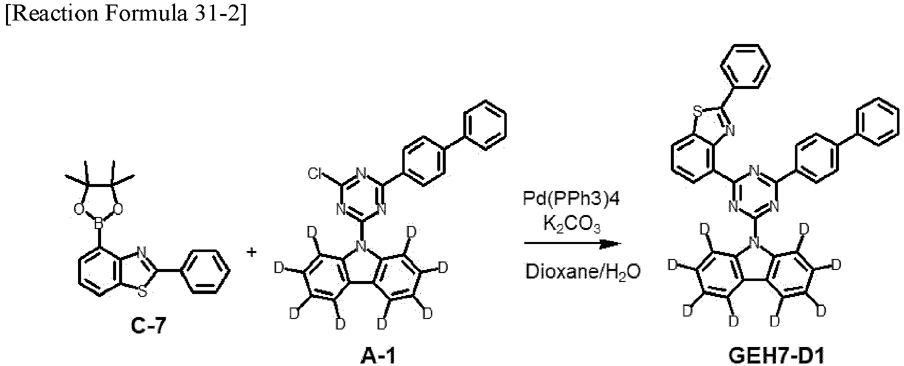

The Intermediate A-1 (10.0 g, 0.022 mol), the Intermediate C-7 (7.8 g, 0.024 mol), Pd(PPh3)4 (1.2 g, 0.0011 mol) and K₂CO₃ (6.1 g, 0.044 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH7-D1 (9.9 g, 70.9%).

### Synthesis Example 32: Synthesis of Compound GEH7-D2

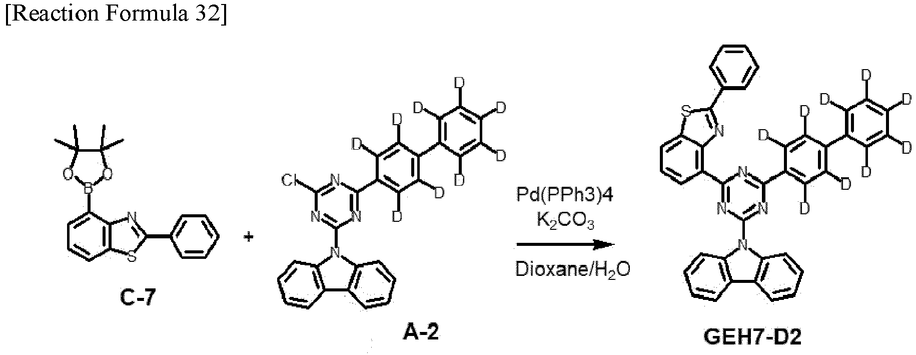

The Intermediate A-2 (10.0 g, 0.022 mol), the Intermediate C-7 (7.8 g, 0.024 mol), Pd(PPh3)4 (1.2 g, 0.0011 mol) and K₂CO₃ (6.1 g, 0.044 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH7-D2 (9.7 g, 69.4%).

### Synthesis Example 33: Synthesis of Compound GEH7-D3

### (1) Synthesis of Intermediate C-8-i

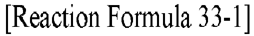

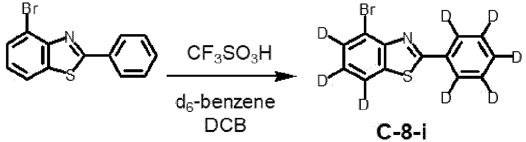

4-boromo-2-phenyl-1,3-benzothiazole (30 g, 0.10 mol) in DCB (300 ml) was added into a round bottom flask with one neck, then d₆-benzene (300 ml) was added into the flask. Trifluoro methane sulfonic acid (120 ml, 1.3 mol) was added slowly into the flast, then the raction was performed at 60°C for 12 hours. The reaction solution was cooled sufficeintly using ice bath and K₃PO₄ aqeuous solution was slowly into the flask to terminate the reaction. The solution was extrcted with MC/H₂O twice, and the extracted solution was concentrated. The concentrated product was dissolved in MC again and subjected to silca gel filter (eluent: Hex: MC = 1:1). The filtered solution was concentrated and dried to give an Intermediate C-8-i (27 g, 88%).

### (2) Synthesis of Intermediate C-8

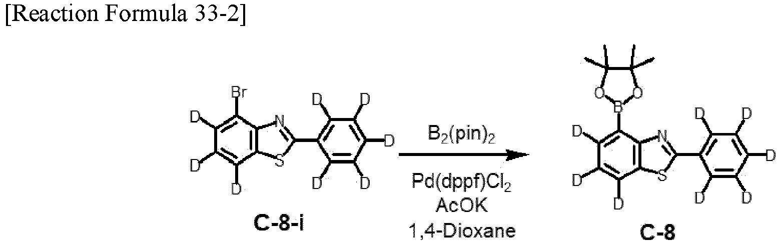

The Intermediate C-8-i (27.0 g, 0.09 mol), Pd(dppf)Cl₂ (3.3 g, 0.0045 mol), Potassium acetate (17.8 g, 0.18 mol) and B₂(pin)₂ (35 g, 0.135 mol) in 1,4-Dioxane (300 ml) were added into a round bottom flask with one neck, then the solution was refluxed for 4 hours. Afther the reaction solution was cooled to a room temperature sufficiently, the solution was fitered, washed with MC and concentrated. The concentrated product was dissolved in MC again, and subjected to silica gel filter using MC. The filtered solution was concentrated and then subjected to H₂O/MeOH slurry to precipitate a solid as an Intermediate C-8 (23 g, 74%).

### (3) Synthesis of Compound GEH7-D3

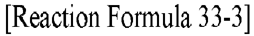

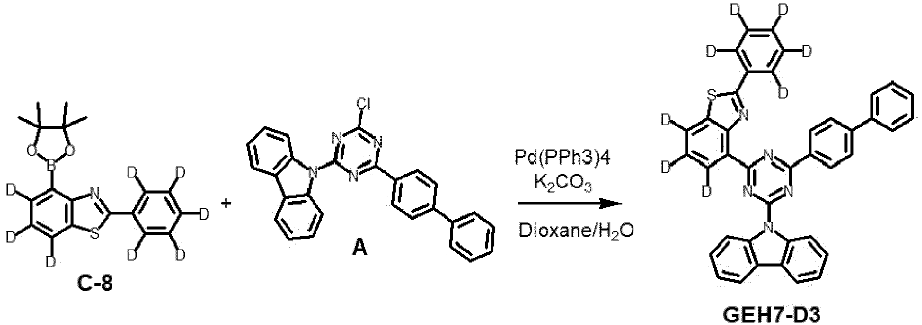

The Intermediate A (10.0 g, 0.023 mol), the Intermediate C-8 (8.7 g, 0.025 mol), Pd(PPh3)4 (1.4 g, 0.0012 mol) and K₂CO₃ (6.3 g, 0.045 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH7-D3 (10.3 g, 74.5%).

### Synthesis Example 34: Synthesis of Compound GEH7-D4

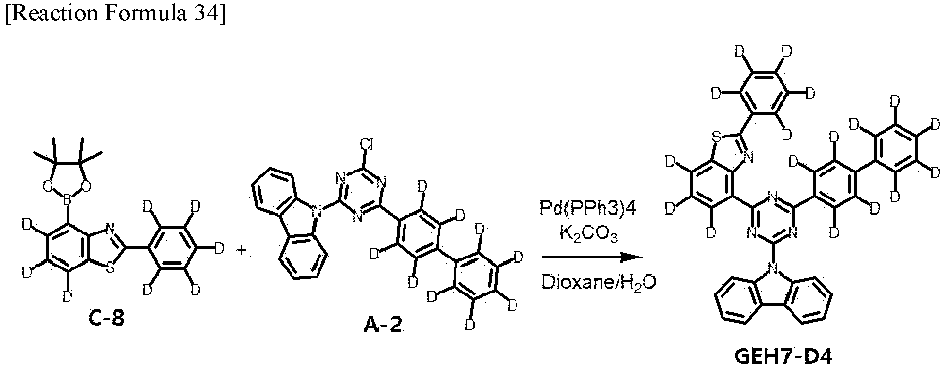

The Intermediate A-2 (10.0 g, 0.022 mol), the Intermediate C-8 (8.3 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH7-D4 (9.5 g, 67.6%).

### Synthesis Example 35: Synthesis of Compound GEH7-D5

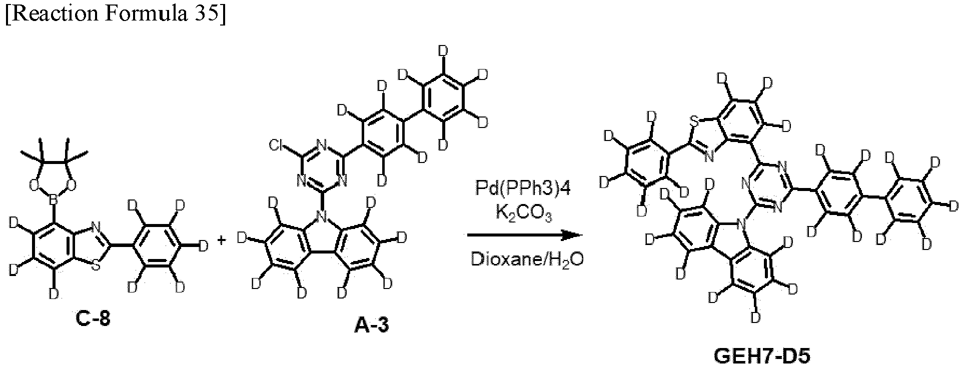

The Intermediate A-3 (10.0 g, 0.022 mol), the Intermediate C-8 (7.68 g, 0.023 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH7-D5 (8.8 g, 62.6%).

### Synthesis Example 36: Synthesis of Compound GEH8-D1

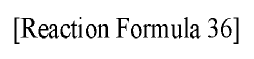

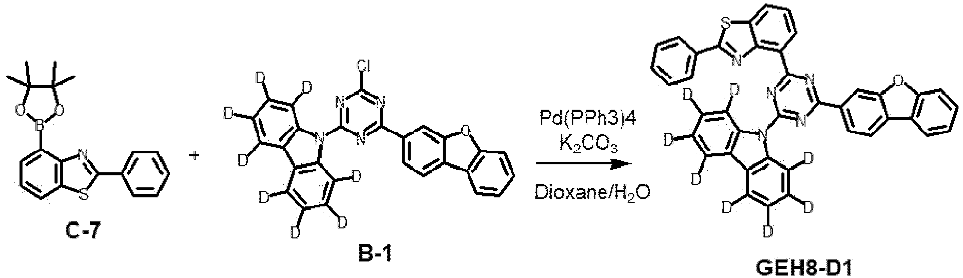

The Intermediate B-1 (10.0 g, 0.022 mol), the Intermediate C-7 (7.8 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH8-D1 (8.9 g, 64.5%).

### Synthesis Example 37: Synthesis of Compound GEH8-D2

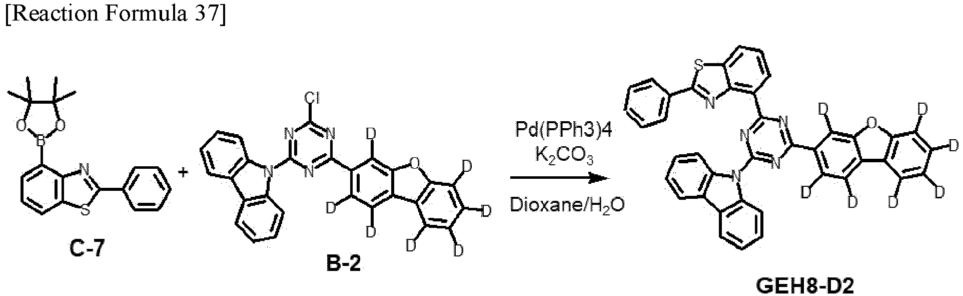

The Intermediate B-2 (9.4 g, 0.021 mol), the Intermediate C-7 (7.8 g, 0.023 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH8-D2 (10.6 g, 83.0%).

### Synthesis Example 38: Synthesis of Compound GEH8-D3

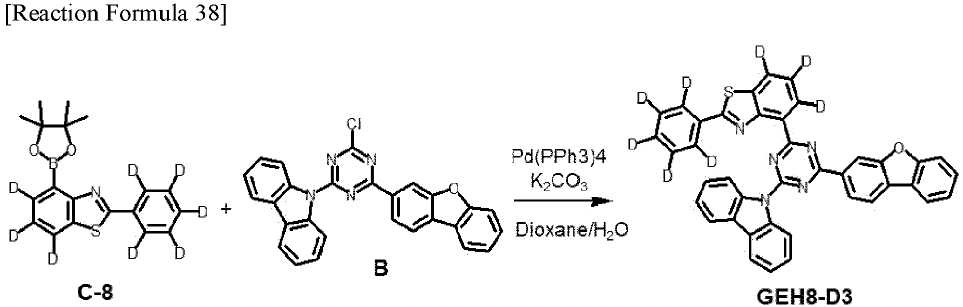

The Intermediate B (10.0 g, 0.022 mol), the Intermediate C-8 (7.9 g, 0.024 mol), Pd(PPh3)4 (1.2 g, 0.0011 mol) and K₂CO₃ (6.0 g, 0.043 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH8-D3 (9.8 g, 69.4%).

### Synthesis Example 39: Synthesis of Compound GEH8-D4

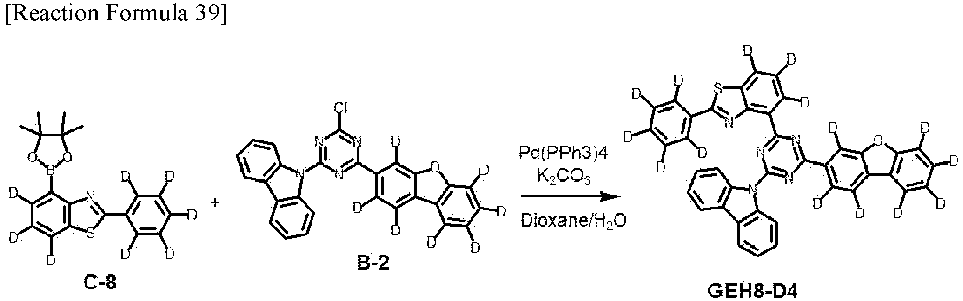

The Intermediate B-2 (10.0 g, 0.022 mol), the Intermediate C-8 (7.9 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH8-D4 (9.8 g, 69.4%).

### Synthesis Example 40: Synthesis of Compound GEH8-D5

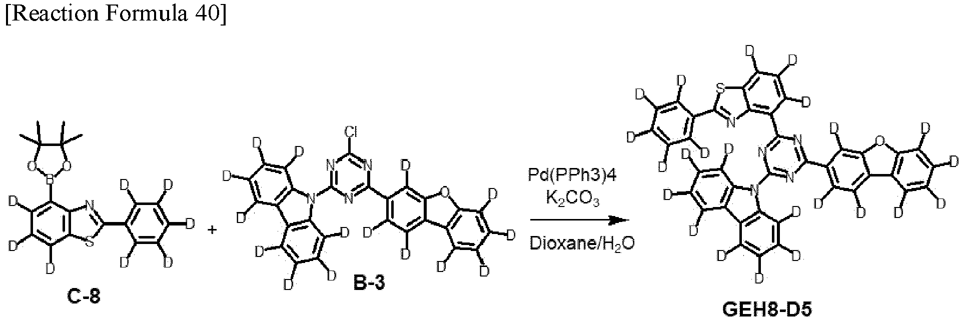

The Intermediate B-3 (9.4 g, 0.021 mol), the Intermediate C-8 (7.68 g, 0.023 mol), Pd(PPh3)4 (1.2 g, 0.0011 mol) and K₂CO₃ (5.4 g, 0.04 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH8-D5 (11.2 g, 83.2%).

### Synthesis Example 41: Synthesis of Compound GEH9-D1

### (1) Synthesis of Intermediate C-9

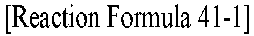

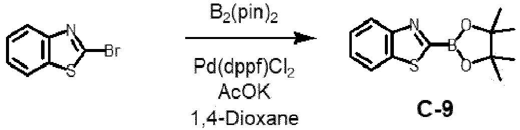

2-bromo-1,3-benzothiazole (15.4 g, 0.072 mol), Pd(dppf)Cl₂ (2.6 g, 0.004 mol), Potassium acetate (14.3 g, 0.14 mol) and B₂(pin)₂ (27.7 g, 0.11 mol) in 1,4-Dioxane (250 ml) were added into a round bottom flask with one neck, then the solution was refluxed for 3 hours. Afther the reaction solution was cooled to a room temperature sufficiently, the solution was fitered, washed with MC and concentrated. The concentrated product was dissolved in MC again, and subjected to silica gel filter using MC. The filtered solution was concentrated and then subjected to methanol slurry to precipitate a solid as an Intermediate C-9 (11.8 g, 62.5%).

### (2) Synthesis of Compound GEH9-D1

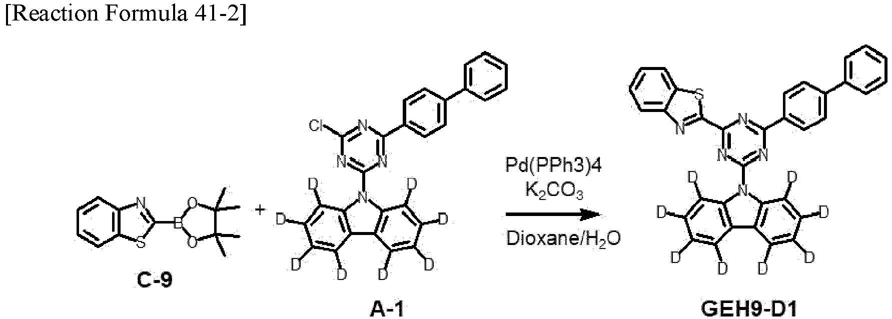

The Intermediate A-1 (10.0 g, 0.022 mol), the Intermediate C-9 (6.3 g, 0.024 mol), Pd(PPh3)4 (1.2 g, 0.0011 mol) and K₂CO₃ (6.1 g, 0.044 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH9-D1 (7.5 g, 63.1%).

### Synthesis Example 42: Synthesis of Compound GEH9-D2

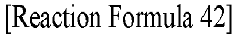

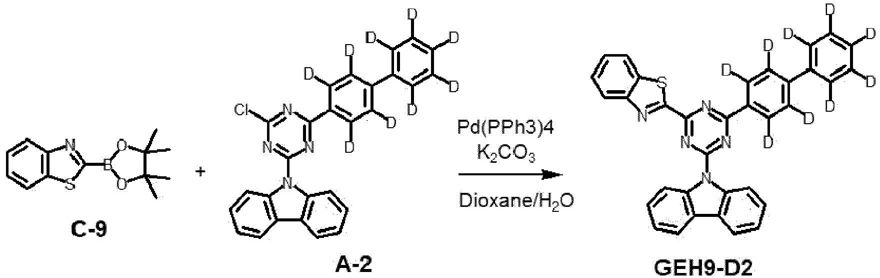

The Intermediate A-2 (10.0 g, 0.022 mol), the Intermediate C-9 (6.3 g, 0.024 mol), Pd(PPh3)4 (1.2 g, 0.0011 mol) and K₂CO₃ (6.1 g, 0.044 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH9-D2 (8.6 g, 72.4%).

### Synthesis Example 43: Synthesis of Compound GEH7-D3

### (1) Synthesis of Intermediate C-10-i

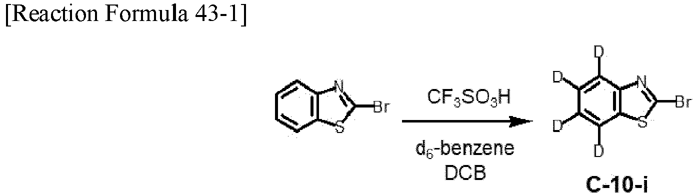

2-boromo-1,3-benzothiazole (30 g, 0.14 mol) in DCB (300 ml) was added into a round bottom flask with one neck, then d₆-benzene (300 ml) was added into the flask. Trifluoro methane sulfonic acid (168 ml, 1.8 mol) was added slowly into the flast, then the raction was performed at 60°C for 12 hours. The reaction solution was cooled sufficeintly using ice bath and K₃PO₄ aqeuous solution was slowly into the flask to terminate the reaction. The solution was extrcted with MC/H₂O twice, and the extracted solution was concentrated. The concentrated product was dissolved in MC again and subjected to silca gel filter (eluent: Hex: MC = 1:1). The filtered solution was concentrated and dried to give an Intermediate C-10-i (28 g, 91.6%).

### (2) Synthesis of Intermediate C-10

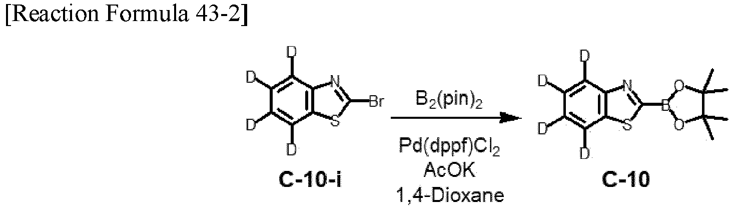

The Intermediate C-10-i (28.0 g, 0.09 mol), Pd(dppf)Cl₂ (4.7 g, 0.0064 mol), Potassium acetate (25.2 g, 0.26 mol) and B₂(pin)₂ (49 g, 0.193 mol) in 1,4-Dioxane (300 ml) were added into a round bottom flask with one neck, then the solution was refluxed for 3 hours. Afther the reaction solution was cooled to a room temperature sufficiently, the solution was fitered, washed with MC and concentrated. The concentrated product was dissolved in MC again, and subjected to silica gel filter using MC. The filtered solution was concentrated and then subjected to H₂O/MeOH slurry to precipitate a solid as an Intermediate C-10 (23 g, 74%).

### (3) Synthesis of Compound GEH9-D3

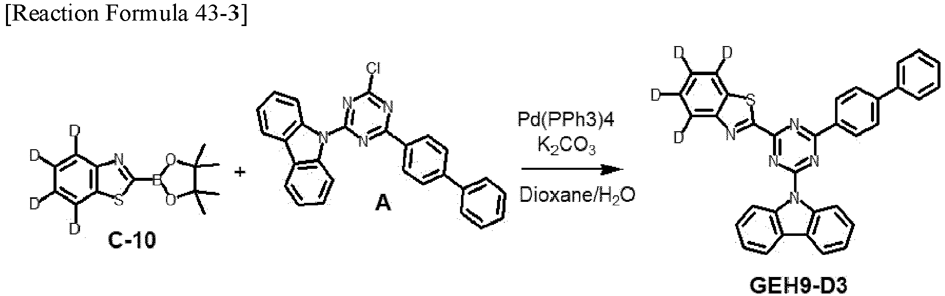

The Intermediate A (10.0 g, 0.023 mol), the Intermediate C-10 (6.7 g, 0.025 mol), Pd(PPh3)4 (1.4 g, 0.0012 mol) and K₂CO₃ (6.3 g, 0.045 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH9-D3 (9.1 g, 77.6%).

### Synthesis Example 44: Synthesis of Compound GEH9-D4

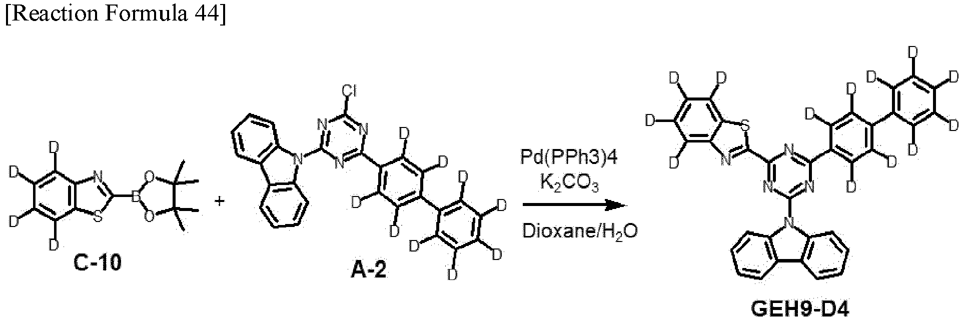

The Intermediate A-2 (10.0 g, 0.022 mol), the Intermediate C-10 (6.2 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH9-D4 (9.0 g, 76.6%).

### Synthesis Example 45: Synthesis of Compound GEH9-D5

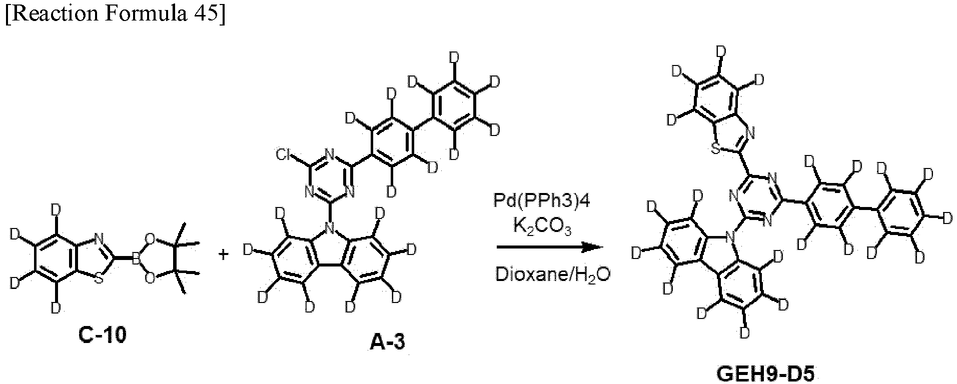

The Intermediate A-3 (10.0 g, 0.022 mol), the Intermediate C-10 (6.3 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH9-D5 (10.0 g, 81.4%).

### Synthesis Example 46: Synthesis of Compound GEH10-D1

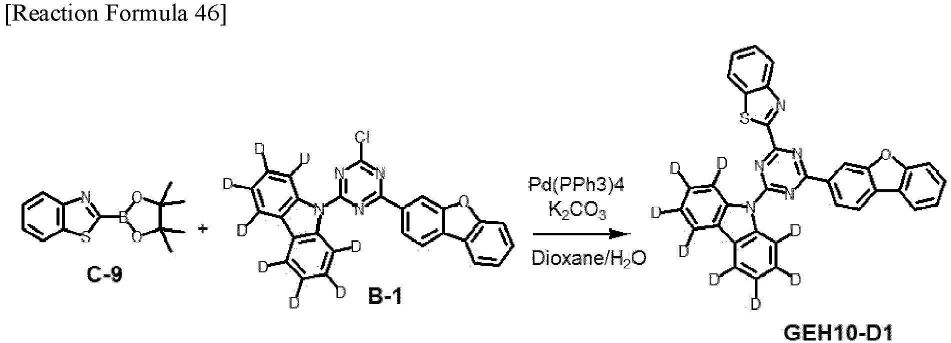

The Intermediate B-1 (10.0 g, 0.022 mol), the Intermediate C-9 (6.3 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH10-D1 (9.9 g, 81.2%).

### Synthesis Example 47: Synthesis of Compound GEH10-D2

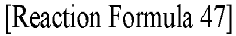

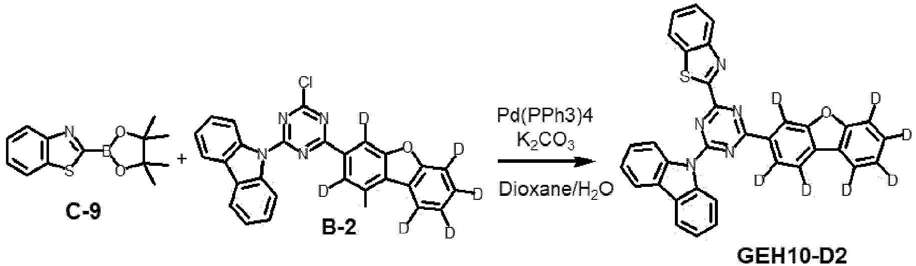

The Intermediate B-2 (10.0 g, 0.022 mol), the Intermediate C-9 (6.3 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH10-D2 (9.0 g, 73.9%).

### Synthesis Example 48: Synthesis of Compound GEH10-D3

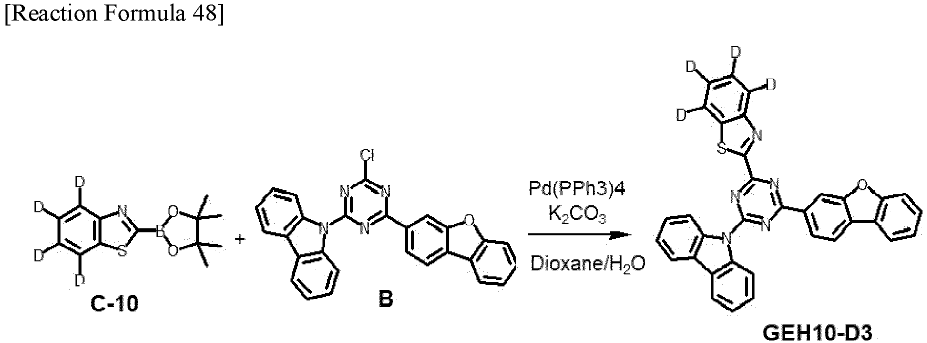

The Intermediate B (10.0 g, 0.022 mol), the Intermediate C-10 (6.4 g, 0.024 mol), Pd(PPh3)4 (1.2 g, 0.0011 mol) and K₂CO₃ (6.0 g, 0.043 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH10-D3 (9.2 g, 75.5%).

### Synthesis Example 49: Synthesis of Compound GEH10-D4

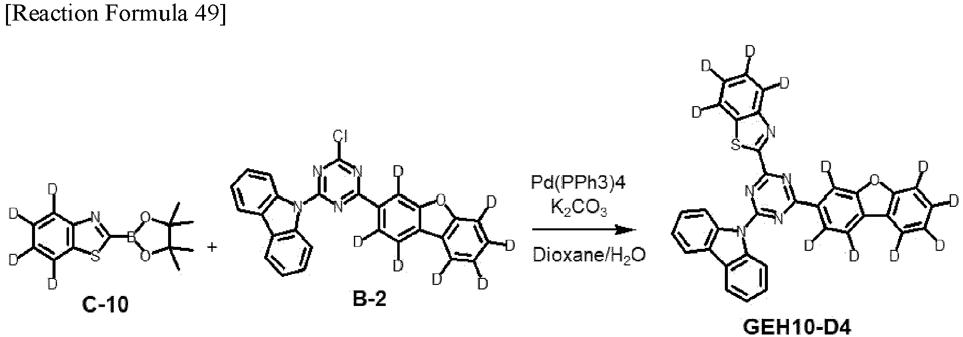

The Intermediate B-2 (10.0 g, 0.022 mol), the Intermediate C-10 (6.4 g, 0.024 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH10-D4 (9.3 g, 75.8%).

### Synthesis Example 50: Synthesis of Compound GEH10-D5

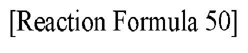

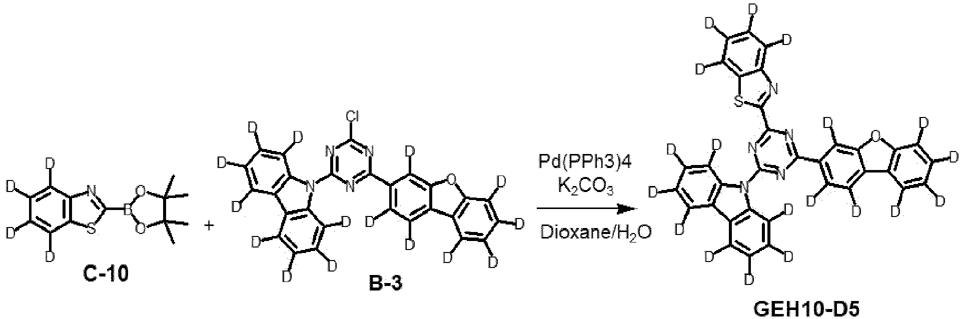

The Intermediate B-3 (9.4 g, 0.021 mol), the Intermediate C-10 (6.3 g, 0.023 mol), Pd(PPh3)4 (1.2 g, 0.0011 mol) and K₂CO₃ (5.4 g, 0.04 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH10-D5 (9.0 g, 73.6%).

### Synthesis Example 51: Synthesis of Compound GEH1

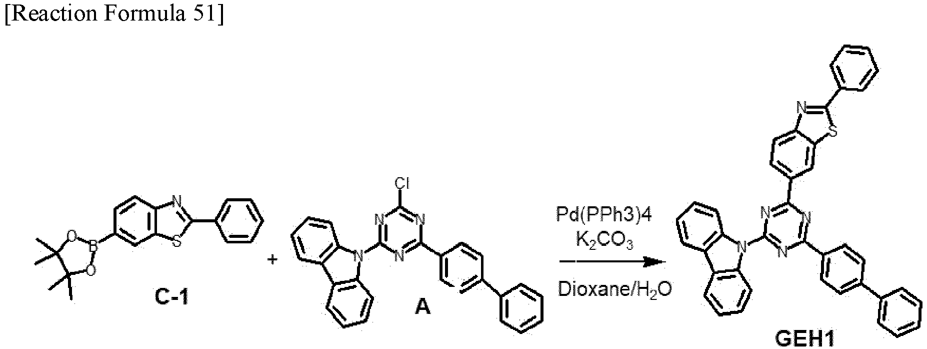

The Intermediate A (10.0 g, 0.022 mol), the Intermediate C-1 (8.56 g, 0.026 mol), Pd(PPh3)4 (1.6 g, 0.0015 mol) and K₂CO₃ (5.17 g, 0.037 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH1 (10.5 g, 81.7%).

### Synthesis Example 52: Synthesis of Compound GEH2

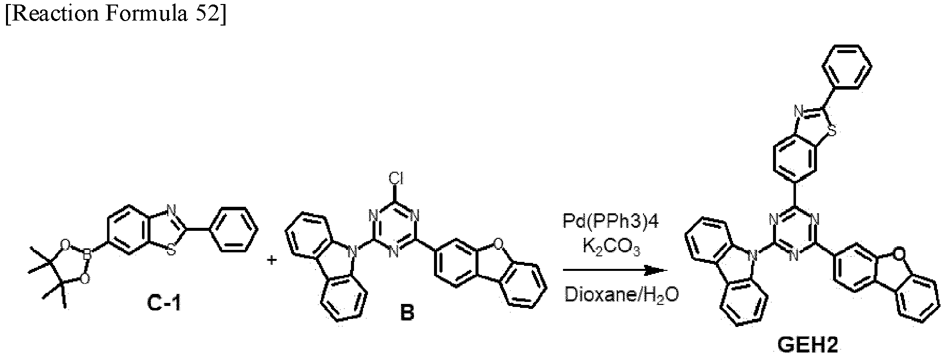

The Intermediate B (9.4 g, 0.021 mol), the Intermediate C-1 (7.68 g, 0.023 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH2 (9.87 g, 77.3%).

### Synthesis Example 53: Synthesis of Compound GEH3

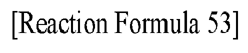

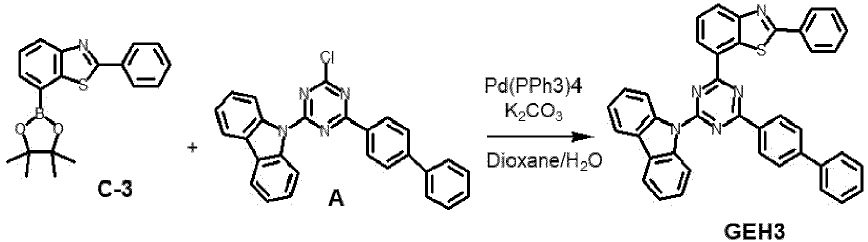

The Intermediate A (8.1 g, 0.019 mol), the Intermediate C-3 (6.94 g, 0.021 mol), Pd(PPh3)4 (1.1 g, 0.0010 mol) and K₂CO₃ (5.17 g, 0.037 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH3 (9.2 g, 80.6%).

### Synthesis Example 54: Synthesis of Compound GEH4

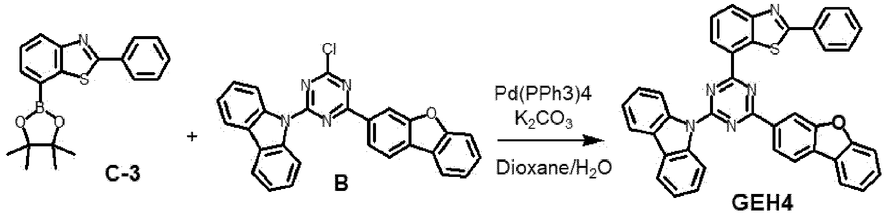

The Intermediate B (9.4 g, 0.021 mol), the Intermediate C-3 (7.68 g, 0.023 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH4 (9.87 g, 77.3%).

### Synthesis Example 55: Synthesis of Compound GEH5

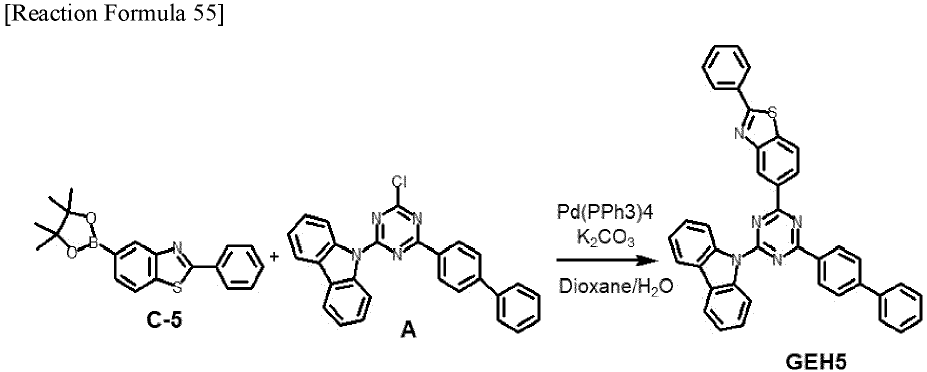

The Intermediate A (8.1 g, 0.019 mol), the Intermediate C-5 (6.94 g, 0.021 mol), Pd(PPh3)4 (1.1 g, 0.0010 mol) and K₂CO₃ (5.17 g, 0.037 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH5 (9.2 g, 80.6%).

### Synthesis Example 56: Synthesis of Compound GEH6

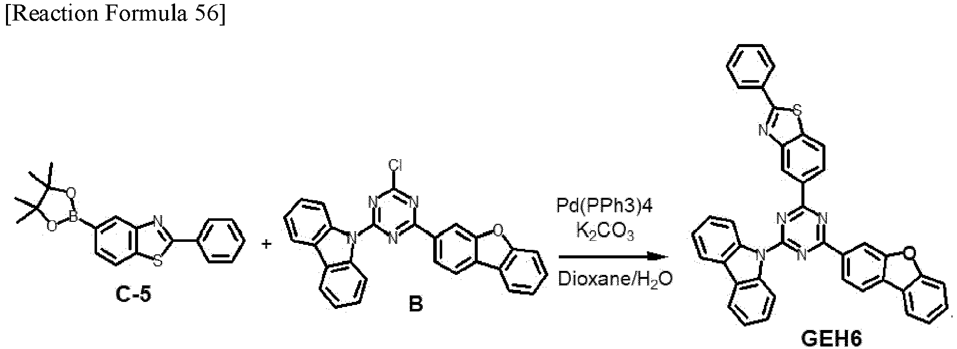

The Intermediate B (9.4 g, 0.021 mol), the Intermediate C-5 (7.68 g, 0.023 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH6 (9.87 g, 77.3%).

### Synthesis Example 57: Synthesis of Compound GEH7

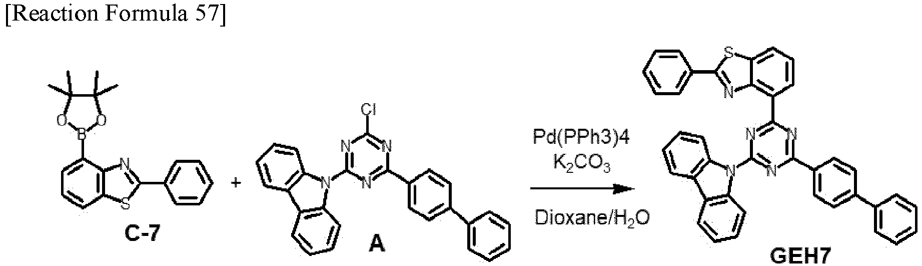

The Intermediate A (8.1 g, 0.019 mol), the Intermediate C-7 (6.94 g, 0.021 mol), Pd(PPh3)4 (1.1 g, 0.0010 mol) and K₂CO₃ (5.17 g, 0.037 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH7 (9.2 g, 80.6%).

### Synthesis Example 58: Synthesis of Compound GEH8

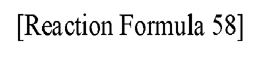

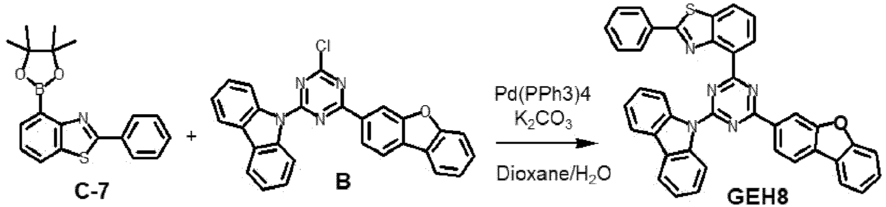

The Intermediate B (9.4 g, 0.021 mol), the Intermediate C-7 (7.68 g, 0.023 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH8 (9.87 g, 77.3%).

### Synthesis Example 59: Synthesis of Compound GEH9

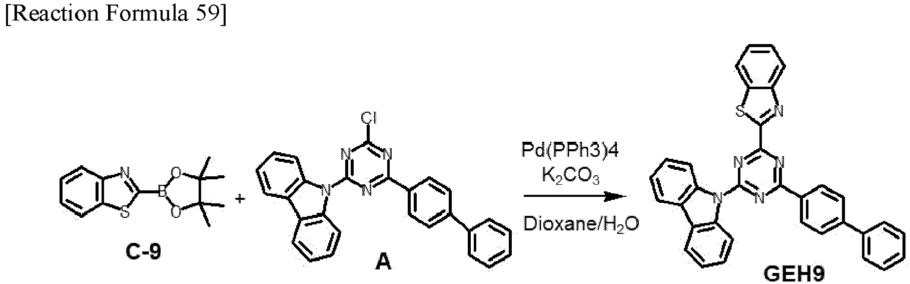

The Intermediate A (8.1 g, 0.019 mol), the Intermediate C-9 (5.48 g, 0.021 mol), Pd(PPh3)4 (1.1 g, 0.0010 mol) and K₂CO₃ (5.17 g, 0.037 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH9 (6.1 g, 59.9%).

### Synthesis Example 60: Synthesis of Compound GEH10

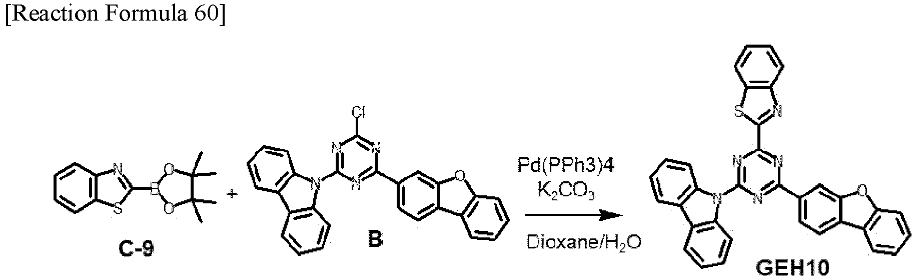

The Intermediate B (9.4 g, 0.021 mol), the Intermediate C-9 (6.00 g, 0.023 mol), Pd(PPh3)4 (1.2 g, 0.0011 mol) and K₂CO₃ (5.7 g, 0.040 mol) in a mixed solvent (1,4-Dioxane 100 ml, water 20 ml) were added into a round bottom flask with one neck, then the reaction was proceed at 110°C for 4 hours with reflux conditon. After the reaction solution was cooled to a room temperature sufficiently, the precipiated solid was filtered and washed with water and methanol sufficiently. The filtered product was heated with DCB to be dissovled completely, and subjected to silica gel filter using CF. The obtined solution was concentrated, subjected to acetone slurry and filtered to give Compound GEH10 (6.95 g, 60.7%).

### Example 1 (Ex.1): Fabrication of OLED

An organic light emitting diode where Compound GEH1 of Synthesis Example 1 was applied to an emitting material layer was fabricated. A glass substrate onto which ITO (50 nm) was coated as a thin film was washed and ultrasonically cleaned by solvent such as isopropyl alcohol, acetone and dried at 100°C oven. The substrate was transferred to a vacuum chamber for depositing emissive layer. Subsequently, an emissive layer and a cathode were deposited by evaporation from a heating boat under about 5-7 × 10⁻⁷ Torr with setting a deposition rate 1 Å/s as the following order:

A hole injection layer (HIL, HAT-CN, 5 nm); a hole transport layer (HTL, NPB, 100 nm thickness); an emitting material layer (G-EML, host (P-type host or green hole host (GHH) BCZ: N-type host or green electron host (GEH) Compound GEH1 = 5:5 by weight, 85 wt%), Ir(ppy)₃ (green dopant (GD), 15 wt%), 30 nm); an electron transport layer (EBL, ETM1 below, 300 nm); an electron injection layer (EIL, LiF, 5 nm); and a cathode (Al, 100 nm).

The structures of materials of HAT-CN, NPB, BCZ, Ir(ppy3) and electron transporting materials are illustrated in the following:

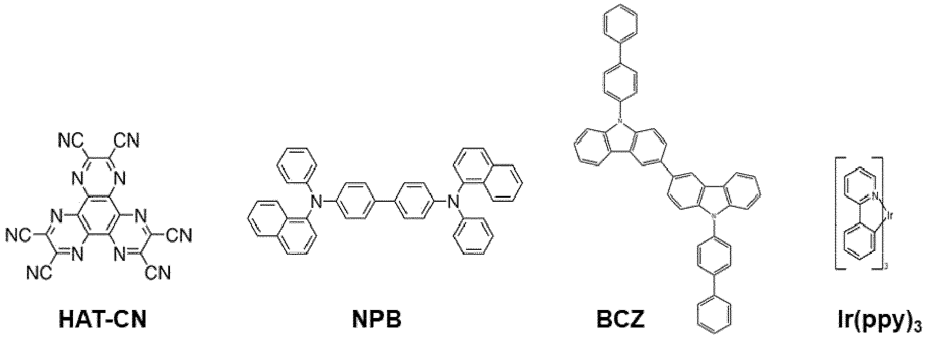

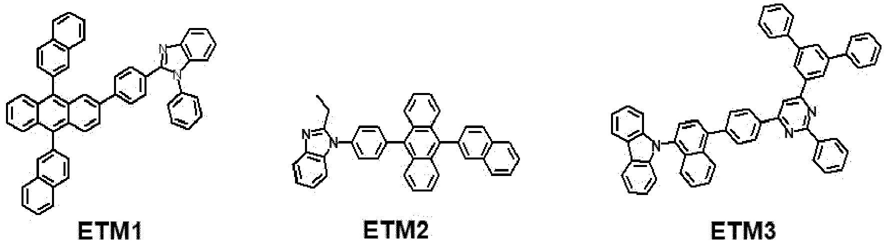

### Examples 2-10 (Ex. 2-10): Fabrication of OLEDs

An OLED was fabricated using the same procedure and the same materials as Example 1, except that each of Compound GEH2 (Ex. 2), Compound GEH3 (Ex. 3), Compound GEH4 (Ex. 4), Compound GEH5 (Ex. 5), Compound GEH6 (Ex. 6), Compound GEH7 (Ex. 7), Compound GEH8 (Ex. 8), Compound GEH9 (Ex. 9) and Compound GEH10 (Ex. 10) instead of Compound GEH1 was used as the N-type host in the EML, respectively.

### Example 11 (Ex. 11): Fabrication of OLED

An OLED was fabricated using the same procedure and the same materials as Example 1, except that ETM2 instead of ETM1 was used as the electron transporting material in the ETL.

### Examples 12-20 (Ex. 12-20): Fabrication of OLEDs

An OLED was fabricated using the same procedure and the same materials as Example 11, except that each of Compound GEH2 (Ex. 12), Compound GEH3 (Ex. 13), Compound GEH4 (Ex. 14), Compound GEH5 (Ex. 15), Compound GEH6 (Ex. 16), Compound GEH7 (Ex. 17), Compound GEH8 (Ex. 18), Compound GEH9 (Ex. 19) and Compound GEH10 (Ex. 20) instead of Compound GEH1 was used as the N-type host in the EML, respectively.

### Example 21 (Ex. 21): Fabrication of OLED

An OLED was fabricated using the same procedure and the same materials as Example 1, except that ETM3 instead of ETM1 was used as the electron transporting material in the ETL.

### Examples 22-30 (Ex. 22-30): Fabrication of OLEDs

An OLED was fabricated using the same procedure and the same materials as Example 21, except that each of Compound GEH2 (Ex. 22), Compound GEH3 (Ex. 23), Compound GEH4 (Ex. 24), Compound GEH5 (Ex. 25), Compound GEH6 (Ex. 26), Compound GEH7 (Ex. 27), Compound GEH8 (Ex. 28), Compound GEH9 (Ex. 29) and Compound GEH10 (Ex. 30) instead of Compound GEH1 was used as the N-type host in the EML, respectively.

### Example 31 (Ex. 31): Fabrication of OLED

An OLED was fabricated using the same procedure and the same materials as Example 1, except that ETM2 and ETM3 (5:5 by weight) instead of ETM1 were used as the electron transporting material in the ETL.

### Examples 32-40 (Ex. 32-40): Fabrication of OLEDs

An OLED was fabricated using the same procedure and the same materials as Example 31, except that each of Compound GEH2 (Ex. 32), Compound GEH3 (Ex. 33), Compound GEH4 (Ex. 34), Compound GEH5 (Ex. 35), Compound GEH6 (Ex. 36), Compound GEH7 (Ex. 37), Compound GEH8 (Ex. 38), Compound GEH9 (Ex. 39) and Compound GEH10 (Ex. 40) instead of Compound GEH1 was used as the N-type host in the EML, respectively.

### Comparative Examples 1-11 (Ref. 1-11): Fabrication of OLEDs

An OLED was fabricated using the same procedure and the same materials as Example 1, except that each of TCPZ (2,4,6-tris(3-carbazol-9-yl)phenyl)triazine) (Ref. 1), a reference compound GEHA (Ref. 2), a reference compound GEHB (Ref. 3), a reference compound GEHC (Ref. 4), a reference compound GEHD (Ref. 5), a reference compound GEHE (Ref. 6), a reference compound GEHF (Ref. 7), a reference compound GEHG (Ref. 8), a reference compound GEHH (Ref. 9), a reference compound GEHI (Ref. 10) and a reference compound GEHJ (Ref. 11), each of which is illustrated in the following, instead of Compound GEH1 was used as the N-type host in the EML, respectively.

### [Reference Compounds]

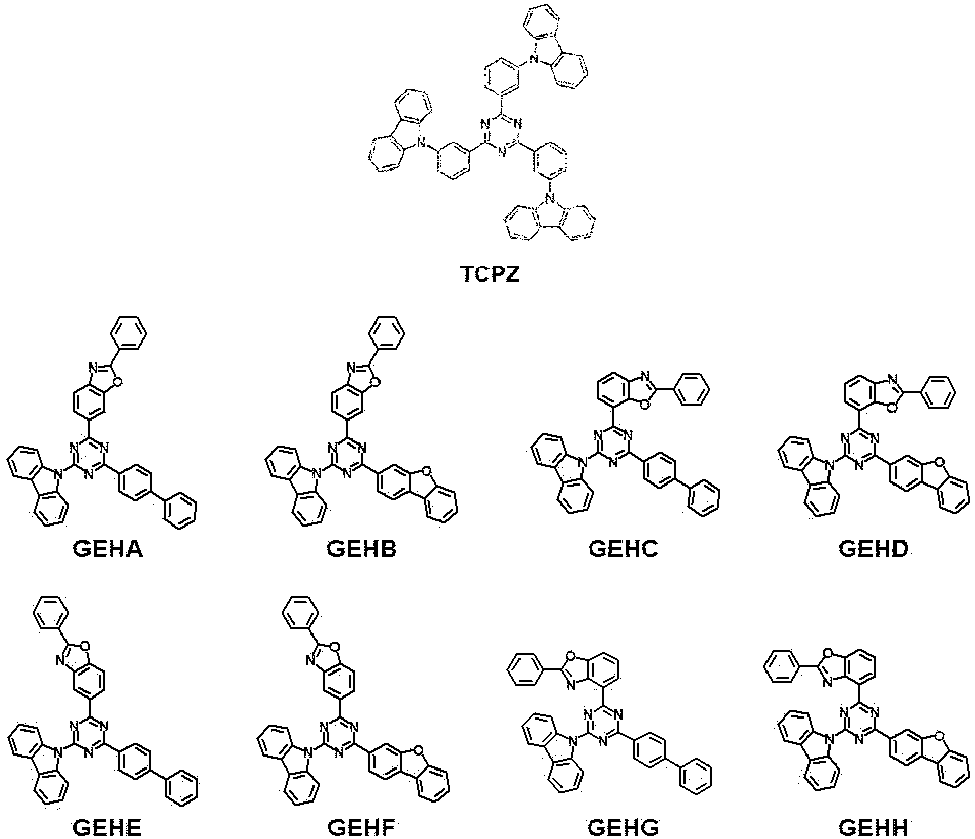

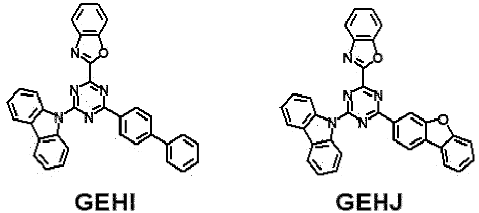

### Experimental Example 1: Measurement of energy level and dipole moment of Compounds

HOMO energy level, LUMO energy level, triplet energy level (T₁) and dipole moment for the Compounds GEH1, GEH2, GEH3, GEHA, GEHB and GEHC as the N-type host in the EML and ETM1, ETM2 and ETM3 as the electron transporting material in the ETL were measured. The measurement results are indicated in the following Table 1.

**Table 1: Energy levels and dipole moment**

| Material | HOMO (eV)^{∗} | LUMO (eV)^{∗∗} | T₁ (eV)^{∗∗∗} | Dipole moment^{∗∗∗} |
|---|---|---|---|---|
| GEHA | -5.80 | -2.09 | 2.73 | 0.09 |
| GEHB | -5.77 | -2.12 | 2.67 | 0.18 |
| GEHC | -5.80 | -2.14 | 2.67 | 0.05 |
| GEH1 | -5.78 | -2.16 | 2.67 | 0.25 |
| GEH2 | -5.78 | -2.17 | 2.57 | 2.36 |
| GEH3 | -5.82 | -2.11 | 2.67 | 2.06 |
| ETM1 | -5.08 | -1.78 | 1.68 | 3.54 |
| ETM2 | -5.28 | -1.81 | 1.73 | 4.36 |
| ETM3 | -5.38 | -1.87 | 2.54 | 2.01 |
| *: Measured with CV (Cyclic voltammetry) | | | | |
| ^{∗∗}: LUMO = HOMO^{∗}+E_{g} (calculated by UV-Vis absorption Edge λ) | | | | |
| ^{∗∗∗}: Calculated by Gaussian B3LYP 6-31G* (Basis set) | | | | |

As indicated in Table 1, compared to the reference compounds GEHA, GEHB and GEHC with a benzoxazole moiety, the dipole moments of the Compounds GEH1, GEH2 and GEH3 having a benzothiazole moiety varied greatly depending on the arrangement of direction of sulfur atoms in the molecule. It was confirmed that the dipole moment of the organic compound having the benzothiazole moiety was greater than that of the reference compounds having the benzothiazole moiety.

### Experimental Example 2: Measurement of Luminous Properties of OLEDs

Each of the OLEDs, having 9 mm² of emission area, fabricated in Examples 1 to 40 and Comparative Examples 1 to 11 was connected to an external power source and then luminous properties for all the OLEDs were evaluated using a constant current source (KEITHLEY) and a photometer PR650 at room temperature. In particular, driving voltage (V, relative value), luminance (L, relative value) and lifetime (LT₉₅, relative value) at which the luminance was reduced to 95% from initial luminance was measured at a current density 10 mA/cm². The measurement results are indicated in the following Tables 2 and 3.

**Table 2: Luminous Properties of OLED**

| Sample | G-EML | | | ETL | V | L | LT₉₅ |
|---|---|---|---|---|---|---|---|
| | GHH | GEH | GD | | | | |
| Ref. 1 | BCZ | TCPZ | Ir(ppy)₃ | ETM1 | 0.00 | 100% | 100% |
| Ref. 2 | BCZ | GEHA | Ir(ppy)₃ | ETM1 | -0.05 | 104% | 106% |
| Ref. 3 | BCZ | GEHB | Ir(ppy)₃ | ETM1 | -0.11 | 106% | 106% |
| Ref. 4 | BCZ | GEHC | Ir(ppy)₃ | ETM1 | -0.03 | 103% | 106% |
| Ref. 5 | BCZ | GEHD | Ir(ppy)₃ | ETM1 | -0.03 | 103% | 106% |
| Ref. 6 | BCZ | GEHE | Ir(ppy)₃ | ETM1 | -0.08 | 105% | 106% |
| Ref. 7 | BCZ | GEHF | Ir(ppy)₃ | ETM1 | -0.10 | 106% | 106% |
| Ref. 8 | BCZ | GEHG | Ir(ppy)₃ | ETM1 | -0.06 | 104% | 106% |
| Ref. 9 | BCZ | GEHH | Ir(ppy)₃ | ETM1 | -0.14 | 107% | 105% |
| Ref. 10 | BCZ | GEHI | Ir(ppy)₃ | ETM1 | -0.06 | 104% | 106% |
| Ref. 11 | BCZ | GEHJ | Ir(ppy)₃ | ETM1 | -0.03 | 103% | 106% |
| Ex. 1 | BCZ | GEH1 | Ir(ppy)₃ | ETM1 | -0.14 | 107% | 106% |
| Ex. 2 | BCZ | GEH2 | Ir(ppy)₃ | ETM1 | -1.32 | 158% | 98% |
| Ex. 3 | BCZ | GEH3 | Ir(ppy)₃ | ETM1 | -1.22 | 154% | 99% |
| Ex. 4 | BCZ | GEH4 | Ir(ppy)₃ | ETM1 | -1.33 | 158% | 98% |
| Ex. 5 | BCZ | GEH5 | Ir(ppy)₃ | ETM1 | -1.17 | 152% | 99% |
| Ex. 6 | BCZ | GEH6 | Ir(ppy)₃ | ETM1 | -0.84 | 137% | 101% |
| Ex. 7 | BCZ | GEH7 | Ir(ppy)3 | ETM1 | -1.03 | 146% | 99% |
| Ex. 8 | BCZ | GEH8 | Ir(ppy)3 | ETM1 | -0.40 | 118% | 104% |
| Ex. 9 | BCZ | GEH9 | Ir(ppy)3 | ETM1 | -0.99 | 144% | 100% |
| Ex. 10 | BCZ | GEH10 | Ir(ppy)3 | ETM1 | -0.90 | 140% | 101% |

**Table 3: Luminous Properties of OLED**

| Sample | G-EML | | | ETL | Voltage | Luminance | LT₉₅ |
|---|---|---|---|---|---|---|---|
| | GHH | GEH | GD | | | | |
| Ex. 11 | BCZ | GEH1 | Ir(ppy)₃ | ETM2 | -0.18 | 109% | 106% |
| Ex. 12 | BCZ | GEH2 | Ir(ppy)₃ | ETM2 | -1.66 | 173% | 97% |
| Ex. 13 | BCZ | GEH3 | Ir(ppy)₃ | ETM2 | -1.54 | 168% | 98% |
| Ex. 14 | BCZ | GEH4 | Ir(ppy)₃ | ETM2 | -1.67 | 173% | 97% |
| Ex. 15 | BCZ | GEH5 | Ir(ppy)₃ | ETM2 | -1.48 | 165% | 98% |
| Ex. 16 | BCZ | GEH6 | Ir(ppy)₃ | ETM2 | -1.06 | 147% | 101% |
| Ex. 17 | BCZ | GEH7 | Ir(ppy)₃ | ETM2 | -1.31 | 157% | 99% |
| Ex. 18 | BCZ | GEH8 | Ir(ppy)₃ | ETM2 | -0.50 | 123% | 104% |
| Ex. 19 | BCZ | GEH9 | Ir(ppy)₃ | ETM2 | -1.25 | 155% | 100% |
| Ex. 20 | BCZ | GEH10 | Ir(ppy)₃ | ETM2 | -1.14 | 150% | 100% |
| Ex. 21 | BCZ | GEH1 | Ir(ppy)₃ | ETM3 | -0.09 | 108% | 133% |
| Ex. 22 | BCZ | GEH2 | Ir(ppy)₃ | ETM3 | -0.82 | 139% | 128% |
| Ex. 23 | BCZ | GEH3 | Ir(ppy)₃ | ETM3 | -0.76 | 137% | 129% |
| Ex. 24 | BCZ | GEH4 | Ir(ppy)₃ | ETM3 | -0.82 | 140% | 128% |
| Ex. 25 | BCZ | GEH5 | Ir(ppy)₃ | ETM3 | -0.73 | 135% | 129% |
| Ex. 26 | BCZ | GEH6 | Ir(ppy)₃ | ETM3 | -0.52 | 127% | 130% |
| Ex. 27 | BCZ | GEH7 | Ir(ppy)₃ | ETM3 | -0.64 | 132% | 129% |
| Ex. 28 | BCZ | GEH8 | Ir(ppy)₃ | ETM3 | -0.25 | 115% | 132% |
| Ex. 29 | BCZ | GEH9 | Ir(ppy)₃ | ETM3 | -0.62 | 131% | 130% |
| Ex. 30 | BCZ | GEH10 | Ir(ppy)₃ | ETM3 | -0.56 | 128% | 130% |
| Ex. 31 | BCZ | GEH1 | Ir(ppy)₃ | ETM2+ETM3 | -0.23 | 118% | 126% |
| Ex. 32 | BCZ | GEH2 | Ir(ppy)₃ | ETM2+ETM3 | -1.35 | 166% | 118% |
| Ex. 33 | BCZ | GEH3 | Ir(ppy)₃ | ETM2+ETM3 | -1.26 | 162% | 119% |
| Ex. 34 | BCZ | GEH4 | Ir(ppy)₃ | ETM2+ETM3 | -1.36 | 167% | 119% |
| Ex. 35 | BCZ | GEH5 | Ir(ppy)₃ | ETM2+ETM3 | -1.21 | 160% | 119% |
| Ex. 36 | BCZ | GEH6 | Ir(ppy)₃ | ETM2+ETM3 | -0.90 | 147% | 121% |
| Ex. 37 | BCZ | GEH7 | Ir(ppy)₃ | ETM2+ETM3 | -1.08 | 154% | 120% |
| Ex. 38 | BCZ | GEH8 | Ir(ppy)₃ | ETM2+ETM3 | -0.48 | 129% | 124% |
| Ex. 39 | BCZ | GEH9 | Ir(ppy)₃ | ETM2+ETM3 | -1.05 | 153% | 120% |
| Ex. 40 | BCZ | GEH10 | Ir(ppy)₃ | ETM2+ETM3 | -0.96 | 149% | 121% |

As indicated in Table, 1, compared to the OLED fabricated in Ref. 1 where TCPZ was used as the N-type host in the EML, in the OLED fabricated in Ex. 1 to 10, the driving voltage was lowered by 1.32 V and the luminance and luminous lifetime was improved by 58% and 6%, respectively. In addition, compared to the OLEDs fabricated in Ref. 2 to Ref. 11 where compounds having a benzoxazole moiety as the N-type host in the EML, in the OLED fabricated in Ex. 1 to 10 where compounds having a benzothiazole moiety as the N-type host in the EML, the driving voltage was further lowered and luminance was greatly improved.

As confirmed in the Experimental Example 1, compared to the reference compound having the benzoxazole moiety, the dipole moment of the compound having the benzothiazole moiety is great. Accordingly, electron injection is improved by energy band bending in the interface between the emitting material layer including the compound having the benzothiazole moiety and the electron transport material disposed adjacently to the emitting material layer. Therefore, in the OLED fabricated in Ex. 1 to Ex. 10, the driving voltage was further lowered and luminous property such as luminance was further improved.

In addition, in the OLED where the ETM3, which has LUMO energy level relatively closer to LUMO energy level of the N-type host and has larger dipole moment, was used as the electron transporting material, the driving voltage was further lowered and luminance was further greatly improved. Also, In the OLED where the ETM3, which LUMO energy level relatively further closer to LUMO energy level of the N-type host and has larger triplet energy level, was uses as the electron transporting material, the lifetime was further greatly improved. More particularly, compared to the OLED fabricated in Ref. 1, in the OLEDs fabricated in Ex. 11 to Ex. 40, the driving voltage was lowered by 1.66 V, and luminance and luminous lifetime was improved by 73% and 33%, respectively.

### Examples 41-45 (Ex. 41-45): Fabrication of OLEDs

An OLED was fabricated using the same procedure and the same materials as Example 11, except that each of Compound GEH1-D1 (Ex. 41), Compound GEH1-D2 (Ex. 42), Compound GEH1-D3 (Ex. 43), Compound GEH1-D4 (Ex. 44) and Compound GEH1-D5 (Ex. 45) instead of Compound GEH1 was used as the N-type host in the EML, respectively.

### Examples 46-50 (Ex. 46-50): Fabrication of OLEDs

An OLED was fabricated using the same procedure and the same materials as Example 21, except that each of Compound GEH1-D1 (Ex. 46), Compound GEH1-D2 (Ex. 47), Compound GEH1-D3 (Ex. 48), Compound GEH1-D4 (Ex. 49) and Compound GEH1-D5 (Ex. 50) instead of Compound GEH1 was used as the N-type host in the EML, respectively.

### Examples 51-55 (Ex. 51-55): Fabrication of OLEDs

An OLED was fabricated using the same procedure and the same materials as Example 31, except that each of Compound GEH1-D1 (Ex. 51), Compound GEH1-D2 (Ex. 52), Compound GEH1-D3 (Ex. 53), Compound GEH1-D4 (Ex. 54) and Compound GEH1-D5 (Ex. 55) instead of Compound GEH1 was used as the N-type host in the EML, respectively.

### Comparative Examples 12-16 (Ref. 12-16): Fabrication of OLEDs

An OLED was fabricated using the same procedure and the same materials as Example 1, except that each of a reference compound GEHA-D1 (Ref. 12), a reference compound GEHA-D2 (Ref. 13), a reference compound GEHA-D3 (Ref. 14), a reference compound GEHA-D4 (Ref. 15) and a reference compound GEHA-D5 (Ref. 16), each of which is illustrated in the following, instead of Compound GEH1 was used as the N-type host in the EML, respectively.

### [Reference Compounds]

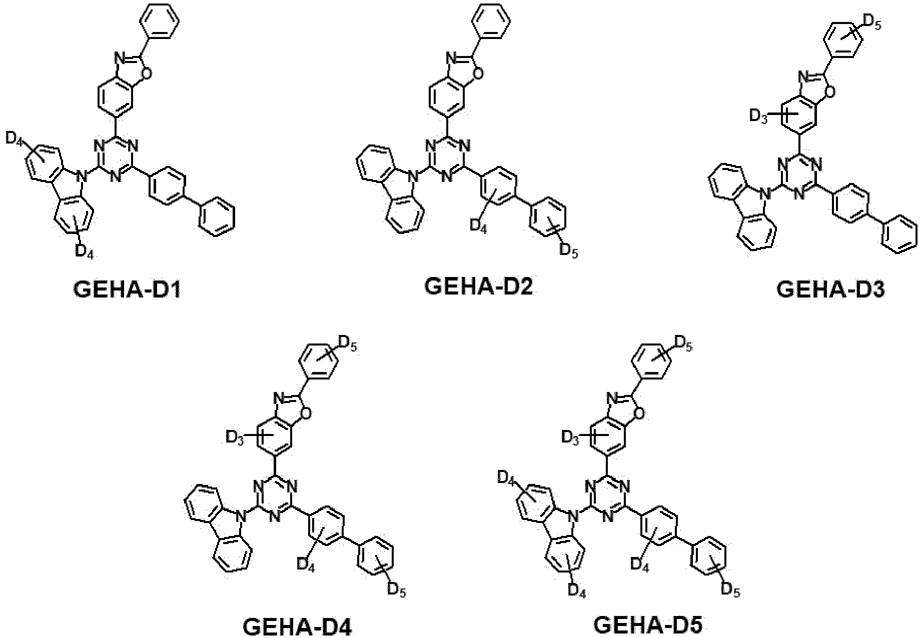

### Experimental Example 3: Measurement of Luminous Properties of OLEDs

Luminous properties for each of the OLEDs fabricated in Examples 41 to 55 and Comparative Examples 12 to 16 were measured as Experimental Example 2. The measurement results are indicated in the following Table 4.

**Table 4: Luminous Properties of OLED**

| Sample | G-EML | | | ETL | Deuteration Rate (DR) | V | L | LT₉₅ |
|---|---|---|---|---|---|---|---|---|
| | GHH | GEH | GD | | | | | |
| Ref. 1 | BCZ | TCPZ | Ir(ppy)₃ | ETM1 | 0% | 0.00 | 100% | 100% |
| Ex. 1 | BCZ | GEH1 | Ir(ppy)₃ | ETM1 | 0% | -0.14 | 107% | 106% |
| Ref. 2 | BCZ | GEHA | Ir(ppy)₃ | ETM1 | 0% | -0.05 | 104% | 106% |
| Ref. 12 | BCZ | GEHA_D1 | Ir(ppy)₃ | ETM1 | 32% | -0.05 | 104% | 133% |
| Ref. 13 | BCZ | GEHA_D2 | Ir(ppy)₃ | ETM1 | 36% | -0.05 | 104% | 109% |
| Ref. 14 | BCZ | GEHA_D3 | Ir(ppy)₃ | ETM1 | 32% | -0.05 | 104% | 109% |
| Ref. 15 | BCZ | GEHA_D4 | Ir(ppy)₃ | ETM1 | 68% | 0.05 | 102% | 114% |
| Ref. 16 | BCZ | GEHA_D5 | Ir(ppy)₃ | ETM1 | 100% | 0.05 | 102% | 140% |
| Ex. 11 | BCZ | GEH1 | Ir(ppy)₃ | ETM2 | 0% | -0.18 | 109% | 106% |
| Ex. 41 | BCZ | GEH1_D1 | Ir(ppy)₃ | ETM2 | 32% | -0.18 | 109% | 133% |
| Ex. 42 | BCZ | GEH1_D2 | Ir(ppy)₃ | ETM2 | 36% | -0.18 | 109% | 110% |
| Ex. 43 | BCZ | GEH1_D3 | Ir(ppy)₃ | ETM2 | 32% | -0.18 | 109% | 110% |
| Ex. 44 | BCZ | GEH1_D4 | Ir(ppy)₃ | ETM2 | 68% | -0.08 | 107% | 114% |
| Ex. 45 | BCZ | GEH1_D5 | Ir(ppy)₃ | ETM2 | 100% | -0.08 | 107% | 140% |
| Ex. 21 | BCZ | GEH1 | Ir(ppy)₃ | ETM3 | 0% | -0.09 | 108% | 133% |
| Ex. 46 | BCZ | GEH1_D1 | Ir(ppy)₃ | ETM3 | 32% | -0.09 | 108% | 166% |
| Ex. 47 | BCZ | GEH1_D2 | Ir(ppy)₃ | ETM3 | 36% | -0.09 | 108% | 137% |
| Ex. 48 | BCZ | GEH1_D3 | Ir(ppy)₃ | ETM3 | 32% | -0.09 | 108% | 137% |
| Ex. 49 | BCZ | GEH1_D4 | Ir(ppy)₃ | ETM3 | 68% | 0.01 | 106% | 142% |
| Ex. 50 | BCZ | GEH1_D5 | Ir(ppy)₃ | ETM3 | 100% | 0.01 | 106% | 175% |
| Ex. 31 | BCZ | GEH1 | Ir(ppy)₃ | ETM2+ETM3 | 0% | -0.23 | 118% | 126% |
| Ex. 51 | BCZ | GEH1_D1 | Ir(ppy)₃ | ETM2+ETM3 | 32% | -0.23 | 118% | 157% |
| Ex. 52 | BCZ | GEH1_D2 | Ir(ppy)₃ | ETM2+ETM3 | 36% | -0.23 | 118% | 129% |
| Ex. 53 | BCZ | GEH1_D3 | Ir(ppy)₃ | ETM2+ETM3 | 32% | -0.23 | 118% | 129% |
| Ex. 54 | BCZ | GEH1_D4 | Ir(ppy)₃ | ETM2+ETM3 | 68% | -0.13 | 116% | 134% |
| Ex. 55 | BCZ | GEH1_D5 | Ir(ppy)₃ | ETM2+ETM3 | 100% | -0.13 | 116% | 166% |

### Examples 56-60 (Ex. 56-60): Fabrication of OLEDs

An OLED was fabricated using the same procedure and the same materials as Example 12, except that each of Compound GEH2-D1 (Ex. 56), Compound GEH2-D2 (Ex. 57), Compound GEH2-D3 (Ex. 58), Compound GEH2-D4 (Ex. 59) and Compound GEH2-D5 (Ex. 60) instead of Compound GEH2 was used as the N-type host in the EML, respectively.

### Examples 61-65 (Ex. 61-65): Fabrication of OLEDs

An OLED was fabricated using the same procedure and the same materials as Example 22, except that each of Compound GEH2-D1 (Ex. 61), Compound GEH2-D2 (Ex. 62), Compound GEH2-D3 (Ex. 63), Compound GEH2-D4 (Ex. 64) and Compound GEH2-D5 (Ex. 65) instead of Compound GEH2 was used as the N-type host in the EML, respectively.

### Examples 66-70 (Ex. 66-70): Fabrication of OLEDs

An OLED was fabricated using the same procedure and the same materials as Example 32, except that each of Compound GEH2-D1 (Ex. 66), Compound GEH2-D2 (Ex. 67), Compound GEH2-D3 (Ex. 68), Compound GEH2-D4 (Ex. 69) and Compound GEH2-D5 (Ex. 70) instead of Compound GEH2 was used as the N-type host in the EML, respectively.

### Comparative Examples 17-21 (Ref. 17-21): Fabrication of OLEDs

An OLED was fabricated using the same procedure and the same materials as Example 2, except that each of a reference compound GEHB-D1 (Ref. 17), a reference compound GEHB-D2 (Ref. 18), a reference compound GEHB-D3 (Ref. 19), a reference compound GEHB-D4 (Ref. 20) and a reference compound GEHB-D5 (Ref. 21), each of which is illustrated in the following, instead of Compound GEH1 was used as the N-type host in the EML, respectively.

### [Reference Compounds]

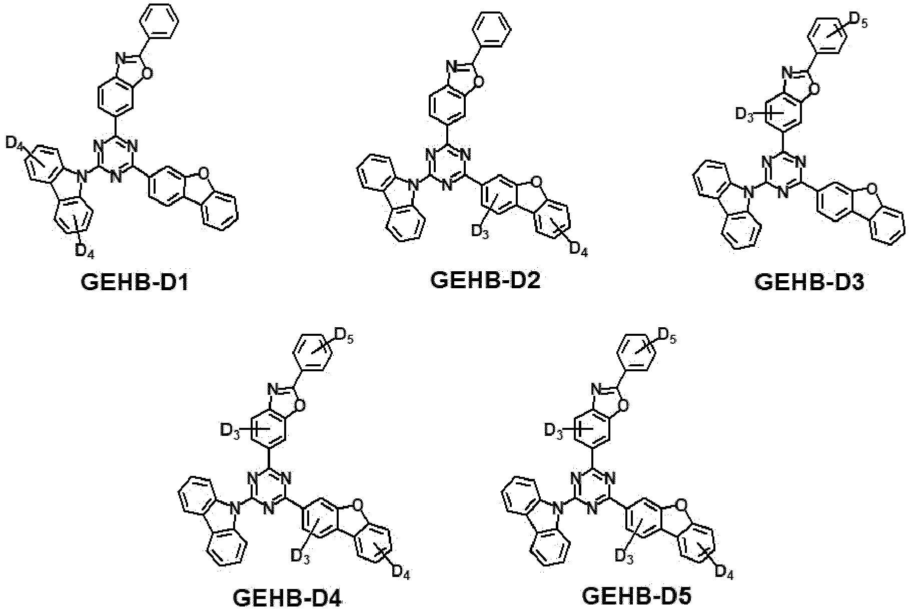

### Experimental Example 4: Measurement of Luminous Properties of OLEDs

Luminous properties for each of the OLEDs fabricated in Examples 56 to 70 and Comparative Examples 17 to 21 were measured as Experimental Example 2. The measurement results are indicated in the following Table 5.

**Table 5: Luminous Properties of OLED**

| Sample | G-EML | | | ETL | DR | V | L | LT₉₅ |
|---|---|---|---|---|---|---|---|---|
| | GHH | GEH | GD | | | | | |
| Ref. 1 | BCZ | TCPZ | Ir(ppy)₃ | ETM1 | 0% | 0.00 | 100% | 100% |
| Ex. 2 | BCZ | GEH2 | Ir(ppy)₃ | ETM1 | 0% | -1.32 | 158% | 98% |
| Ref. 3 | BCZ | GEHB | Ir(ppy)₃ | ETM1 | 0% | -0.11 | 106% | 106% |
| Ref. 17 | BCZ | GEHB_D1 | Ir(ppy)₃ | ETM1 | 35% | -0.11 | 106% | 132% |
| Ref. 18 | BCZ | GEHB_D2 | Ir(ppy)₃ | ETM1 | 30% | -0.11 | 106% | 109% |
| Ref. 19 | BCZ | GEHB_D3 | Ir(ppy)₃ | ETM1 | 35% | -0.11 | 106% | 109% |
| Ref. 20 | BCZ | GEHB_D4 | Ir(ppy)₃ | ETM1 | 65% | -0.01 | 104% | 113% |
| Ref. 21 | BCZ | GEHBD5 | Ir(ppy)₃ | ETM1 | 100% | -0.01 | 104% | 140% |
| Ex. 12 | BCZ | GEH2 | Ir(ppy)₃ | ETM2 | 0% | -1.66 | 173% | 97% |
| Ex. 56 | BCZ | GEH2_D1 | Ir(ppy)₃ | ETM2 | 35% | -1.66 | 173% | 121% |
| Ex. 57 | BCZ | GEH2_D2 | Ir(ppy)₃ | ETM2 | 39% | -1.66 | 173% | 100% |
| Ex. 58 | BCZ | GEH2_D3 | Ir(ppy)₃ | ETM2 | 35% | -1.66 | 173% | 100% |
| Ex. 59 | BCZ | GEH2_D4 | Ir(ppy)₃ | ETM2 | 65% | -1.56 | 169% | 104% |
| Ex. 60 | BCZ | GEH2_D5 | Ir(ppy)₃ | ETM2 | 100% | -1.56 | 169% | 128% |
| Ex. 22 | BCZ | GEH2 | Ir(ppy)₃ | ETM3 | 0% | -0.82 | 139% | 128% |
| Ex. 61 | BCZ | GEH2_D1 | Ir(ppy)₃ | ETM3 | 35% | -0.82 | 139% | 160% |
| Ex. 62 | BCZ | GEH2_D2 | Ir(ppy)₃ | ETM3 | 39% | -0.82 | 139% | 132% |
| Ex. 63 | BCZ | GEH2_D3 | Ir(ppy)₃ | ETM3 | 35% | -0.82 | 139% | 132% |
| Ex. 64 | BCZ | GEH2_D4 | Ir(ppy)₃ | ETM3 | 65% | -0.72 | 137% | 137% |
| Ex. 65 | BCZ | GEH2_D5 | Ir(ppy)₃ | ETM3 | 100% | -0.72 | 137% | 169% |
| Ex. 32 | BCZ | GEH2 | Ir(ppy)₃ | ETM2+ETM3 | 0% | -1.35 | 166% | 118% |
| Ex. 66 | BCZ | GEH2_D1 | Ir(ppy)₃ | ETM2+ETM3 | 35% | -1.35 | 166% | 148% |
| Ex. 67 | BCZ | GEH2_D2 | Ir(ppy)₃ | ETM2+ETM3 | 39% | -1.35 | 166% | 122% |
| Ex. 68 | BCZ | GEH2_D3 | Ir(ppy)₃ | ETM2+ETM3 | 35% | -1.35 | 166% | 122% |
| Ex. 69 | BCZ | GEH2_D4 | Ir(ppy)₃ | ETM2+ETM3 | 65% | -1.25 | 163% | 127% |
| Ex. 70 | BCZ | GEH2_D5 | Ir(ppy)₃ | ETM2+ETM3 | 100% | -1.25 | 163% | 156% |

### Examples 71-75 (Ex. 71-75): Fabrication of OLEDs

An OLED was fabricated using the same procedure and the same materials as Example 13, except that each of Compound GEH3-D1 (Ex. 71), Compound GEH3-D2 (Ex. 72), Compound GEH3-D3 (Ex. 73), Compound GEH3-D4 (Ex. 74) and Compound GEH3-D5 (Ex. 75) instead of Compound GEH3 was used as the N-type host in the EML, respectively.

### Examples 76-80 (Ex. 76-80): Fabrication of OLEDs

An OLED was fabricated using the same procedure and the same materials as Example 23, except that each of Compound GEH3-D1 (Ex. 76), Compound GEH3-D2 (Ex. 77), Compound GEH3-D3 (Ex. 78), Compound GEH3-D4 (Ex. 79) and Compound GEH3-D5 (Ex. 80) instead of Compound GEH3 was used as the N-type host in the EML, respectively.

### Examples 81-85 (Ex. 81-85): Fabrication of OLEDs

An OLED was fabricated using the same procedure and the same materials as Example 33, except that each of Compound GEH3-D1 (Ex. 81), Compound GEH3-D2 (Ex. 82), Compound GEH3-D3 (Ex. 83), Compound GEH3-D4 (Ex. 84) and Compound GEH3-D5 (Ex. 85) instead of Compound GEH3 was used as the N-type host in the EML, respectively.

### Comparative Examples 22-26 (Ref. 22-26): Fabrication of OLEDs

An OLED was fabricated using the same procedure and the same materials as Example 3, except that each of a reference compound GEHC-D1 (Ref. 22), a reference compound GEHC-D2 (Ref. 23), a reference compound GEHC-D3 (Ref. 24), a reference compound GEHC-D4 (Ref. 25) and a reference compound GEHC-D5 (Ref. 26), each of which is illustrated in the following, instead of Compound GEH1 was used as the N-type host in the EML, respectively.

### [Reference Compounds]

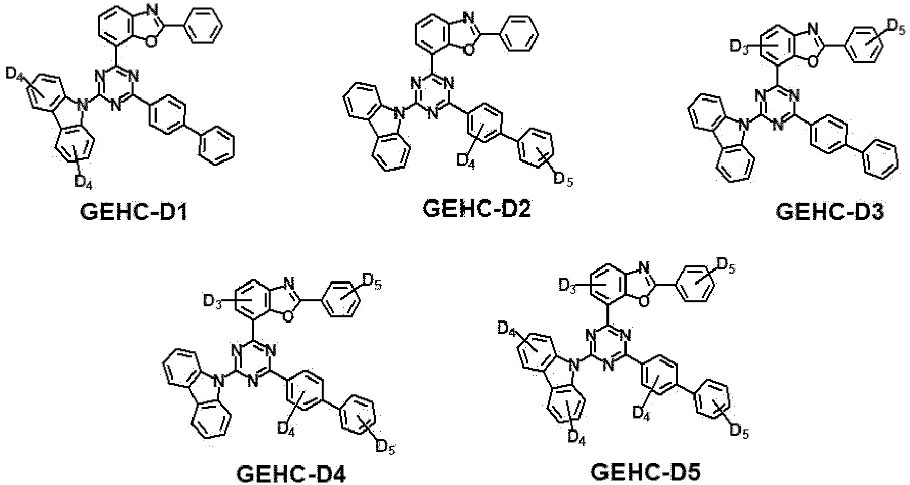

### Experimental Example 5: Measurement of Luminous Properties of OLEDs

Luminous properties for each of the OLEDs fabricated in Examples 71 to 85 and Comparative Examples 22 to 26 were measured as Experimental Example 2. The measurement results are indicated in the following Table 6.

**Table 6: Luminous Properties of OLED**

| Sample | G-EML | | | ETL | DR | V | L | LT₉₅ |
|---|---|---|---|---|---|---|---|---|
| | GHH | GEH | GD | | | | | |
| Ref. 1 | BCZ | TCPZ | Ir(ppy)3 | ETM1 | 0% | 0.00 | 100% | 100% |
| Ex. 3 | BCZ | GEH3 | Ir(ppy)₃ | ETM1 | 0% | -1.22 | 154% | 99% |
| Ref. 4 | BCZ | GEHC | Ir(ppy)₃ | ETM1 | 0% | -0.03 | 103% | 106% |
| Ref. 22 | BCZ | GEHC_D1 | Ir(ppy)₃ | ETM1 | 32% | -0.03 | 103% | 133% |
| Ref. 23 | BCZ | GEHC_D2 | Ir(ppy)₃ | ETM1 | 36% | -0.03 | 103% | 110% |
| Ref. 24 | BCZ | GEHC_D3 | Ir(ppy)₃ | ETM1 | 32% | -0.03 | 103% | 110% |
| Ref. 25 | BCZ | GEH C_D4 | Ir(ppy)₃ | ETM1 | 68% | 0.07 | 101% | 114% |
| Ref. 26 | BCZ | GEH C_D5 | Ir(ppy)₃ | ETM1 | 100% | 0.07 | 101% | 140% |
| Ex. 13 | BCZ | GEH3 | Ir(ppy)₃ | ETM2 | 0% | -1.54 | 168% | 98% |
| Ex. 71 | BCZ | GEH3_D1 | Ir(ppy)₃ | ETM2 | 32% | -1.54 | 168% | 122% |
| Ex. 72 | BCZ | GEH3_D2 | Ir(ppy)₃ | ETM2 | 36% | -1.54 | 168% | 101% |
| Ex. 73 | BCZ | GEH3_D3 | Ir(ppy)₃ | ETM2 | 32% | -1.54 | 168% | 101% |
| Ex. 74 | BCZ | GEH3_D4 | Ir(ppy)₃ | ETM2 | 68% | -1.44 | 164% | 105% |
| Ex. 75 | BCZ | GEH3_D5 | Ir(ppy)₃ | ETM2 | 100% | -1.44 | 164% | 129% |
| Ex. 23 | BCZ | GEH3 | Ir(ppy)₃ | ETM3 | 0% | -0.76 | 137% | 129% |
| Ex. 76 | BCZ | GEH3_D1 | Ir(ppy)₃ | ETM3 | 32% | -0.76 | 137% | 161% |
| Ex. 77 | BCZ | GEH3_D2 | Ir(ppy)₃ | ETM3 | 36% | -0.76 | 137% | 133% |
| Ex. 78 | BCZ | GEH3_D3 | Ir(ppy)₃ | ETM3 | 32% | -0.76 | 137% | 133% |
| Ex. 79 | BCZ | GEH3_D4 | Ir(ppy)₃ | ETM3 | 68% | -0.66 | 134% | 138% |
| Ex. 80 | BCZ | GEH3_D5 | Ir(ppy)₃ | ETM3 | 100% | -0.66 | 134% | 170% |
| Ex. 33 | BCZ | GEH3 | Ir(ppy)₃ | ETM2+ETM3 | 0% | -1.26 | 162% | 119% |
| Ex. 81 | BCZ | GEH3_D1 | Ir(ppy)₃ | ETM2+ETM3 | 32% | -1.26 | 162% | 149% |
| Ex. 82 | BCZ | GEH3_D2 | Ir(ppy)₃ | ETM2+ETM3 | 36% | -1.26 | 162% | 123% |
| Ex. 83 | BCZ | GEH3_D3 | Ir(ppy)₃ | ETM2+ETM3 | 32% | -1.26 | 162% | 123% |
| Ex. 84 | BCZ | GEH3_D4 | Ir(ppy)₃ | ETM2+ETM3 | 68% | -1.16 | 159% | 127% |
| Ex. 85 | BCZ | GEH3_D5 | Ir(ppy)₃ | ETM2+ETM3 | 100% | -1.16 | 159% | 157% |

### Examples 86-90 (Ex. 86-90): Fabrication of OLEDs

An OLED was fabricated using the same procedure and the same materials as Example 14, except that each of Compound GEH4-D1 (Ex. 86), Compound GEH4-D2 (Ex. 87), Compound GEH4-D3 (Ex. 88), Compound GEH4-D4 (Ex. 89) and Compound GEH4-D5 (Ex. 90) instead of Compound GEH4 was used as the N-type host in the EML, respectively.

### Examples 91-95 (Ex. 91-95): Fabrication of OLEDs

An OLED was fabricated using the same procedure and the same materials as Example 24, except that each of Compound GEH4-D1 (Ex. 91), Compound GEH4-D2 (Ex. 92), Compound GEH4-D3 (Ex. 93), Compound GEH4-D4 (Ex. 94) and Compound GEH4-D5 (Ex. 95) instead of Compound GEH4 was used as the N-type host in the EML, respectively.

### Examples 96-100 (Ex. 96-100): Fabrication of OLEDs

An OLED was fabricated using the same procedure and the same materials as Example 34, except that each of Compound GEH4-D1 (Ex. 96), Compound GEH4-D2 (Ex. 97), Compound GEH4-D3 (Ex. 98), Compound GEH4-D4 (Ex. 99) and Compound GEH4-D5 (Ex. 100) instead of Compound GEH4 was used as the N-type host in the EML, respectively.

### Experimental Example 6: Measurement of Luminous Properties of OLEDs

Luminous properties for each of the OLEDs fabricated in Examples 86 to 100 were measured as Experimental Example 2. The measurement results are indicated in the following Table 7.

**Table 7: Luminous Properties of OLED**

| Sample | G-EML | | | ETL | DR | V | L | LT₉₅ |
|---|---|---|---|---|---|---|---|---|
| | GHH | GEH | GD | | | | | |
| Ref. 1 | BCZ | TCPZ | Ir(ppy)₃ | ETM1 | 0% | 0.00 | 100% | 100% |
| Ex. 4 | BCZ | GEH4 | Ir(ppy)₃ | ETM1 | 0% | -1.33 | 158% | 98% |
| Ex. 14 | BCZ | GEH4 | Ir(ppy)₃ | ETM2 | 0% | -1.67 | 173% | 97% |
| Ex. 86 | BCZ | GEH4_D1 | Ir(ppy)₃ | ETM2 | 35% | -1.67 | 173% | 121% |
| Ex. 87 | BCZ | GEH4_D2 | Ir(ppy)₃ | ETM2 | 30% | -1.67 | 173% | 100% |
| Ex. 88 | BCZ | GEH4_D3 | Ir(ppy)₃ | ETM2 | 35% | -1.67 | 173% | 100% |
| Ex. 89 | BCZ | GEH4_D4 | Ir(ppy)₃ | ETM2 | 65% | -1.57 | 170% | 104% |
| Ex. 90 | BCZ | GEH4_D5 | Ir(ppy)₃ | ETM2 | 100% | -1.57 | 170% | 128% |
| Ex. 24 | BCZ | GEH4 | Ir(ppy)₃ | ETM3 | 0% | -0.82 | 140% | 128% |
| Ex. 91 | BCZ | GEH4_D1 | Ir(ppy)₃ | ETM3 | 35% | -0.82 | 140% | 161% |
| Ex. 92 | BCZ | GEH4_D2 | Ir(ppy)₃ | ETM3 | 30% | -0.82 | 140% | 132% |
| Ex. 93 | BCZ | GEH4_D3 | Ir(ppy)₃ | ETM3 | 35% | -0.82 | 140% | 132% |
| Ex. 94 | BCZ | GEH4_D4 | Ir(ppy)₃ | ETM3 | 65% | -0.72 | 137% | 137% |
| Ex. 95 | BCZ | GEH4_D5 | Ir(ppy)₃ | ETM3 | 100% | -0.72 | 137% | 169% |
| Ex. 34 | BCZ | GEH4 | Ir(ppy)₃ | ETM2+ETM3 | 0% | -1.36 | 167% | 119% |
| Ex. 96 | BCZ | GEH4_D1 | Ir(ppy)₃ | ETM2+ETM3 | 35% | -1.36 | 167% | 148% |
| Ex. 97 | BCZ | GEH4_D2 | Ir(ppy)₃ | ETM2+ETM3 | 30% | -1.36 | 167% | 122% |
| Ex. 98 | BCZ | GEH4_D3 | Ir(ppy)₃ | ETM2+ETM3 | 35% | -1.36 | 167% | 122% |
| Ex. 99 | BCZ | GEH4_D4 | Ir(ppy)₃ | ETM2+ETM3 | 65% | -1.26 | 163% | 127% |
| Ex. 100 | BCZ | GEH4_D5 | Ir(ppy)₃ | ETM2+ETM3 | 100% | -1.26 | 163% | 156% |

### Examples 101-105 (Ex. 101-105): Fabrication of OLEDs

An OLED was fabricated using the same procedure and the same materials as Example 15, except that each of Compound GEHS-D1 (Ex. 101), Compound GEH5-D2 (Ex. 102), Compound GEH5-D3 (Ex. 103), Compound GEH5-D4 (Ex. 104) and Compound GEH5-D5 (Ex. 105) instead of Compound GEH5 was used as the N-type host in the EML, respectively.

### Examples 106-110 (Ex. 106-110): Fabrication of OLEDs

An OLED was fabricated using the same procedure and the same materials as Example 25, except that each of Compound GEHS-D1 (Ex. 106), Compound GEH5-D2 (Ex. 107), Compound GEH5-D3 (Ex. 108), Compound GEH5-D4 (Ex. 109) and Compound GEH5-D5 (Ex. 110) instead of Compound GEH5 was used as the N-type host in the EML, respectively.

### Examples 111-115 (Ex. 111-115): Fabrication of OLEDs

An OLED was fabricated using the same procedure and the same materials as Example 35, except that each of Compound GEH5-D1 (Ex. 111), Compound GEH5-D2 (Ex. 112), Compound GEH5-D3 (Ex. 113), Compound GEH5-D4 (Ex. 114) and Compound GEH5-D5 (Ex. 115) instead of Compound GEH5 was used as the N-type host in the EML, respectively.

### Experimental Example 7: Measurement of Luminous Properties of OLEDs

Luminous properties for each of the OLEDs fabricated in Examples 101 to 115 were measured as Experimental Example 2. The measurement results are indicated in the following Table 8.

**Table 8: Luminous Properties of OLED**

| Sample | G-EML | | | ETL | DR | V | L | LT₉₅ |
|---|---|---|---|---|---|---|---|---|
| | GHH | GEH | GD | | | | | |
| Ref. 1 | BCZ | TCPZ | Ir(ppy)₃ | ETM1 | 0% | 0.00 | 100% | 100% |
| Ex. 5 | BCZ | GEH5 | Ir(ppy)₃ | ETM1 | 0% | -1.17 | 152% | 99% |
| Ex. 15 | BCZ | GEH5 | Ir(ppy)₃ | ETM2 | 0% | -1.48 | 165% | 98% |
| Ex. 101 | BCZ | GEH5_D1 | Ir(ppy)₃ | ETM2 | 32% | -1.48 | 165% | 122% |
| Ex. 102 | BCZ | GEH5_D2 | Ir(ppy)₃ | ETM2 | 36% | -1.48 | 165% | 101% |
| Ex. 103 | BCZ | GEH5_D3 | Ir(ppy)₃ | ETM2 | 32% | -1.48 | 165% | 101% |
| Ex. 104 | BCZ | GEH5_D4 | Ir(ppy)₃ | ETM2 | 68% | -1.38 | 161% | 105% |
| Ex. 105 | BCZ | GEH5_D5 | Ir(ppy)₃ | ETM2 | 100% | -1.38 | 161% | 129% |
| Ex. 25 | BCZ | GEH5 | Ir(ppy)₃ | ETM3 | 0% | -0.73 | 135% | 129% |
| Ex. 106 | BCZ | GEH5_D1 | Ir(ppy)₃ | ETM3 | 32% | -0.73 | 135% | 161% |
| Ex. 107 | BCZ | GEH5_D2 | Ir(ppy)₃ | ETM3 | 36% | -0.73 | 135% | 132% |
| Ex. 108 | BCZ | GEH5_D3 | Ir(ppy)₃ | ETM3 | 32% | -0.73 | 135% | 132% |
| Ex. 109 | BCZ | GEH5_D4 | Ir(ppy)₃ | ETM3 | 68% | -0.63 | 133% | 138% |
| Ex. 110 | BCZ | GEH5_D5 | Ir(ppy)₃ | ETM3 | 100% | -0.63 | 133% | 170% |
| Ex. 35 | BCZ | GEH5 | Ir(ppy)₃ | ETM2+ETM3 | 0% | -1.21 | 160% | 119% |
| Ex. 111 | BCZ | GEH5_D1 | Ir(ppy)₃ | ETM2+ETM3 | 32% | -1.21 | 160% | 149% |
| Ex. 112 | BCZ | GEH5_D2 | Ir(ppy)₃ | ETM2+ETM3 | 36% | -1.21 | 160% | 123% |
| Ex. 113 | BCZ | GEH5_D3 | Ir(ppy)₃ | ETM2+ETM3 | 32% | -1.21 | 160% | 123% |
| Ex. 114 | BCZ | GEH5_D4 | Ir(ppy)₃ | ETM2+ETM3 | 68% | -1.11 | 157% | 127% |
| Ex. 115 | BCZ | GEH5_D5 | Ir(ppy)₃ | ETM2+ETM3 | 100% | -1.11 | 157% | 157% |

### Examples 116-120 (Ex. 116-120): Fabrication of OLEDs

An OLED was fabricated using the same procedure and the same materials as Example 16, except that each of Compound GEH6-D1 (Ex. 116), Compound GEH6-D2 (Ex. 117), Compound GEH6-D3 (Ex. 118), Compound GEH6-D4 (Ex. 119) and Compound GEH6-D5 (Ex. 120) instead of Compound GEH6 was used as the N-type host in the EML, respectively.

### Examples 121-125 (Ex. 121-125): Fabrication of OLEDs

An OLED was fabricated using the same procedure and the same materials as Example 26, except that each of Compound GEH6-D1 (Ex. 121), Compound GEH6-D2 (Ex. 122), Compound GEH6-D3 (Ex. 123), Compound GEH6-D4 (Ex. 124) and Compound GEH6-D5 (Ex. 125) instead of Compound GEH6 was used as the N-type host in the EML, respectively.

### Examples 126-130 (Ex. 126-130): Fabrication of OLEDs

An OLED was fabricated using the same procedure and the same materials as Example 36, except that each of Compound GEH6-D1 (Ex. 126), Compound GEH6-D2 (Ex. 127), Compound GEH6-D3 (Ex. 128), Compound GEH6-D4 (Ex. 129) and Compound GEH6-D5 (Ex. 130) instead of Compound GEH6 was used as the N-type host in the EML, respectively.

### Experimental Example 8: Measurement of Luminous Properties of OLEDs

Luminous properties for each of the OLEDs fabricated in Examples 116 to 130 were measured as Experimental Example 2. The measurement results are indicated in the following Table 9.

**Table 9: Luminous Properties of OLED**

| Sample | G-EML | | | ETL | DR | V | L | LT₉₅ |
|---|---|---|---|---|---|---|---|---|
| | GHH | GEH | GD | | | | | |
| Ref. 1 | BCZ | TCPZ | Ir(ppy)₃ | ETM1 | 0% | 0.00 | 100% | 100% |
| Ex. 6 | BCZ | GEH6 | Ir(ppy)₃ | ETM1 | 0% | -0.84 | 137% | 101% |
| Ex. 16 | BCZ | GEH6 | Ir(ppy)₃ | ETM2 | 0% | -1.06 | 147% | 101% |
| Ex. 116 | BCZ | GEH6_D1 | Ir(ppy)₃ | ETM2 | 35% | -1.06 | 147% | 126% |
| Ex. 117 | BCZ | GEH6_D2 | Ir(ppy)₃ | ETM2 | 30% | -1.06 | 147% | 104% |
| Ex. 118 | BCZ | GEH6_D3 | Ir(ppy)₃ | ETM2 | 35% | -1.06 | 147% | 104% |
| Ex. 119 | BCZ | GEH6_D4 | Ir(ppy)₃ | ETM2 | 65% | -0.96 | 144% | 108% |
| Ex. 120 | BCZ | GEH6_D5 | Ir(ppy)₃ | ETM2 | 100% | -0.96 | 144% | 133% |
| Ex. 26 | BCZ | GEH6 | Ir(ppy)₃ | ETM3 | 0% | -0.52 | 127% | 130% |
| Ex. 121 | BCZ | GEH6_D1 | Ir(ppy)₃ | ETM3 | 35% | -0.52 | 127% | 163% |
| Ex. 122 | BCZ | GEH6_D2 | Ir(ppy)₃ | ETM3 | 30% | -0.52 | 127% | 134% |
| Ex. 123 | BCZ | GEH6_D3 | Ir(ppy)₃ | ETM3 | 35% | -0.52 | 127% | 134% |
| Ex. 124 | BCZ | GEH6_D4 | Ir(ppy)₃ | ETM3 | 65% | -0.42 | 124% | 139% |
| Ex. 125 | BCZ | GEH6_D5 | Ir(ppy)₃ | ETM3 | 100% | -0.42 | 124% | 172% |
| Ex. 36 | BCZ | GEH6 | Ir(ppy)₃ | ETM2+ETM3 | 0% | -0.90 | 147% | 121% |
| Ex. 126 | BCZ | GEH6_D1 | Ir(ppy)₃ | ETM2+ETM3 | 35% | -0.90 | 147% | 152% |
| Ex. 127 | BCZ | GEH6_D2 | Ir(ppy)₃ | ETM2+ETM3 | 30% | -0.90 | 147% | 125% |
| Ex. 128 | BCZ | GEH6_D3 | Ir(ppy)₃ | ETM2+ETM3 | 35% | -0.90 | 147% | 125% |
| Ex. 129 | BCZ | GEH6_D4 | Ir(ppy)₃ | ETM2+ETM3 | 65% | -0.80 | 144% | 130% |
| Ex. 130 | BCZ | GEH6_D5 | Ir(ppy)₃ | ETM2+ETM3 | 100% | -0.80 | 144% | 160% |

### Examples 131-135 (Ex. 131-135): Fabrication of OLEDs

An OLED was fabricated using the same procedure and the same materials as Example 17, except that each of Compound GEH7-D1 (Ex. 131), Compound GEH7-D2 (Ex. 132), Compound GEH7-D3 (Ex. 133), Compound GEH7-D4 (Ex. 134) and Compound GEH7-D5 (Ex. 135) instead of Compound GEH7 was used as the N-type host in the EML, respectively.

### Examples 136-140 (Ex. 136-140): Fabrication of OLEDs

An OLED was fabricated using the same procedure and the same materials as Example 27, except that each of Compound GEH7-D1 (Ex. 136), Compound GEH7-D2 (Ex. 137), Compound GEH7-D3 (Ex. 138), Compound GEH7-D4 (Ex. 139) and Compound GEH7-D5 (Ex. 140) instead of Compound GEH7 was used as the N-type host in the EML, respectively.

### Examples 141-145 (Ex. 141-145): Fabrication of OLEDs

An OLED was fabricated using the same procedure and the same materials as Example 37, except that each of Compound GEH7-D1 (Ex. 141), Compound GEH7-D2 (Ex. 142), Compound GEH7-D3 (Ex. 143), Compound GEH7-D4 (Ex. 144) and Compound GEH7-D5 (Ex. 145) instead of Compound GEH7 was used as the N-type host in the EML, respectively.

### Experimental Example 9: Measurement of Luminous Properties of OLEDs

Luminous properties for each of the OLEDs fabricated in Examples 131 to 145 were measured as Experimental Example 2. The measurement results are indicated in the following Table 10.

**Table 10: Luminous Properties of OLED**

| Sample | G-EML | | | ETL | DT | V | L | LT₉₅ |
|---|---|---|---|---|---|---|---|---|
| | GHH | GEH | GD | | | | | |
| Ref. 1 | BCZ | TCPZ | Ir(ppy)₃ | ETM1 | 0% | 0.00 | 100% | 100% |
| Ex. 7 | BCZ | GEH7 | Ir(ppy)₃ | ETM1 | 0% | -1.03 | 146% | 99% |
| Ex. 17 | BCZ | GEH7 | Ir(ppy)₃ | ETM2 | 0% | -1.31 | 157% | 99% |
| Ex. 131 | BCZ | GEH7_D1 | Ir(ppy)₃ | ETM2 | 32% | -1.31 | 157% | 123% |
| Ex. 132 | BCZ | GEH7_D2 | Ir(ppy)₃ | ETM2 | 28% | -1.31 | 157% | 102% |
| Ex. 133 | BCZ | GEH7_D3 | Ir(ppy)₃ | ETM2 | 32% | -1.31 | 157% | 102% |
| Ex. 134 | BCZ | GEH7_D4 | Ir(ppy)₃ | ETM2 | 60% | -1.21 | 154% | 105% |
| Ex. 135 | BCZ | GEH7_D5 | Ir(ppy)₃ | ETM2 | 100% | -1.21 | 154% | 130% |
| Ex. 27 | BCZ | GEH7 | Ir(ppy)₃ | ETM3 | 0% | -0.64 | 132% | 129% |
| Ex. 136 | BCZ | GEH7_D1 | Ir(ppy)₃ | ETM3 | 32% | -0.64 | 132% | 161% |
| Ex. 137 | BCZ | GEH7_D2 | Ir(ppy)₃ | ETM3 | 28% | -0.64 | 132% | 133% |
| Ex. 138 | BCZ | GEH7_D3 | Ir(ppy)₃ | ETM3 | 32% | -0.64 | 132% | 133% |
| Ex. 139 | BCZ | GEH7_D4 | Ir(ppy)₃ | ETM3 | 60% | -0.54 | 129% | 138% |
| Ex. 140 | BCZ | GEH7_D5 | Ir(ppy)₃ | ETM3 | 100% | -0.54 | 129% | 170% |
| Ex. 37 | BCZ | GEH7 | Ir(ppy)₃ | ETM2+ETM3 | 0% | -1.08 | 154% | 120% |
| Ex. 141 | BCZ | GEH7_D1 | Ir(ppy)₃ | ETM2+ETM3 | 32% | -1.08 | 154% | 149% |
| Ex. 142 | BCZ | GEH7_D2 | Ir(ppy)₃ | ETM2+ETM3 | 28% | -1.08 | 154% | 123% |
| Ex. 143 | BCZ | GEH7_D3 | Ir(ppy)₃ | ETM2+ETM3 | 32% | -1.08 | 154% | 123% |
| Ex. 144 | BCZ | GEH7_D4 | Ir(ppy)₃ | ETM2+ETM3 | 60% | -0.98 | 151% | 128% |
| Ex. 145 | BCZ | GEH7_D5 | Ir(ppy)₃ | ETM2+ETM3 | 100% | -0.98 | 151% | 158% |

### Examples 146-150 (Ex. 146-150): Fabrication of OLEDs

An OLED was fabricated using the same procedure and the same materials as Example 18, except that each of Compound GEH8-D1 (Ex. 146), Compound GEH8-D2 (Ex. 147), Compound GEH8-D3 (Ex. 148), Compound GEH8-D4 (Ex. 149) and Compound GEH8-D5 (Ex. 150) instead of Compound GEH8 was used as the N-type host in the EML, respectively.

### Examples 151-155 (Ex. 151-155): Fabrication of OLEDs

An OLED was fabricated using the same procedure and the same materials as Example 28, except that each of Compound GEH8-D1 (Ex. 151), Compound GEH8-D2 (Ex. 152), Compound GEH8-D3 (Ex. 153), Compound GEH8-D4 (Ex. 154) and Compound GEH8-D5 (Ex. 155) instead of Compound GEH8 was used as the N-type host in the EML, respectively.

### Examples 156-160 (Ex. 156-160): Fabrication of OLEDs

An OLED was fabricated using the same procedure and the same materials as Example 38, except that each of Compound GEH8-D1 (Ex. 156), Compound GEH8-D2 (Ex. 157), Compound GEH8-D3 (Ex. 158), Compound GEH8-D4 (Ex. 159) and Compound GEH8-D5 (Ex. 160) instead of Compound GEH8 was used as the N-type host in the EML, respectively.

### Experimental Example 10: Measurement of Luminous Properties of OLEDs

Luminous properties for each of the OLEDs fabricated in Examples 146 to 160 were measured as Experimental Example 2. The measurement results are indicated in the following Table 11.

**Table 11: Luminous Properties of OLED**

| Sample | G-EML | | | ETL | DR | V | L | LT₉₅ |
|---|---|---|---|---|---|---|---|---|
| | GHH | GEH | GD | | | | | |
| Ref. 1 | BCZ | TCPZ | Ir(ppy)₃ | ETM1 | 0% | 0.00 | 100% | 100% |
| Ex. 8 | BCZ | GEH8 | Ir(ppy)₃ | ETM1 | 0% | -0.40 | 118% | 104% |
| Ex. 18 | BCZ | GEH8 | Ir(ppy)₃ | ETM2 | 0% | -0.50 | 123% | 104% |
| Ex. 146 | BCZ | GEH8_D1 | Ir(ppy)₃ | ETM2 | 35% | -0.50 | 123% | 130% |
| Ex. 147 | BCZ | GEH8_D2 | Ir(ppy)₃ | ETM2 | 30% | -0.50 | 123% | 107% |
| Ex. 148 | BCZ | GEH8_D3 | Ir(ppy)₃ | ETM2 | 35% | -0.50 | 123% | 107% |
| Ex. 149 | BCZ | GEH8_D4 | Ir(ppy)₃ | ETM2 | 65% | -0.40 | 120% | 111% |
| Ex. 150 | BCZ | GEH8_D5 | Ir(ppy)₃ | ETM2 | 100% | -0.40 | 120% | 137% |
| Ex. 28 | BCZ | GEH8 | Ir(ppy)₃ | ETM3 | 0% | -0.25 | 115% | 132% |
| Ex. 151 | BCZ | GEH8_D1 | Ir(ppy)₃ | ETM3 | 35% | -0.25 | 115% | 164% |
| Ex. 152 | BCZ | GEH8_D2 | Ir(ppy)₃ | ETM3 | 30% | -0.25 | 115% | 136% |
| Ex. 153 | BCZ | GEH8_D3 | Ir(ppy)₃ | ETM3 | 35% | -0.25 | 115% | 136% |
| Ex. 154 | BCZ | GEH8_D4 | Ir(ppy)₃ | ETM3 | 65% | -0.15 | 113% | 141% |
| Ex. 155 | BCZ | GEH8_D5 | Ir(ppy)₃ | ETM3 | 100% | -0.15 | 113% | 174% |
| Ex. 38 | BCZ | GEH8 | Ir(ppy)₃ | ETM2+ETM3 | 0% | -0.48 | 129% | 124% |
| Ex. 156 | BCZ | GEH8_D1 | Ir(ppy)₃ | ETM2+ETM3 | 35% | -0.48 | 129% | 155% |
| Ex. 157 | BCZ | GEH8_D2 | Ir(ppy)₃ | ETM2+ETM3 | 30% | -0.48 | 129% | 127% |
| Ex. 158 | BCZ | GEH8_D3 | Ir(ppy)₃ | ETM2+ETM3 | 35% | -0.48 | 129% | 127% |
| Ex. 159 | BCZ | GEH8_D4 | Ir(ppy)₃ | ETM2+ETM3 | 65% | -0.38 | 126% | 132% |
| Ex. 160 | BCZ | GEH8_D5 | Ir(ppy)₃ | ETM2+ETM3 | 100% | -0.38 | 126% | 163% |

### Examples 161-165 (Ex. 161-165): Fabrication of OLEDs

An OLED was fabricated using the same procedure and the same materials as Example 19, except that each of Compound GEH9-D1 (Ex. 161), Compound GEH9-D2 (Ex. 162), Compound GEH9-D3 (Ex. 163), Compound GEH9-D4 (Ex. 164) and Compound GEH9-D5 (Ex. 165) instead of Compound GEH9 was used as the N-type host in the EML, respectively.

### Examples 166-170 (Ex. 166-170): Fabrication of OLEDs

An OLED was fabricated using the same procedure and the same materials as Example 29, except that each of Compound GEH9-D1 (Ex. 166), Compound GEH9-D2 (Ex. 167), Compound GEH9-D3 (Ex. 168), Compound GEH9-D4 (Ex. 179) and Compound GEH7-D5 (Ex. 170) instead of Compound GEH9 was used as the N-type host in the EML, respectively.

### Examples 171-175 (Ex. 171-175): Fabrication of OLEDs

An OLED was fabricated using the same procedure and the same materials as Example 39, except that each of Compound GEH9-D1 (Ex. 171), Compound GEH9-D2 (Ex. 172), Compound GEH9-D3 (Ex. 173), Compound GEH9-D4 (Ex. 174) and Compound GEH9-D5 (Ex. 175) instead of Compound GEH9 was used as the N-type host in the EML, respectively.

### Experimental Example 11: Measurement of Luminous Properties of OLEDs

Luminous properties for each of the OLEDs fabricated in Examples 161 to 175 were measured as Experimental Example 2. The measurement results are indicated in the following Table 12.

**Table 12: Luminous Properties of OLED**

| Sample | G-EML | | | ETL | DR | V | L | LT₉₅ |
|---|---|---|---|---|---|---|---|---|
| | GHH | GEH | GD | | | | | |
| Ref. 1 | BCZ | TCPZ | Ir(ppy)₃ | ETM1 | 0% | 0.00 | 100% | 100% |
| Ex. 9 | BCZ | GEH9 | Ir(ppy)₃ | ETM1 | 0% | -0.99 | 144% | 100% |
| Ex. 19 | BCZ | GEH9 | Ir(ppy)₃ | ETM2 | 0% | -1.25 | 155% | 100% |
| Ex. 161 | BCZ | GEH9_D1 | Ir(ppy)₃ | ETM2 | 38% | -1.25 | 155% | 125% |
| Ex. 162 | BCZ | GEH9_D2 | Ir(ppy)₃ | ETM2 | 43% | -1.25 | 155% | 103% |
| Ex. 163 | BCZ | GEH9_D3 | Ir(ppy)₃ | ETM2 | 19% | -1.25 | 155% | 103% |
| Ex. 164 | BCZ | GEH9_D4 | Ir(ppy)₃ | ETM2 | 62% | -1.15 | 152% | 107% |
| Ex. 165 | BCZ | GEH9_D5 | Ir(ppy)₃ | ETM2 | 100% | -1.15 | 152% | 131% |
| Ex. 29 | BCZ | GEH9 | Ir(ppy)₃ | ETM3 | 0% | -0.62 | 131% | 130% |
| Ex. 166 | BCZ | GEH9_D1 | Ir(ppy)₃ | ETM3 | 38% | -0.62 | 131% | 162% |
| Ex. 167 | BCZ | GEH9_D2 | Ir(ppy)₃ | ETM3 | 43% | -0.62 | 131% | 134% |
| Ex. 168 | BCZ | GEH9_D3 | Ir(ppy)₃ | ETM3 | 19% | -0.62 | 131% | 134% |
| Ex. 169 | BCZ | GEH9_D4 | Ir(ppy)₃ | ETM3 | 62% | -0.52 | 128% | 139% |
| Ex. 170 | BCZ | GEH9_D5 | Ir(ppy)₃ | ETM3 | 100% | -0.52 | 128% | 171% |
| Ex. 39 | BCZ | GEH9 | Ir(ppy)₃ | ETM2+ETM3 | 0% | -1.05 | 153% | 120% |
| Ex. 171 | BCZ | GEH9_D1 | Ir(ppy)₃ | ETM2+ETM3 | 38% | -1.05 | 153% | 151% |
| Ex. 172 | BCZ | GEH9_D2 | Ir(ppy)₃ | ETM2+ETM3 | 43% | -1.05 | 153% | 124% |
| Ex. 173 | BCZ | GEH9_D3 | Ir(ppy)₃ | ETM2+ETM3 | 19% | -1.05 | 153% | 124% |
| Ex. 174 | BCZ | GEH9_D4 | Ir(ppy)₃ | ETM2+ETM3 | 62% | -0.95 | 150% | 129% |
| Ex. 175 | BCZ | GEH9_D5 | Ir(ppy)₃ | ETM2+ETM3 | 100% | -0.95 | 150% | 159% |

### Examples 176-180 (Ex. 176-180): Fabrication of OLEDs

An OLED was fabricated using the same procedure and the same materials as Example 20, except that each of Compound GEH10-D1 (Ex. 176), Compound GEH10-D2 (Ex. 177), Compound GEH10-D3 (Ex. 178), Compound GEH10-D4 (Ex. 179) and Compound GEH10-DS (Ex. 180) instead of Compound GEH10 was used as the N-type host in the EML, respectively.

### Examples 181-185 (Ex. 181-185): Fabrication of OLEDs

An OLED was fabricated using the same procedure and the same materials as Example 30, except that each of Compound GEH10-D1 (Ex. 181), Compound GEH10-D2 (Ex. 182), Compound GEH10-D3 (Ex. 183), Compound GEH10-D4 (Ex. 184) and Compound GEH10-DS (Ex. 185) instead of Compound GEH10 was used as the N-type host in the EML, respectively.

### Examples 186-190 (Ex. 186-190): Fabrication of OLEDs

An OLED was fabricated using the same procedure and the same materials as Example 40, except that each of Compound GEH10-D1 (Ex. 186), Compound GEH10-D2 (Ex. 187), Compound GEH10-D3 (Ex. 188), Compound GEH10-D4 (Ex. 189) and Compound GEH10-DS (Ex. 190) instead of Compound GEH10 was used as the N-type host in the EML, respectively.

### Experimental Example 12: Measurement of Luminous Properties of OLEDs

Luminous properties for each of the OLEDs fabricated in Examples 176 to 190 were measured as Experimental Example 2. The measurement results are indicated in the following Table 13.

**Table 13: Luminous Properties of OLED**

| Sample | G-EML | | | ETL | DR | V | L | LT₉₅ |
|---|---|---|---|---|---|---|---|---|
| | GHH | GEH | GD | | | | | |
| Ref. 1 | BCZ | TCPZ | Ir(ppy)₃ | ETM1 | 0% | 0.00 | 100% | 100% |
| Ex. 10 | BCZ | GEH10 | Ir(ppy)₃ | ETM1 | 0% | -0.90 | 140% | 101% |
| Ex. 20 | BCZ | GEH10 | Ir(ppy)₃ | ETM2 | 0% | -1.14 | 150% | 100% |
| Ex. 176 | BCZ | GEH10_D1 | Ir(ppy)₃ | ETM2 | 42% | -1.14 | 150% | 126% |
| Ex. 177 | BCZ | GEH10_D2 | Ir(ppy)₃ | ETM2 | 37% | -1.14 | 150% | 103% |
| Ex. 178 | BCZ | GEH10_D3 | Ir(ppy)₃ | ETM2 | 21% | -1.14 | 150% | 103% |
| Ex. 179 | BCZ | GEH10_D4 | Ir(ppy)₃ | ETM2 | 58% | -1.04 | 147% | 107% |
| Ex. 180 | BCZ | GEH10_D5 | Ir(ppy)₃ | ETM2 | 100% | -1.04 | 147% | 133% |
| Ex. 30 | BCZ | GEH10 | Ir(ppy)₃ | ETM3 | 0% | -0.56 | 128% | 130% |
| Ex. 181 | BCZ | GEH10_D1 | Ir(ppy)₃ | ETM3 | 42% | -0.56 | 128% | 163% |
| Ex. 182 | BCZ | GEH10_D2 | Ir(ppy)₃ | ETM3 | 37% | -0.56 | 128% | 134% |
| Ex. 183 | BCZ | GEH10_D3 | Ir(ppy)₃ | ETM3 | 21% | -0.56 | 128% | 134% |
| Ex. 184 | BCZ | GEH10_D4 | Ir(ppy)₃ | ETM3 | 58% | -0.46 | 126% | 139% |
| Ex. 185 | BCZ | GEH10_D5 | Ir(ppy)₃ | ETM3 | 100% | -0.46 | 126% | 172% |
| Ex. 40 | BCZ | GEH10 | Ir(ppy)₃ | ETM2+ETM3 | 0% | -0.96 | 149% | 121% |
| Ex. 186 | BCZ | GEH10_D1 | Ir(ppy)₃ | ETM2+ETM3 | 42% | -0.96 | 149% | 151% |
| Ex. 187 | BCZ | GEH10_D2 | Ir(ppy)₃ | ETM2+ETM3 | 37% | -0.96 | 149% | 125% |
| Ex. 188 | BCZ | GEH10_D3 | Ir(ppy)₃ | ETM2+ETM3 | 21% | -0.96 | 149% | 125% |
| Ex. 189 | BCZ | GEH10_D4 | Ir(ppy)₃ | ETM2+ETM3 | 58% | -0.86 | 146% | 130% |
| Ex. 190 | BCZ | GEH10_D5 | Ir(ppy)₃ | ETM2+ETM3 | 100% | -0.86 | 146% | 160% |

As indicated in Tables 4 to 6, with the same deuteration rate, compared to the OLEDs fabricated in Ref. 13-26 where the reference compounds having the benzoxazole moiety were used as the N-type host in the EML, in the OLEDs fabricated in Ex. 41-85 where the compounds having the benzothiazole moiety were used as the N-type host in the EML, the driving voltage was further lowered, and luminance and luminous lifetime were significantly improved. In addition, as indicated in Tables 4 to 13, as the deuterium substitution rate in the molecule increased, the driving voltage and luminance were maintained at the same level, but the luminescence lifetime was greatly improved.

It will be apparent to those skilled in the art that various modifications and variations may be made in the present invention without departing from the scope of the invention. Thus, it is intended that the present invention cover the modifications and variations of the present invention provided they come within the scope of the appended claims.

## Claims

1. An organic compound having the following structure of Chemical Formula 1: 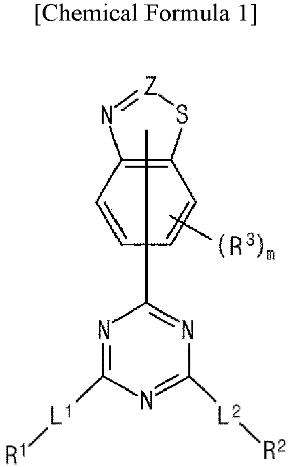 wherein, in the Chemical Formula 1,
each of R¹ and R² is independently an unsubstituted or substituted C₆-C₃₀ aryl group or an unsubstituted or substituted C₃-C₃₀ hetero aryl group;
R³ is independently deuterium, an unsubstituted or substituted C₁-C₂₀ alkyl group, an unsubstituted or substituted C₆-C₃₀ aryl group or an unsubstituted or substituted C₃-C₃₀ hetero aryl group, where each R³ is identical to or different from each other when m is 2, 3 or 4;
Z is CR⁴ or a carbon atom linked to a triazine moiety, where R⁴ is hydrogen, deuterium, an unsubstituted or substituted C₁-C₂₀ alkyl group, an unsubstituted or substituted C₆-C₃₀ aryl group or an unsubstituted or substituted C₃-C₃₀ hetero aryl group,
wherein at least one of R¹, R², R³ and R⁴ is deuterium, a deuterium-substituted C₁-C₂₀ alkyl group, a deuterium-substituted C₆-C₃₀ aryl group or a deuterium-substituted C₃-C₃₀ hetero aryl group, and at least one of R¹, R² and R⁴ is an unsubstituted or substituted C₁₀-C₃₀ fused hetero aryl group with at least one nitrogen atom;
each of L¹ and L² is independently a single bond or an unsubstituted or substituted C6-C30 arylene group; and
m is 0, 1, 2 or 3 when Z is CR⁴ and m is 0, 1, 2, 3 or 4 when Z is the carbon atom linked to the triazine moiety.

2. The organic compound of claim 1, wherein the organic compound has the following structure of Chemical Formula 2: 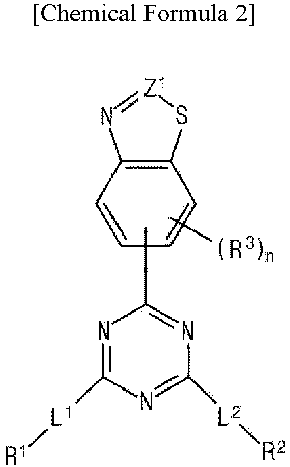 wherein, in the Chemical Formula 2,
each of R¹, R², R³, L¹ and L² is a same as defined in Chemical Formula 1;
Z¹ is CR⁴, where R⁴ is a same as defined in Chemical Formula 1; and
n is 0, 1, 2 or 3.

3. The organic compound of claim 1 or 2, wherein the organic compound has the following structure of Chemical Formula 3A, Chemical Formula 3B, Chemical Formula 3C or Chemical Formula 3D: 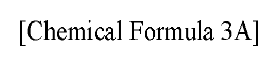 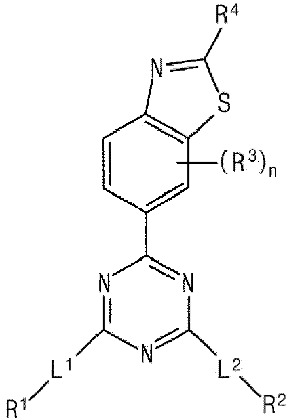 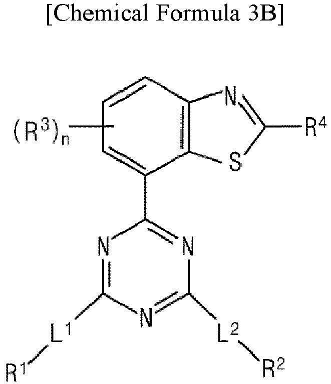 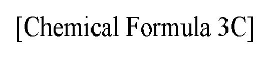 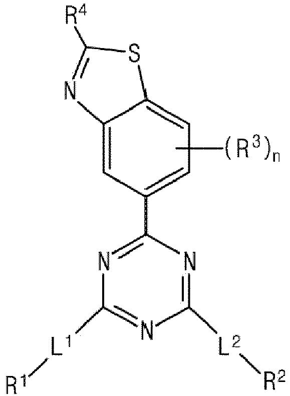 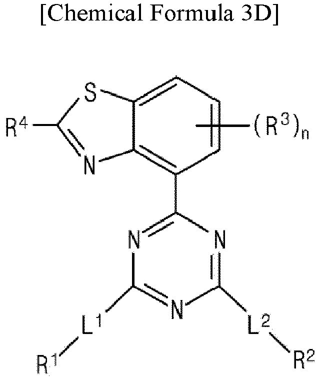 wherein, in the Chemical Formulae 3A, 3B, 3C and 3D,
each of R¹, R², R³, R⁴, L¹ and L² is a same as defined in Chemical Formula 1; and n is 0, 1, 2 or 3.

4. The organic compound of claim 1, wherein the organic compound has the following structure of Chemical Formula 4: 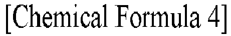 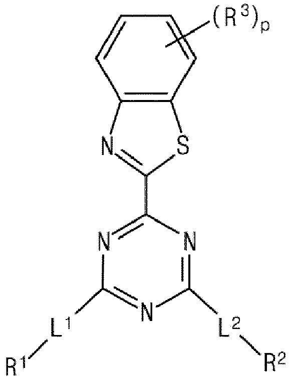 wherein, in the Chemical Formula 4,
each of R¹, R², R³, L¹ and L² is a same as defined in Chemical Formula 1; and
p is 0, 1, 2, 3 or 4.

5. The organic compound of any of claims 1 to 4, wherein one of R¹ and R² in Chemical Formula 1 is a carbazolyl group unsubstituted or substituted with deuterium or a C₁-C₂₀ alkyl group, where the C₁-C₂₀ alkyl group substituted to the carbazolyl group is unsubstituted or substituted with deuterium, the other of R¹ and R² in Chemical Formula 1 is selected from the group consisting of phenyl, biphenyl, pyrenyl, fluorenyl, dibenzofuranyl, dibenzothiophenyl and carbazolyl each of which is independently unsubstituted or substituted with at least one of deuterium, a C₁-C₂₀ alkyl group and a C₆-C₃₀ aryl group, where each of the C₁-C₂₀ alkyl group and the C₆-C₃₀ aryl group substituted to R² is independently unsubstituted or further substituted with deuterium, R³ in Chemical Formula 1 is deuterium or a C₁-C₂₀ alkyl group unsubstituted or substituted with deuterium, R⁴ in Chemical Formula 1 is phenyl unsubstituted or substituted with deuterium or a C₁-C₂₀ alkyl group, where the C₁-C₂₀ alkyl group substituted to the phenyl is unsubstituted with deuterium, and m is 0, 1, 2, 3 or 4.

6. The organic compound of any of claims 1 to 3 and 5, wherein the organic compound is at least one of the following compounds:

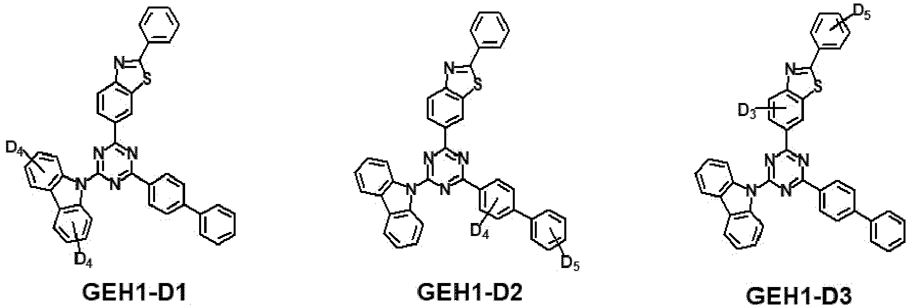

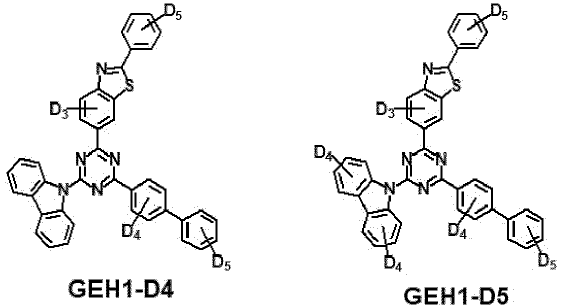

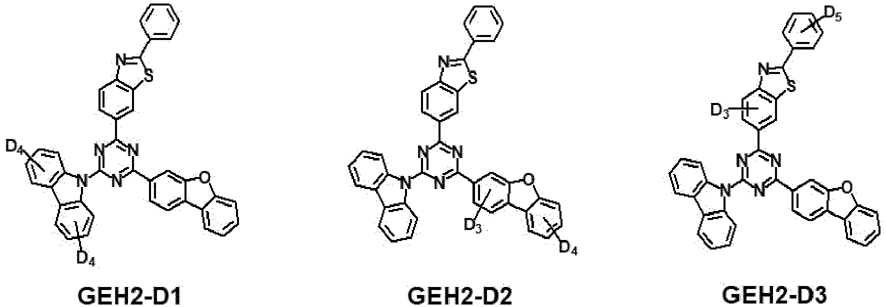

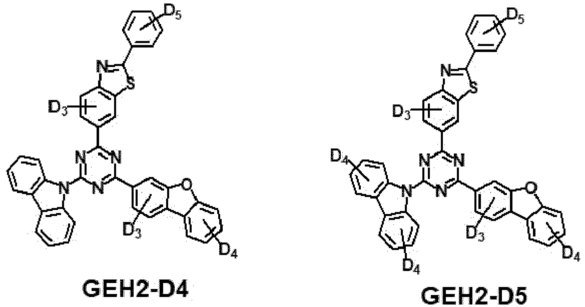

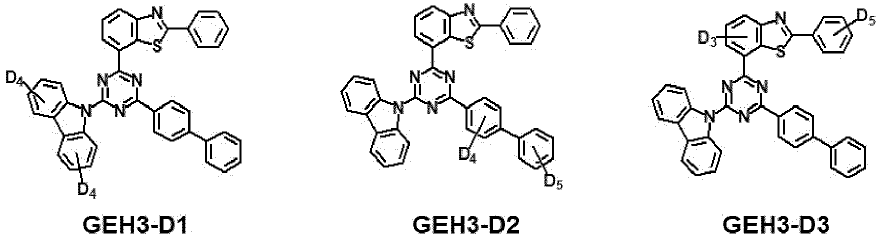

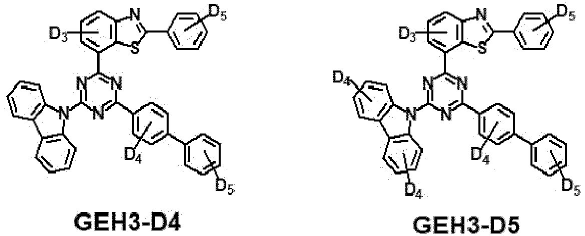

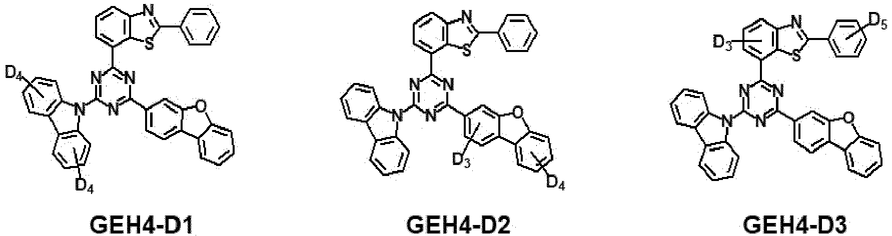

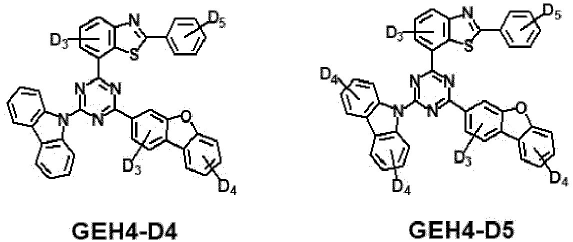

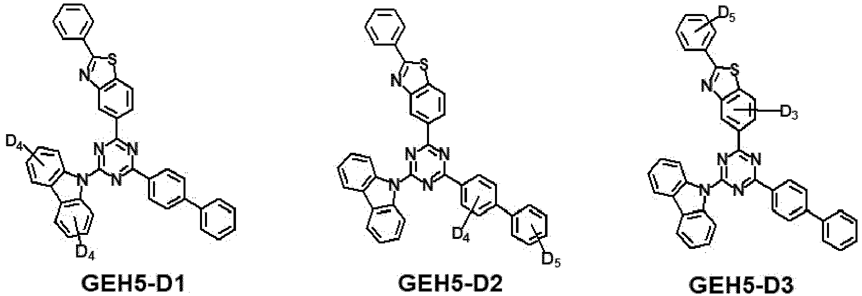

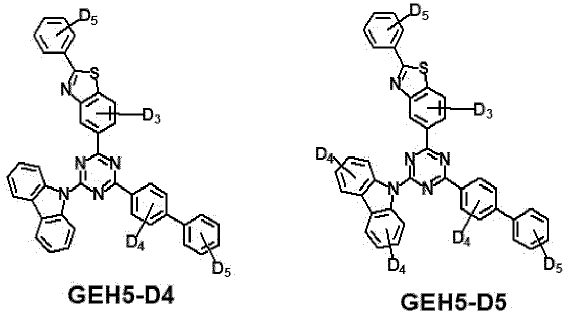

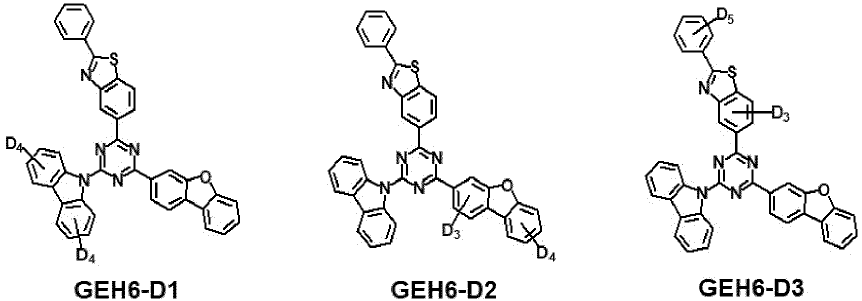

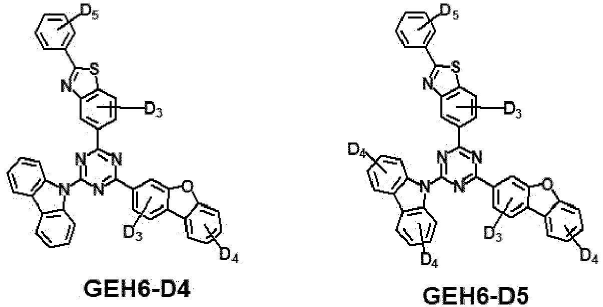

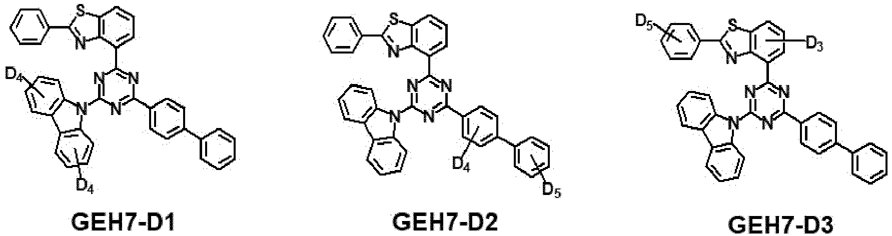

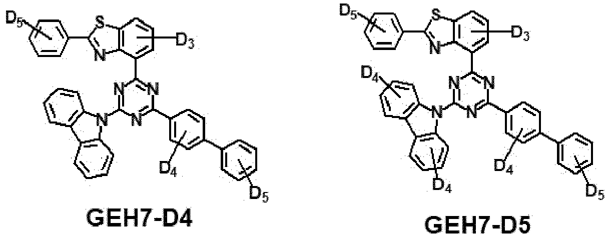

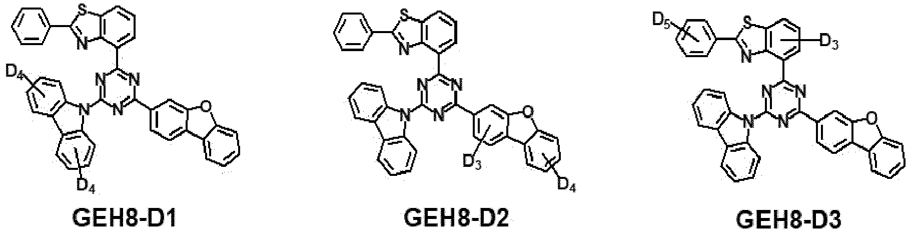

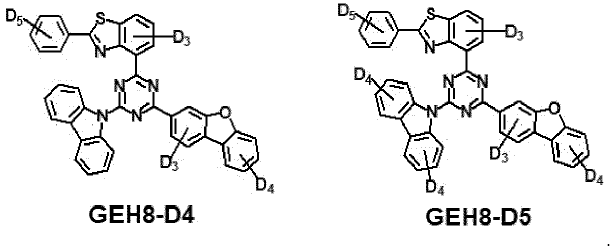

7. The organic compound of any of claims 1, 4 and 5, wherein the organic compound is at least one of the following compounds:

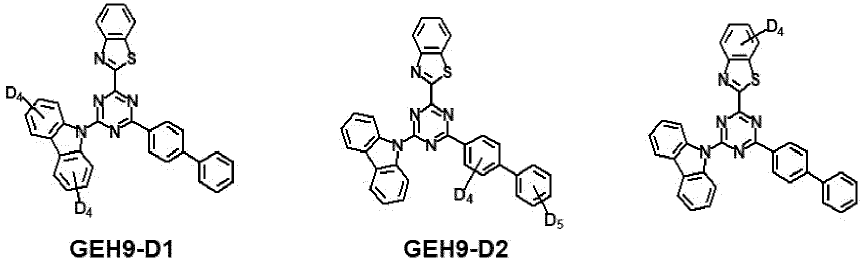

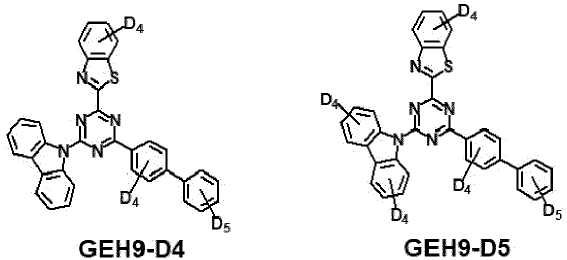

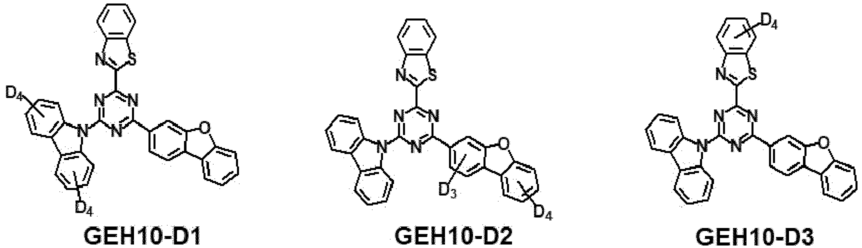

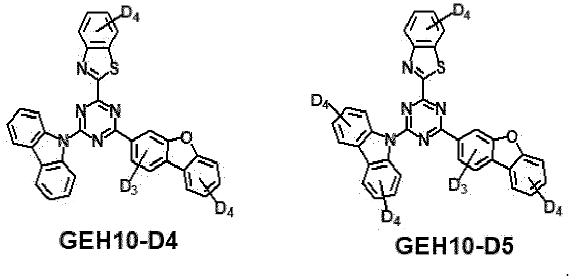

8. An organic light emitting diode, including:
a first electrode;
a second electrode facing the first electrode; and
an emissive layer disposed between the first electrode and the second electrode,
wherein the emissive layer includes an organic compound of any of claims 1 to 7.

9. The organic light emitting diode of claim 8, wherein the at least one emitting material layer includes a first host and a dopant, and wherein the first host includes the organic compound.

10. The organic light emitting diode of claim 9, wherein the at least one emitting material layer further includes a second host.

11. The organic light emitting diode of any of claims 8 to 10, wherein the emissive layer includes:
a first emitting part disposed between the first electrode and the second electrode and including a first emitting material layer;
a second emitting part disposed between the first emitting part and the second electrode and including a second emitting material layer; and
a first charge generation layer disposed between the first emitting part and the second emitting part,
wherein at least one of the first emitting material layer and the second emitting material layer includes the organic compound.

12. The organic light emitting diode of claim 11, wherein the second emitting material layer includes:
a first layer disposed between the first charge generation layer and the second electrode; and
a second layer disposed between the first layer and the second electrode,
wherein at least one of the first layer and the second layer includes the organic compound.

13. The organic light emitting diode of claim 12, wherein one of the first layer and the second layer is a red emitting material layer and the other of the first layer and the second layer is a green emitting material layer, and wherein the green emitting material layer includes the organic compound.

14. The organic light emitting diode of any of claims 11 to 13, wherein the second emitting material layer further includes a third layer disposed between the first layer and the second layer.

15. The organic light emitting diode of any of claims 11 to14, wherein the emissive layer further includes:
a third emitting part disposed between the second emitting part and the second electrode and including a third emitting material layer; and
a second charge generation layer disposed between the second emitting part and the third emitting part.
